# EUROPEAN PATENT APPLICATION

(11) **EP 4 792 848 A2**
(43) Date of publication of application: **19.08.2026**
(21) Application number: 26159450.1
(22) Date of filing: 18.02.2026
(51) Int. Cl.: A61M 1/34, A61M 1/36

(54) **BLOOD PURIFICATION SYSTEM AND BLOOD PURIFICATION DEVICE**

(30) Priority: 18.02.2025 CN 202510180888; 04.02.2026 CN 202610163335
(71) Applicant: Shenzhen Mindray Bio-Medical Electronics Co., Ltd., Shenzhen, Guangdong 518057 (CN)
(72) Inventor: WEI, Kai, Shenzhen, 518057 (CN); ZOU, Huan, Shenzhen, 518057 (CN); ZOU, Zhihua, Shenzhen, 518057 (CN); AI, Shiming, Shenzhen, 518057 (CN); LUO, Caijin, Shenzhen, 518057 (CN)
(74) Representative: KIPA AB

(57) **Abstract**

The application discloses a blood purification system and a blood purification device. The blood purification system includes a panel kit and a blood purification device, the panel kit includes a power component and a monitoring component, the power component includes a power panel and at least two pump pipeline sections, the monitoring component includes a monitoring panel and at least two monitoring pipeline sections, the monitoring pipeline sections are configured to monitor parameter information of a medical liquid and/or a blood, and at least one of the monitoring pipeline sections is provided with a first interface; the blood purification device includes a device housing, an assembly and disassembly component, a pump component and at least two second interfaces.

## Description

### CROSS-REFERENCE TO RELATED APPLICATION

This application claims priority to Chinese patent application No. 202510180888.X filed at China National Intellectual Property Administration (CNIPA) on February 18, 2025 and Chinese patent application No. 202610163335.8 filed at China National Intellectual Property Administration (CNIPA) on February 4, 2026.

### TECHNICAL FIELD

The application belongs to the field of medical devices, and particularly relates to a blood purification system and a blood purification device.

### BACKGROUND

Blood purification therapy is a treatment method that draws a patient's blood out of the patient's body and then removes pathogenic substances from the patient's blood by a filter element, to achieve purification of the blood; main treatment methods include hemodialysis, hemofiltration, hemodiafiltration, hemoperfusion, plasma exchange, etc.

In the related art, the blood purification therapy is usually achieved by connecting a blood purification device to the patient's blood vessel. Therefore, it needs to provide many pipeline sections or the like on the blood purification device, to deliver the patient's blood, replacement fluid, anticoagulant, etc. Furthermore, in order to ensure safety of the blood flowing back to the patient's body, the pipeline sections should be replaced after each time they are used, then there is a large difficulty of mounting the pipeline sections since there is a large number of pipeline sections.

### SUMMARY

According to a first aspect, an embodiment of the application provides a blood purification system, the blood purification system includes a panel kit and a blood purification device, the panel kit includes a power component and a monitoring component, the power component includes a power panel and at least two pump pipeline sections mounted to the power panel; the monitoring component includes a monitoring panel and at least two monitoring pipeline sections mounted to the monitoring panel, the at least two monitoring pipeline sections are configured to monitor parameter information of a medical liquid and/or a blood, the parameter information at least includes a pressure parameter, and at least one of the monitoring pipeline sections is provided with a first interface; here the blood purification device includes a device housing, an assembly and disassembly component, a pump component and at least two second interfaces.

The device housing is provided with a power region and a parameter region.

The assembly and disassembly component includes a first assembly and disassembly member and a second assembly and disassembly member, the power panel can be detachably connected to the first assembly and disassembly member so as to achieve that the power panel is mounted at a first target position in the power region, the monitoring panel can be detachably connected to the second assembly and disassembly member so as to achieve that the monitoring panel is mounted at a second target position in the parameter region, the power panel and the monitoring panel are independent of each other and can be assembled and disassembled separately.

The pump component is arranged in the power region, the pump component includes a blood pump and at least one medical liquid pump, and if the power panel is mounted at the first target position, the blood pump is capable of being adapted to at least one of the pump pipeline sections so as to drive the blood to flow along the at least one of the pump pipeline sections, and the at least one medical liquid pump is capable of being adapted to other pump pipeline sections so as to drive the medical liquid to flow along the other pump pipeline sections.

The second interfaces are arranged in the parameter region, here the monitoring component is capable of being manually mounted such that the monitoring panel is mounted at the second target position and the second interfaces are synchronously connected to the first interfaces respectively, and if the second interfaces are connected to the first interfaces respectively, the blood purification device is capable of monitoring the parameter information through the monitoring pipeline sections.

According to a second aspect, an embodiment of the application provides a blood purification system, the blood purification system includes a panel kit and a blood purification device, the panel kit includes a power component and a monitoring component, the power component includes a power panel and at least two pump pipeline sections mounted to the power panel; the monitoring component includes a monitoring panel and at least two monitoring pipeline sections mounted to the monitoring panel, the at least two monitoring pipeline sections are configured to monitor parameter information of a medical liquid and/or a blood, the parameter information at least includes a pressure parameter, and at least one of the monitoring pipeline sections is provided with a first interface; here the blood purification device includes a device housing, an assembly and disassembly component, a pump component and at least two second interfaces.

The device housing is provided with a power region and a parameter region.

The assembly and disassembly component includes a first assembly and disassembly member and a second assembly and disassembly member, the power panel can be detachably connected to the first assembly and disassembly member so as to achieve that the power panel is mounted at a first target position in the power region, the monitoring panel can be detachably connected to the second assembly and disassembly member so as to achieve that the monitoring panel is mounted at a second target position in the parameter region, the power panel and the monitoring panel are independent of each other and can be assembled and disassembled separately.

The pump component is arranged in the power region, the pump component includes a blood pump and at least one medical liquid pump, and if the power panel is mounted at the first target position, the blood pump is capable of being adapted to at least one of the pump pipeline sections so as to drive the blood to flow along the at least one of the pump pipeline sections, and the at least one medical liquid pump is capable of being adapted to other pump pipeline sections so as to drive the medical liquid to flow along the other pump pipeline sections.

The second interfaces are arranged in the parameter region, here if the monitoring panel is mounted at the second target position, the second interfaces can be extended to be connected to the first interfaces respectively, and if the second interfaces are connected to the first interfaces respectively, the blood purification device is capable of monitoring the parameter information through the monitoring pipeline sections.

According to a third aspect, an embodiment of the application provides a blood purification system, the blood purification system includes a panel kit and a blood purification device, the panel kit includes a power component and a monitoring component, the power component includes a power panel and at least two pump pipeline sections mounted to the power panel; the monitoring component includes a monitoring panel and at least two monitoring pipeline sections mounted to the monitoring panel, the at least two monitoring pipeline sections are configured to monitor parameter information of a medical liquid and/or a blood, the parameter information at least includes a pressure parameter, and at least one of the monitoring pipeline sections is provided with a first interface; here the blood purification device includes a device housing, an assembly and disassembly component, a pump component and at least two second interfaces.

The device housing is provided with a power region and a parameter region.

The assembly and disassembly component includes a first assembly and disassembly member and a second assembly and disassembly member, the power panel can be detachably connected to the first assembly and disassembly member so as to achieve that the power panel is mounted at a first target position in the power region, the monitoring panel can be detachably connected to the second assembly and disassembly member so as to achieve that the monitoring panel is mounted at a second target position in the parameter region, the power panel and the monitoring panel are independent of each other and can be assembled and disassembled separately.

The pump component is arranged in the power region, the pump component includes a blood pump and at least one medical liquid pump, and if the power panel is mounted at the first target position, the blood pump is capable of being adapted to at least one of the pump pipeline sections so as to drive the blood to flow along the at least one of the pump pipeline sections, and the at least one medical liquid pump is capable of being adapted to other pump pipeline sections so as to drive the medical liquid to flow along the other pump pipeline sections.

The second interfaces are arranged in the parameter region, here if the monitoring panel is mounted at a second preset position of the device housing, the second assembly and disassembly member is capable of driving the monitoring panel to move to the second target position, such that the second interfaces are connected to the first interfaces respectively, and if the second interfaces are connected to the first interfaces respectively, the blood purification device is capable of monitoring the parameter information through the monitoring pipeline sections.

According to a fourth aspect, an embodiment of the application provides a blood purification system, the blood purification system includes a blood purification device and a panel kit, the blood purification device includes a device housing, a pump component and a second interface; the panel kit includes a power component and a monitoring component.

The power component includes a power panel and at least two pump pipeline sections, the power panel is configured to be detachably mounted to the device housing, the at least two pump pipeline sections are mounted to the power panel, at least one of the pump pipeline sections is configured to cooperate with the pump component to drive blood to flow along the at least one of the pump pipeline sections, and at least another one of the pump pipeline sections is configured to cooperate with the pump component to drive a medical liquid to flow along the at least another one of the pump pipeline sections; if the power panel is mounted at a first target position of the device housing, the pump pipeline sections are adapted to the pump component, and if the pump pipeline sections are adapted to the pump component, the pump pipeline sections can be driven by the pump component to drive the corresponding blood or medical liquid to flow.

The monitoring component includes a monitoring panel and at least two monitoring pipeline sections, the monitoring panel is configured to be detachably mounted to the device housing, the monitoring panel is arranged separately from the power panel such that the monitoring panel and the power panel are independent of each other and can be assembled and disassembled separately, the at least two monitoring pipeline sections are mounted to the monitoring panel, the at least two monitoring pipeline sections are configured to monitor parameter information of the medical liquid and/or the blood, and the parameter information at least includes a pressure parameter; at least one of the monitoring pipeline sections is provided with a first interface; if the second interface is connected to the first interface, the blood purification device is capable of monitoring the parameter information through the monitoring pipeline sections; the first interface is fixedly arranged relative to the monitoring panel in a process of mounting the monitoring panel to the device housing.

According to a fifth aspect, an embodiment of the application provides a blood purification system, the blood purification system includes a blood purification device and a panel kit, the blood purification device includes a device housing, a pump component and a second interface; the panel kit includes a power component and a monitoring component.

The power component includes a power panel and at least two pump pipeline sections, the power panel is configured to be detachably mounted to the device housing, the at least two pump pipeline sections are mounted to the power panel, at least one of the pump pipeline sections is configured to cooperate with the pump component to drive blood to flow along the at least one of the pump pipeline sections, and at least another one of the pump pipeline sections is configured to cooperate with the pump component to drive a medical liquid to flow along the at least another one of the pump pipeline sections; if the power panel is mounted at a first target position of the device housing, the pump pipeline sections are adapted to the pump component, and if the pump pipeline sections are adapted to the pump component, the pump pipeline sections can be driven by the pump component to drive the corresponding blood or medical liquid to flow.

The monitoring component includes a monitoring panel and at least two monitoring pipeline sections, the monitoring panel is configured to be detachably mounted to the device housing, the monitoring panel is arranged separately from the power panel such that the monitoring panel and the power panel are independent of each other and can be assembled and disassembled separately, the at least two monitoring pipeline sections are mounted to the monitoring panel, the at least two monitoring pipeline sections are configured to monitor parameter information of the medical liquid and/or the blood, and the parameter information at least includes a pressure parameter; at least one of the monitoring pipeline sections is provided with a first interface; if the second interface is connected to the first interface, the blood purification device is capable of monitoring the parameter information through the monitoring pipeline sections; the first interface is movably arranged relative to the monitoring panel, and the movable arrangement allows a user to achieve connection of the first interface to the second interface by operating the first interface.

According to a sixth aspect, an embodiment of the application provides a blood purification system, the blood purification system includes a panel kit and a blood purification device, the panel kit includes a power component and a monitoring component, the power component includes a power panel and at least two pump pipeline sections mounted to the power panel; the monitoring component includes a monitoring panel and at least two monitoring pipeline sections mounted to the monitoring panel, the at least two monitoring pipeline sections are configured to monitor parameter information of a medical liquid and/or a blood, and the parameter information at least includes a pressure parameter, and at least one of the monitoring pipeline sections is provided with a first interface; here the blood purification device includes a device housing, an assembly and disassembly component, a pump component and at least two second interfaces.

The device housing is provided with a power region and a parameter region.

The assembly and disassembly component includes a first assembly and disassembly member and a second assembly and disassembly member, the power panel can be detachably connected to the first assembly and disassembly member so as to achieve that the power panel is mounted at a first target position in the power region, the monitoring panel can be detachably connected to the second assembly and disassembly member so as to achieve that the monitoring panel is mounted at a second target position in the parameter region, the power panel and the monitoring panel are independent of each other and can be assembled and disassembled separately.

The pump component is arranged in the power region, the pump component includes a blood pump and at least one medical liquid pump, and if the power panel is mounted at the first target position, the blood pump is capable of being adapted to at least one of the pump pipeline sections so as to drive the blood to flow along the at least one of the pump pipeline sections, and the at least one medical liquid pump is capable of being adapted to other pump pipeline sections so as to drive the medical liquid to flow along the other pump pipeline sections.

The second interfaces are arranged in the parameter region, here if the monitoring panel is mounted at the second target position, the first interfaces are capable of being manually operated to achieve connection of the first interfaces to the second interfaces, and if the second interfaces are connected to the first interfaces respectively, the blood purification device is capable of monitoring the parameter information through the monitoring pipeline sections.

According to a seventh aspect, an embodiment of the application provides a blood purification device, the blood purification device is configured to be adapted to a panel kit so as to perform extracorporeal blood purification, the panel kit includes a power component and a monitoring component, the power component includes a power panel and at least two pump pipeline sections mounted to the power panel; the monitoring component includes a monitoring panel and at least two monitoring pipeline sections mounted to the monitoring panel, the at least two monitoring pipeline sections are configured to monitor parameter information of a medical liquid and/or a blood, the parameter information at least includes a pressure parameter, and at least one of the monitoring pipeline sections is provided with a first interface; here the blood purification device includes a device housing, an assembly and disassembly component, a pump component and at least two second interfaces.

The device housing is provided with a power region and a parameter region.

The assembly and disassembly component includes a first assembly and disassembly member and a second assembly and disassembly member, the power panel can be detachably connected to the first assembly and disassembly member so as to achieve that the power panel is mounted at a first target position in the power region, the monitoring panel can be detachably connected to the second assembly and disassembly member so as to achieve that the monitoring panel is mounted at a second target position in the parameter region, the power panel and the monitoring panel are independent of each other and can be assembled and disassembled separately.

The pump component is arranged in the power region, the pump component includes a blood pump and at least one medical liquid pump, and if the power panel is mounted at the first target position, the blood pump is capable of being adapted to at least one of the pump pipeline sections so as to drive the blood to flow along the at least one of the pump pipeline sections, and the at least one medical liquid pump is capable of being adapted to other pump pipeline sections so as to drive the medical liquid to flow along the other pump pipeline sections.

The second interfaces are arranged in the parameter region, here the monitoring component is capable of being manually mounted such that the monitoring panel is mounted at the second target position and the second interfaces are synchronously connected to the first interfaces respectively, and if the second interfaces are connected to the first interfaces respectively, the blood purification device is capable of monitoring the parameter information through the monitoring pipeline sections.

According to an eighth aspect, an embodiment of the application provides a blood purification device, the blood purification device is configured to be adapted to a panel kit so as to perform extracorporeal blood purification, the panel kit includes a power component and a monitoring component, the power component includes a power panel and at least two pump pipeline sections mounted to the power panel; the monitoring component includes a monitoring panel and at least two monitoring pipeline sections mounted to the monitoring panel, the at least two monitoring pipeline sections are configured to monitor parameter information of a medical liquid and/or a blood, the parameter information at least includes a pressure parameter, and at least one of the monitoring pipeline sections is provided with a first interface; here the blood purification device includes a device housing, an assembly and disassembly component, a pump component and at least two second interfaces.

The device housing is provided with a power region and a parameter region.

The assembly and disassembly component includes a first assembly and disassembly member and a second assembly and disassembly member, the power panel can be detachably connected to the first assembly and disassembly member so as to achieve that the power panel is mounted at a first target position in the power region, the monitoring panel can be detachably connected to the second assembly and disassembly member so as to achieve that the monitoring panel is mounted at a second target position in the parameter region, the power panel and the monitoring panel are independent of each other and can be assembled and disassembled separately.

The pump component is arranged in the power region, the pump component includes a blood pump and at least one medical liquid pump, and if the power panel is mounted at the first target position, the blood pump is capable of being adapted to at least one of the pump pipeline sections so as to drive the blood to flow along the at least one of the pump pipeline sections, and the at least one medical liquid pump is capable of being adapted to other pump pipeline sections so as to drive the medical liquid to flow along the other pump pipeline sections.

The second interfaces are arranged in the parameter region telescopically, here if the monitoring panel is mounted at the second target position, the second interfaces can be extended to be connected to the first interfaces respectively, and if the second interfaces are connected to the first interfaces respectively, the blood purification device is capable of monitoring the parameter information through the monitoring pipeline sections.

According to a ninth aspect, an embodiment of the application provides a blood purification device, the blood purification device is configured to be adapted to a panel kit so as to perform extracorporeal blood purification, the panel kit includes a power component and a monitoring component, the power component includes a power panel and at least two pump pipeline sections mounted to the power panel; the monitoring component includes a monitoring panel and at least two monitoring pipeline sections mounted to the monitoring panel, the at least two monitoring pipeline sections are configured to monitor parameter information of a medical liquid and/or a blood, the parameter information at least includes a pressure parameter, and at least one of the monitoring pipeline sections is provided with a first interface; here the blood purification device includes a device housing, an assembly and disassembly component, a pump component and at least two second interfaces.

The device housing is provided with a power region and a parameter region.

The assembly and disassembly component includes a first assembly and disassembly member and a second assembly and disassembly member, the power panel can be detachably connected to the first assembly and disassembly member so as to achieve that the power panel is mounted at a first target position in the power region, the monitoring panel can be detachably connected to the second assembly and disassembly member so as to achieve that the monitoring panel is mounted at a second target position in the parameter region, the power panel and the monitoring panel can be assembled and disassembled separately.

The pump component is arranged in the power region, the pump component includes a blood pump and at least one medical liquid pump, and if the power panel is mounted at the first target position, the blood pump is capable of being adapted to at least one of the pump pipeline sections so as to drive the blood to flow along the at least one of the pump pipeline sections, and the at least one medical liquid pump is capable of being adapted to other pump pipeline sections so as to drive the medical liquid to flow along the other pump pipeline sections.

The second interfaces are arranged in the parameter region, here if the monitoring panel is mounted at a second preset position of the device housing, the second assembly and disassembly member is capable of driving the monitoring panel to move to the second target position, such that the second interfaces are connected to the first interfaces respectively, and if the second interfaces are connected to the first interfaces respectively, the blood purification device is capable of monitoring the parameter information through the monitoring pipeline sections.

According to a tenth aspect, an embodiment of the application provides a blood purification device, the blood purification device is configured to be adapted to a panel kit so as to perform extracorporeal blood purification, the panel kit includes a power component and a monitoring component, the power component includes a power panel and at least two pump pipeline sections mounted to the power panel; the monitoring component includes a monitoring panel and at least two monitoring pipeline sections mounted to the monitoring panel, the at least two monitoring pipeline sections are configured to monitor parameter information of a medical liquid and/or a blood, the parameter information at least includes a pressure parameter, and at least one of the monitoring pipeline sections is provided with a first interface; here the blood purification device includes a device housing, an assembly and disassembly component, a pump component and at least two second interfaces.

The device housing is provided with a power region and a parameter region.

The assembly and disassembly component includes a first assembly and disassembly member and a second assembly and disassembly member, the power panel can be detachably connected to the first assembly and disassembly member so as to achieve that the power panel is mounted at a first target position in the power region, the monitoring panel can be detachably connected to the second assembly and disassembly member so as to achieve that the monitoring panel is mounted at a second target position in the parameter region, the power panel and the monitoring panel are independent of each other and can be assembled and disassembled separately.

The pump component is arranged in the power region, the pump component includes a blood pump and at least one medical liquid pump, and if the power panel is mounted at the first target position, the blood pump is capable of being adapted to at least one of the pump pipeline sections so as to drive the blood to flow along the at least one of the pump pipeline sections, and the at least one medical liquid pump is capable of being adapted to other pump pipeline sections so as to drive the medical liquid to flow along the other pump pipeline sections.

The second interfaces are arranged in the parameter region, here if the monitoring panel is mounted at the second target position, the first interfaces are capable of being manually operated to achieve connection of the first interfaces to the second interfaces, and if the second interfaces are connected to the first interfaces respectively, the blood purification device is capable of monitoring the parameter information through the monitoring pipeline sections.

According to an eleventh aspect, an embodiment of the application provides a blood purification device, the blood purification device is configured to be adapted to a panel kit so as to perform extracorporeal blood purification, the panel kit includes a power component, a monitoring component, a heating component and a degassing pot, the power component includes a power panel and at least two pump pipeline sections mounted to the power panel; the monitoring component includes a monitoring panel and at least two monitoring pipeline sections mounted to the monitoring panel, the at least two monitoring pipeline sections are configured to monitor parameter information of a medical liquid and/or a blood, the parameter information at least includes a pressure parameter, and at least one of the monitoring pipeline sections is provided with a first interface; here the blood purification device includes a device housing, an assembly and disassembly component, a pump component and at least two second interfaces.

The device housing includes a first surface, a second surface, a third surface, a fourth surface and a fifth surface; the second surface, the fourth surface, the first surface, the fifth surface and the third surface are sequentially and directly connected along a horizontal direction, the third surface is arranged opposite to the second surface, the first surface is provided with a power region and a parameter region, the second surface is provided with a heating region where the heating component is configured to be detachably mounted, the medical liquid and/or the blood in the heating component are capable of being heated by the blood purification device if the heating component is mounted on the heating region, and each of the third surface and the first surface is bent and connected to the fifth surface; each of the second surface and the first surface is bent and connected to the fourth surface, a syringe pump mounting region is arranged on one of the fourth surface and the fifth surface, a safety monitoring region is arranged on the other of the fourth surface and the fifth surface, a syringe pump is configured to be mounted on the syringe pump mounting region and is configured to inject the medical liquid into pipelines of the panel kit, at least two of a degassing pot fixing seat, a degassing pot monitoring element, a bubble monitoring element and a liquid stopping clamp are arranged on the safety monitoring region, the degassing pot is configured to be detachably mounted and fixed to the degassing pot fixing seat, the bubble monitoring element is configured to monitor whether there are bubbles in a downstream pipeline connected to the degassing pot, and the liquid stopping clamp is configured to control on/off of the downstream pipeline connected to the degassing pot; or, the device housing includes a first surface, a second surface and a third surface, the third surface, here the first surface and the second surface are sequentially and directly connected along a horizontal direction, the third surface is arranged opposite to the second surface, the first surface is provided with a power region and a parameter region, the second surface is provided with a heating region where the heating component is configured to be detachably mounted, the medical liquid and/or the blood in the heating component are capable of being heated by the blood purification device if the heating component is mounted on the heating region, the first surface is further provided with a syringe pump mounting region and a safety monitoring region, a syringe pump is configured to be mounted on the syringe pump mounting region and is configured to inject the medical liquid into pipelines of the panel kit, at least two of a degassing pot fixing seat, a degassing pot monitoring element, a bubble monitoring element and a liquid stopping clamp are arranged on the safety monitoring region, the degassing pot is configured to be detachably mounted and fixed to the degassing pot fixing seat, the bubble monitoring element is configured to monitor whether there are bubbles in a downstream pipeline connected to the degassing pot, and the liquid stopping clamp is configured to control on/off of the downstream pipeline connected to the degassing pot.

The assembly and disassembly component includes a first assembly and disassembly member and a second assembly and disassembly member, the power panel can be detachably connected to the first assembly and disassembly member so as to achieve that the power panel is mounted at a first target position in the power region, the monitoring panel can be detachably connected to the second assembly and disassembly member so as to achieve that the monitoring panel is mounted at a second target position in the parameter region, the power panel and the monitoring panel are independent of each other and can be assembled and disassembled separately.

The pump component is arranged in the power region, the pump component includes a blood pump and at least one medical liquid pump, and if the power panel is mounted at the first target position, the blood pump is capable of being adapted to at least one of the pump pipeline sections so as to drive the blood to flow along the at least one of the pump pipeline sections, and the at least one medical liquid pump is capable of being adapted to other pump pipeline sections so as to drive the medical liquid to flow along the other pump pipeline sections.

The second interfaces are arranged in the parameter region, here if the monitoring panel is mounted at the second target position, the second interfaces are connected to the first interfaces respectively, and if the second interfaces are connected to the first interfaces respectively, the blood purification device is capable of monitoring the parameter information through the monitoring pipeline sections.

In the embodiments of the application, on one hand, with the power panel mounted to the blood purification device, installation and cooperation of a blood loop portion and the pump component and installation and cooperation of a dialysate loop portion and the pump component may be completed, thereby improving a situation where it is easy to mount pipelines of the blood loop portion and the dialysate loop portion reversely, and reducing operations of mounting at least two pump pipeline sections, and thus reducing difficulty of mounting the at least two pump pipeline sections; on the other hand, in the embodiments of the application, connection of the first interface to the second interface may be achieved by mounting the monitoring panel, thereby reducing installation operations of mounting the panel kit to the blood purification device; alternatively, connection of the second interface to the first interface may be further achieved by operating the first interface manually after the monitoring panel is mounted in place, thereby avoiding that it is difficult to mount the first interface of the panel kit in place once, thereby reducing difficulty of mounting the panel kit.

### BRIEF DESCRIPTION OF THE DRAWINGS

Technical solutions of the application and advantageous effects thereof will be apparent from detailed descriptions of specific implementations of the application below with reference to the drawings.
FIG. 1 is a schematic structural diagram of a blood purification device according to an embodiment of the application.
FIG. 2 is a schematic structural diagram of cooperation and installation of the blood purification device shown in FIG. 1 and a panel kit.
FIG. 3 is a schematic diagram of a second structure of a monitoring panel of the panel kit shown in FIG. 2.
FIG. 4 is a schematic structural diagram of a monitoring pipeline section of the panel kit shown in FIG. 2.
FIG. 5 is a schematic diagram of a second structure of a power component of the panel kit shown in FIG. 2.
FIG. 6 is a schematic diagram of a second structure of a panel kit according to an embodiment of the application.
FIG. 7 is a schematic diagram of a flow path of blood in the panel kit shown in FIG. 6.
FIG. 8 is a schematic diagram of a path for a medical liquid in the panel kit shown in FIG. 6 to flow out of a filter.
FIG. 9 is a schematic structural diagram of a secondary membrane pressure monitoring pipeline section of a panel kit according to an embodiment of the application.
FIG. 10 is a schematic diagram of a flow path of an anticoagulant drug in the panel kit shown in FIG. 6.
FIG. 11 is a schematic diagram of flow paths of a replacement fluid and dialysate in the panel kit shown in FIG. 6.
FIG. 12 is a schematic diagram of a third structure of a panel kit according to an embodiment of the application.
FIG. 13 is a first schematic diagram of a second structure of a blood purification device according to an embodiment of the application.
FIG. 14 is a second schematic diagram of a second structure of a blood purification device according to an embodiment of the application.
FIG. 15 is a third schematic diagram of a second structure of a blood purification device according to an embodiment of the application.
FIG. 16 is a first schematic diagram of a third structure of a blood purification device according to an embodiment of the application.
FIG. 17 is a second schematic diagram of a third structure of a blood purification device according to an embodiment of the application.
FIG. 18 is a schematic structural diagram of a device housing in the blood purification device shown in FIG. 14.

### List of reference numerals in the figures:

100. panel kit;
11. power component; 111. power panel; 1111. first connection part; 112. pump pipeline section; 1121. blood pump pipeline section; 1122. waste liquid pressure pump pipeline section; 1123. pump pipeline section of front pump that is before blood pump; 1124. dialysate pump pipeline section; 1125. replacement fluid pump pipeline section;
12. monitoring component; 121. monitoring panel; 1211. second connection part; 122. monitoring pipeline section; 1221. first interface; 1222. pressure monitoring module; 12221. fluid part; 12222. gas part; 12223. diaphragm; 1223. blood drawing pressure monitoring pipeline section; 1224. pressure monitoring pipeline section before filtering; 1225. waste liquid pressure monitoring pipeline section; 1226. blood leakage monitoring pipeline section; 1227. secondary membrane pressure monitoring pipeline section;
1301. blood drawing flow path; 1302. blood pumping flow path; 1303. waste liquid flow path; 1304. connection flow path; 1305. blood return flow path; 1306. injection flow path; 1307. first liquid inlet flow path; 1308. second liquid inlet flow path; 1309. third liquid inlet flow path; 1310. fourth liquid inlet flow path; 1311. fifth liquid inlet flow path; 1312. sixth liquid inlet flow path; 1313. degassing pot; 1314. degassing pot monitoring interface; 1315. collection flow path;
14. electrostatic discharge (ESD) element; 15. heating component; 151. heating panel; 152. heating container; 1521. first heating container; 1522. second heating container; 153. liquid outlet flow path; 1531. first liquid outlet flow path; 1532. second liquid outlet flow path; 1533. third liquid outlet flow path;
200. blood purification device;
201. device housing; 2011. power region; 20111. first pump body region; 20112. second pump body region; 2012. parameter region; 2013. first surface; 2014. second surface; 2015. third surface; 2016. fourth surface; 2017. fifth surface; 2018. sixth surface; 202. assembly and disassembly component; 2021. first assembly and disassembly member; 20211. first claw; 20212. first driving mechanism; 2022. second assembly and disassembly member; 20221. second claw; 20222. second driving mechanism; 203. pump component; 2031. blood pump; 2032. medical liquid pump; 204. second interface; 205. second position detection mechanism; 206. first position detection mechanism; 207. heating region; 208. pipeline switching unit; 2081. first pipeline switching unit; 2082. second pipeline switching unit; 209. degassing pot monitoring element; 210. blood composition analysis component; 211. infusion pump; 212. syringe pump mounting region; 213. syringe pump; 214. safety monitoring region; 215. mounting arm; 216. first weighing element; 217. base; 2171. avoidance part; 2172. distal end; 2173. extension part; 218. support frame; 219. weighing bar; 220. second weighing element; 221. degassing pot fixing seat; 222. bubble monitoring element; 223. liquid stopping clamp;
300. filter.

### DETAILED DESCRIPTION

[**0085]** The technical solutions in the embodiments of the application will be clearly and completely described below with reference to the drawings in the embodiments of the application. It is apparent that the described embodiments are only a part of the embodiments of the application, rather than all of the embodiments. Based on the embodiments in the application, all other embodiments obtained by those skilled in the art without paying any creative work fall within the scope of protection of the application.

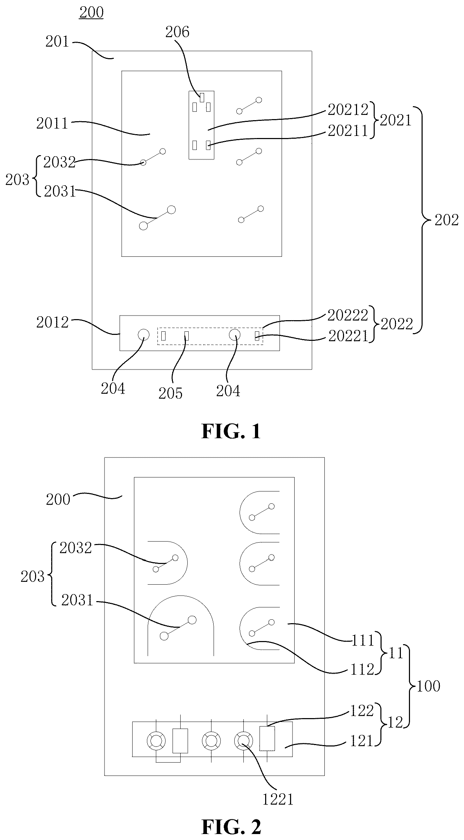

[**0086]** With reference to FIG. 1 and FIG. 2, according to a first aspect, an embodiment of the application provides a blood purification system, the blood purification system includes a panel kit 100 and a blood purification device 200.

[**0087]** The panel kit 100 includes a power component 11 and a monitoring component 12. The power component 11 includes a power panel 111 and at least two pump pipeline sections 112 mounted to the power panel 111. The monitoring component 12 includes a monitoring panel 121 and at least two monitoring pipeline sections 122 mounted to the monitoring panel 121. The at least two monitoring pipeline sections 122 are configured to monitor parameter information of a medical liquid and/or a blood, the parameter information at least includes a pressure parameter, and at least one of the monitoring pipeline sections 122 is provided with a first interface 1221.

[ **0088]** The blood purification device 200 may include a hemodialysis machine, a Continuous Renal Replacement Therapy (CRRT) device or the like, which is not limited in the embodiment of the application. The blood purification device 200 includes a device housing 201, an assembly and disassembly component 202, a pump component 203 and at least two second interfaces 204.

[**0089]** The device housing 201 is provided with a power region 2011 and a parameter region 2012.

[**0090]** The assembly and disassembly component 202 includes a first assembly and disassembly member 2021 and a second assembly and disassembly member 2022, the power panel 111 can be detachably connected to the first assembly and disassembly member 2021 so as to achieve that the power panel 111 is mounted at a first target position in the power region 2011, the monitoring panel 121 can be detachably connected to the second assembly and disassembly member 2022 so as to achieve that the monitoring panel 121 is mounted at a second target position in the parameter region 2012, the power panel 111 and the monitoring panel 121 can be assembled and disassembled separately. It should be noted that in the embodiment of the application, when it is mentioned that the power panel 111 and the monitoring panel 121 can be assembled and disassembled separately, it means that the power panel 111 and the monitoring panel 121 are independent of each other and can be assembled and disassembled separately.

[**0091]** The pump component 203 is arranged in the power region 2011, the pump component 203 includes a blood pump 2031 and at least one medical liquid pump 2032, and if the power panel 111 is mounted at the first target position, the blood pump 2031 is capable of being adapted to at least one of the pump pipeline sections 112 so as to drive the blood to flow along the at least one of the pump pipeline sections 112. Therefore, after the power panel 111 is mounted at the first target position, it is unnecessary for a user to manually mount and fix the pump pipeline sections 112 to the pump component 203, such that installation of the power panel 111 is simpler, and a risk of mounting the at least two pump pipeline sections 112 at wrong positions may be reduced.

[**0092]** The second interfaces 204 are arranged in the parameter region 2012. If the second interfaces 204 are connected to the first interfaces 1221 respectively, the blood purification device 200 is capable of monitoring the parameter information through the monitoring pipeline sections 122.

[**0093]** In a first mode of mounting the monitoring component 12, the panel kit 100 is capable of being manually mounted such that the monitoring panel 121 is mounted at the second target position and the first interfaces 1221 are synchronously connected to the second interfaces 204 respectively. Therefore, operations of mounting the first interfaces 1221 independently may be reduced, and it may avoid that the user mounts the first interfaces 1221 at wrong positions when the user mounts the first interfaces 1221 manually, thereby improving accuracy of mounting the first interfaces 1221.

[**0094]** It should be noted that the monitoring component 12 is capable of being manually mounted such that the monitoring panel 121 is mounted at the second target position and the first interfaces 1221 are synchronously connected to the second interfaces 204 respectively, and it may also be understood that the user may allow the monitoring panel 121 to synchronously drive the first interfaces 1221 to connect to the second interfaces 204 respectively by an action of mounting the monitoring panel 121 at the second target position. In this process, two actions of mounting the monitoring panel 121 at the second target position and connecting the first interfaces 1221 to the second interfaces 204 respectively may be completed simultaneously, or there may be a certain time difference between the two actions, for example, when the first interfaces 1221 are movably arranged relative to the monitoring panel 121, it is possible that the monitoring panel 121 still continues to move by a certain distance after the first interfaces 1221 are connected to the second interfaces 204 respectively, and then may be mounted at the second target position of the device housing 201, which is not limited in the embodiment of the application.

[**0095]** It should also be noted that the monitoring component 12 may be manually mounted once such that the monitoring panel 121 is mounted at the second target position and the first interfaces 1221 are synchronously connected to the second interfaces 204 respectively, or the monitoring component 12 may be manually mounted at least twice such that the monitoring panel 121 is mounted at the second target position and the first interfaces 1221 are synchronously connected to the second interfaces 204 respectively, which is not limited in the embodiment of the application.

[**0096]** In a second mode of mounting the monitoring component 12, if the monitoring panel 121 is mounted at the second target position, the second interfaces 204 can be extended to be connected to the first interfaces 1221 respectively. Therefore, the user only needs to mount the monitoring panel 121 at the second target position simply, the second interfaces 204 on the blood purification device 200 may be extended to be connected to the first interfaces 1221 of the monitoring pipeline sections 122 respectively, and it is unnecessary for the user to mount the monitoring panel 121 at the second target position of the device housing 201 and then manually connect the second interfaces 204 to the first interfaces 1221 respectively, such that installation of the panel kit 100 is simpler.

[**0097]** In some implementations, if the monitoring panel 121 is mounted at a second preset position of the device housing 201, the blood purification device 200 is capable of driving the monitoring panel 121 to move to the second target position.

[**0098]** Optionally, the user may also mount the monitoring panel 121 at the second target position of the device housing 201 directly, which is not limited in the embodiment of the application.

[**0099]** That is, in the second mode of mounting the monitoring component 12, the user may manually mount the monitoring panel 121 at the second preset position, and then the blood purification device 200 automatically drives the monitoring panel 121 to the second target position to complete installation, or the user may directly mount the monitoring panel 121 at the second target position to complete installation, which is not limited in the embodiment of the application.

[**0100]** In a third mode of mounting the monitoring component 12, if the monitoring panel 121 is mounted at a second preset position of the device housing 201, the device housing 201 is capable of driving the monitoring panel 121 to move to the second target position, such that the second interfaces 1221 are fixedly connected to the first interfaces 204 respectively. Therefore, for installation of the panel kit 100, the user only needs to mount the monitoring panel 121 at the second preset position of the device housing 201, and then the device housing 201 automatically drives the monitoring panel 121 to the second target position so as to achieve automatic installation of the monitoring panel 121, such that installation of the panel kit 100 is simpler.

[**0101]** In a fourth mode of mounting the monitoring component 12, if the monitoring panel 121 is mounted at the second target position, the first interfaces 1221 are capable of being manually operated to achieve connection of the first interfaces 1221 to the second interfaces 204, and if the second interfaces 204 are connected to the first interfaces 1221 respectively, the blood purification device 200 is capable of monitoring the parameter information through the monitoring pipeline sections 122.

[**0102]** After the monitoring panel 121 is mounted in place, it may facilitate the user to manually connect the first interface 1221 to the second interface 204, to avoid failure to align and seal between the first interface 1221 and the second interface 204.

[**0103]** In an embodiment, in actual production and manufacturing processes, it is inevitable that each part has a form tolerance, and it is also inevitable that each part has a position tolerance there-between during assembly of each part. Therefore, after the monitoring panel 121 is mounted at the second target position, it is enough for the user to manually operate to achieve connection of the first interface 1221 to the second interface 204, then it is unnecessary to use a manufacturing process with high cost and great difficulty to ensure that an accumulated geometric tolerance of each part such as the first interface 1221, the monitoring panel 121, the blood purification device 200 or the like is located in an acceptable range so as to achieve that the first interface 1221 and the monitoring panel 121 are mounted in place once, and difficulty of manufacturing the monitoring component 12 and difficulty of mounting the monitoring component 12 to the device housing 201 may be reduced finally.

[**0104]** Furthermore, in an actual assembly process of mounting the panel kit 100 to the blood purification device 200, when there is a large number of parts required to be mounted in place once, such as the monitoring panel 121, the first interface 1221 or the like, it is difficult to ensure that all these parts may be mounted in place synchronously. Therefore, after the monitoring panel 121 is mounted in place, the user manually connects the first interface 1221 to the second interface 204, which may make installation of the panel kit 100 simpler and more convenient.

[**0105]** In an embodiment, connection of the first interface 1221 to the second interface 204 may be sealed connection between the first interface 1221 and the second interface 204.

[**0106]** It may also be understood that in the fourth mode of mounting the monitoring component 12, the user may manually mount the monitoring panel 121 at the second preset position, and then the blood purification device 200 automatically drives the monitoring panel 121 to the second target position to complete installation, or the user may directly mount the monitoring panel 121 at the second target position to complete installation, which is not limited in the embodiment of the application.

[**0107]** In some implementations, the first interface 1221 may be connected to the second interface 204 by screws, snap-fit, plug-in, etc. Based on this, if the monitoring panel 121 is mounted at the second target position, the first interface 1221 may be manually operated by tightening the first interface 1221, pressing the first interface 1221, plugging the first interface 1221 or the like, to achieve connection of the first interface 1221 to the second interface 204, which is not limited in the embodiment of the application.

[**0108]** For example, the first interface 1221 is provided with a knob. If the monitoring panel 121 is mounted at the second target position, connection of the first interface 1221 to the second interface 204 may be achieved by an operation of turning the knob of the first interface 1221 by the user.

[**0109]** The above descriptions are an overall introduction of four modes of mounting the monitoring panel 121 in the embodiments of the application, and the modes of the monitoring panel 121 will be continuously introduced below with reference to structures of the assembly and disassembly component 202.

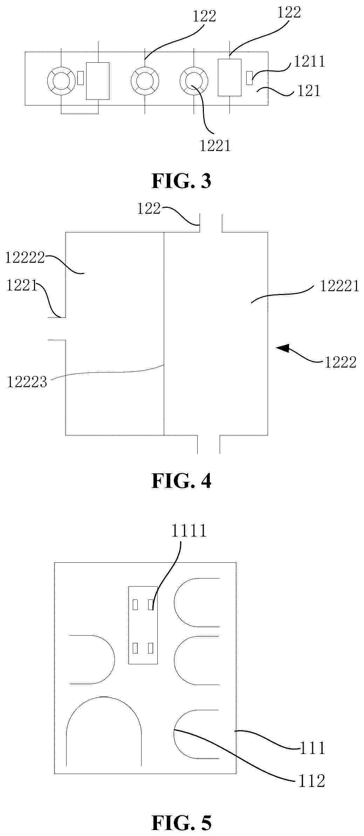

[**0110]** With reference to FIG. 1 and FIG. 3, in some implementations, the second assembly and disassembly member 2022 includes a second claw 20221, the second claw 20221 is clamped to a second connection part 1211 of the monitoring panel 121 to achieve detachable connection.

[**0111]** Exemplarily, the second connection part 1211 may include a second clamping hole, and the second claw 20221 may be clamped into the second clamping hole. Of course, it is also possible that the second claw 20221 includes at least two second clamping arms, and the second connection part 1211 is a convex structure on the monitoring panel 121, such that the second connection part 1211 may be clamped between two second clamping arms, which is not limited in the embodiment of the application.

[**0112]** In some implementations, the second claw 20221 is fixedly arranged on the device housing 201.

[**0113]** For example, in the first mode of mounting the monitoring panel 121, with the second claw 20221 fixedly arranged on the device housing 201, it may avoid that the monitoring panel 121 is mounted out of place due to shake of the second claw 20221 when the user directly mounts the monitoring panel 121 at the second target position, and thus it may improve accuracy of a detection result of the blood purification device 200.

[**0114]** Furthermore, in the second and fourth modes of mounting the monitoring panel 121, when the monitoring panel 121 is also directly mounted at the second target position by the user, it may also avoid that the monitoring panel 121 is mounted out of place due to shake of the second claw 20221 when the user directly mounts the monitoring panel 121 at the second target position, and thus it may improve accuracy of the detection result of the blood purification device 200.

[**0115]** In some implementations, the assembly and disassembly component 202 further includes a second driving mechanism 20222, the second driving mechanism 20222 is transmissively connected to the second claw 20221, and if the monitoring panel 121 is mounted at a second preset position by the second claw 20221, the second driving mechanism 20222 is configured to drive the second claw 20221 to move so as to drive the monitoring panel 121 to be mounted at the second target position. Therefore, the user only needs to mount the monitoring panel 121 to the second claw 20221 simply, and then the second driving mechanism 20222 of the blood purification device 200 automatically drives the second claw 20221 to move such that the monitoring component 12 is accurately mounted at the second target position of the device housing 201, and thus the monitoring component 12 has advantages of convenient installation, accuracy and reliability.

[**0116]** In some implementations, if the monitoring panel 121 is located at the second preset position, the first interface 1221 is not connected to the second interface 204 on the device housing 201. Therefore, if the monitoring panel 121 is located at the second preset position, a certain gap may be reserved between the first interface 1221 and the second interface 204, such that the first interface 1221 may move toward the second interface 204 correspondingly when the monitoring panel 121 moves toward the second target position.

[**0117]** Furthermore, when the user needs to replace the monitoring component 12 on the device housing 201 (that is, when the user needs to remove the monitoring component 12 from the device housing 201), the device housing 201 may drive the monitoring panel 121 to move from the second target position to the second preset position so as to separate the first interface 1221 from the second interface 204, such that the user may remove the panel kit 100 from the device housing 201 more conveniently.

[**0118]** In some implementations, the blood purification device 200 further includes a second position detection mechanism 205. When the second claw 20221 is fixedly arranged on the device housing 201, the second position detection mechanism 205 is configured to detect whether the monitoring panel 121 is mounted at the second target position. When the second claw 20221 is transmissively connected to the second driving mechanism 20222, the second position detection mechanism 205 is configured to detect whether the monitoring panel 121 is mounted at the second preset position and/or the second target position.

[**0119]** Therefore, the blood purification device 200 may determine whether the monitoring panel 121 is mounted in place when the monitoring panel 121 is mounted at the second preset position, based on a detection result of the second position detection mechanism 205, thereby determining whether the blood purification device 200 needs to drive the monitoring panel 121 to move from the second preset position to the second target position; and after the blood purification device 200 drives the monitoring panel 121 to move from the second preset position to the second target position, the blood purification device 200 may further determine whether the monitoring panel 121 is mounted in place, based on the detection result of the second position detection mechanism 205.

[**0120]** Of course, when the monitoring panel 121 is directly mounted at the second target position by the user, the second position detection mechanism 205 may be only configured to detect whether the monitoring panel 121 is located at the second preset position, which is not limited in the embodiment of the application.

[**0121]** In some implementations, if the monitoring panel 121 is mounted at the second preset position and/or the second target position, the monitoring panel 121 blocks the second position detection mechanism 205 or comes into contact with the second position detection mechanism 205, such that the second position detection mechanism 205 may detect position of the monitoring panel 121.

[**0122]** Exemplarily, the second position detection mechanism 205 may include a photoelectric sensor such as an infrared ranging sensor, a laser ranging sensor, a photoelectric gate or the like, such that the second position detection mechanism 205 may detect the position of the monitoring panel 121 by detecting a distance of the monitoring panel 121, detecting whether the monitoring panel 121 blocks the corresponding photoelectric gate, etc.

[**0123]** Optionally, the second position detection mechanism 205 may further include a pressure sensor, to detect the position of the monitoring panel 121 by a pressure applied to the second position detection mechanism 205 when the monitoring panel 121 is mounted at the second target position.

[**0124]** Of course, in some other implementations, the second position detection mechanism 205 may further include a travel switch, a Hall sensor, a proximity switch or the like, which is not limited in the embodiment of the application.

[**0125]** It may also be understood that when the second position detection mechanism 205 is configured to detect whether the monitoring panel 121 is mounted at the second preset position and the second target position, the second position detection mechanism 205 may include at least two second sensors, at least one of the second sensors is configured to detect whether the monitoring panel 121 is mounted at the second preset position, and at least another one of the second sensors is configured to detect whether the monitoring panel 121 is mounted at the second target position.

[**0126]** In some implementations, the panel kit 100 further includes a second flexible pipeline, the second flexible pipeline is connected between at least one of the pump pipeline sections 112 and at least one of the monitoring pipeline sections 122.

[**0127]** It may be understood that when the pump component 203 drives the pump pipeline sections 112 to operate, the pump component 203 not only causes the power panel 111 to vibrate but also pulls the pump pipeline sections 112. Therefore, on a basis that the power panel 111 is separated from the monitoring panel 121 to prevent vibration of the power panel 111 from transmitting to the monitoring panel 121, when the pump pipeline sections 112 are pulled, the second flexible pipeline may be adaptively deformed to prevent the pump pipeline sections 112 from pulling the monitoring pipeline sections 122 connected thereto, thereby preventing the first interface 1221 from loosening and falling off by interference of the monitoring panel 121 and/or the second flexible pipeline connected thereto, and accuracy of the detection result of the blood purification device 200 may be improved finally.

[**0128]** In some implementations, flow paths connected between the pump pipeline sections 112 and the monitoring pipeline sections 122 are hoses.

[**0129]** In some implementations, the first interface 1221 is fixedly arranged relative to the monitoring panel 121. For example, the first interface 1221 is fixedly arranged relative to the monitoring panel 121 in a process of mounting the monitoring panel 121 to the device housing 201. Therefore, in the process of mounting the monitoring panel 121, it may avoid that the first interface 1221 is located at a wrong position after the first interface 1221 moves relative to the monitoring panel 121, and thus it may avoid that the second interface 204 collides with the first interface 1221 and cannot be mounted in place. Of course, when the first interface 1221 is prevented from moving relative to the monitoring panel 121, it may also avoid that the first interface 1221 is shaken during operation of the blood purification device 200 to cause the second interface 204 to loosen and fall off relative to the first interface 1221, therefore accuracy and reliability of the detection result of the blood purification device 200 may be improved.

[**0130]** In some implementations, the first interface 1221 is movably arranged relative to the monitoring panel 121.

[**0131]** For example, the first interface 1221 is movably arranged relative to the monitoring panel 121, and the movable arrangement allows a user to achieve connection of the first interface 1221 to the second interface 204 by operating the first interface 1221.

[**0132]** After the monitoring panel 121 is mounted in place, it may facilitate the user to manually connect the first interface 1221 to the second interface 204, to avoid failure to align and seal between the first interface 1221 and the second interface 204.

[**0133]** In an embodiment, in actual production and manufacturing processes, it is inevitable that each part has a form tolerance, and it is also inevitable that each part has a position tolerance there-between during assembly of each part. Therefore, after the monitoring panel 121 is mounted at the second target position, it is enough for the user to manually operate to achieve connection of the first interface 1221 to the second interface 204, then it is unnecessary to use a manufacturing process with high cost and great difficulty to ensure that an accumulated geometric tolerance of each part such as the first interface 1221, the monitoring panel 121, the blood purification device 200 or the like is located in an acceptable range so as to achieve that the first interface 1221 and the monitoring panel 121 are mounted in place once, and difficulty of manufacturing the monitoring component 12 and difficulty of mounting the monitoring component 12 to the device housing 201 may be reduced finally.

[**0134]** Furthermore, in an actual assembly process of mounting the panel kit 100 to the blood purification device 200, when there is a large number of parts required to be mounted in place once, such as the monitoring panel 121, the first interface 1221 or the like, it is difficult to ensure that all these parts may be mounted in place synchronously. Therefore, after the monitoring panel 121 is mounted in place, the user manually connects the first interface 1221 to the second interface 204, which may make installation of the panel kit 100 simpler and more convenient.

[**0135]** Optionally, in the process of mounting the monitoring panel 121 to the device housing 201, the first interface 1221 is movable relative to the monitoring panel 121 to compensate for a tolerance of at least one of the first interface 1221, the monitoring panel 121 and the blood purification device 200.

[**0136]** Therefore, when the first interface 1221 is movable relative to the monitoring panel 121, it may avoid that the second interface 204 cannot be connected to the first interface 1221 due to a geometric tolerance accumulated in processes of manufacturing and assembling each part of the first interface 1221, the monitoring panel 121 and the blood purification device 200. Furthermore, compared to the movable arrangement allowing the user to achieve connection of the first interface 1221 to the second interface 204 by operating the first interface 1221, in the embodiment of the application, the first interface 1221 is controlled to be movable in a small range only to compensate for the tolerance, it may also avoid that there is an excessive movement amplitude of the first interface 1221 relative to the monitoring panel 121, and thus it may avoid that the first interface 1221 collides with the second interface 204 and cannot be mounted in place. Of course, when the excessive movement amplitude of the first interface 1221 relative to the monitoring panel 121 is avoided, it may also avoid that the first interface 1221 is shaken during operation of the blood purification device 200 to cause the first interface 1221 to loosen and fall off relative to the second interface 204, therefore accuracy and reliability of the detection result of the blood purification device 200 may be improved.

[**0137]** In some implementations, the first interface 1221 is non-detachably connected to a corresponding one of the monitoring pipeline sections 122. For example, the first interface 1221 may be connected to the corresponding monitoring pipeline section 122 by bonding, integrally molding, welding thereto or the like, therefore situations where the first interface 1221 is loosened or even fallen off or the like to cause an inaccurate detection result of the blood purification device 200, may be avoided.

[**0138]** Optionally, in some other implementations, the first interface 1221 is detachably connected to the corresponding monitoring pipeline section 122. For example, the first interface 1221 is connected to the corresponding monitoring pipeline section 122 by screw-connecting, clamping, magnetically fixing thereto or the like, such that appropriate first interfaces 1221 may be replaced based on variations of models of the second interfaces 204 of the blood purification device 200.

[**0139]** In some implementations, the first interface 1221 is connected to the corresponding monitoring pipeline section 122 by a connector. The connector may be a Luer connector or other forms of connectors, which is not limited in the embodiment of the application.

[**0140]** In some implementations, the monitoring pipeline sections 122 are non-detachably connected to the monitoring panel 121. For example, the monitoring pipeline sections 122 may be connected to the monitoring panel 121 by bonding, integrally molding, fusion-connecting, welding thereto or the like, therefore situations where the monitoring pipeline sections 122 are loosened or even fallen off or the like to cause an inaccurate detection result of the blood purification device 200, may be avoided.

[**0141]** Optionally, the monitoring pipeline sections 122 may also be detachably connected to the monitoring panel 121. For example, the monitoring pipeline sections 122 may be connected to the monitoring panel 121 by screw-connecting, clamping, magnetically fixing thereto or the like, such that appropriate monitoring pipeline sections 122 or monitoring panels 121 may be replaced based on variations of the blood purification device 200.

[**0142]** With continuous reference to FIG. 4, in some implementations, at least one of the monitoring pipeline sections 122 includes a pressure monitoring module 1222, the pressure monitoring module 1222 includes a fluid part 12221, a gas part 12222, a diaphragm 12223 and the first interface 1221. A side cavity for fluid defined at least partially by the fluid part 12221 is separated from a side cavity for gas defined at least partially by the gas part 12222 through the diaphragm 12223, the side cavity for fluid is used for the blood or medical liquid to flow therethrough, the first interface 1221 is fluidly communicated with the side cavity for gas, and if the second interface 204 is connected to the first interface 1221, the monitoring pipeline sections 122 are used for the blood purification device 200 to monitor a pressure parameter of the medical liquid or the blood flowing through the side cavity for fluid.

[**0143]** Therefore, when the blood or medical liquid flows through the side cavity for fluid, the blood or medical liquid may squeeze the diaphragm 12223 to cause a gas pressure in the side cavity for gas to change, and then the pressure parameter of the corresponding blood or medical liquid may be measured by docking the second interface 204 on the blood purification device 200 with the first interface 1221 to measure the gas pressure in the side cavity for gas, such that it is unnecessary for the second interface 204 to come into contact with the corresponding blood or medical liquid, thereby avoiding that the blood or medical liquid is contaminated.

[**0144]** Of course, in some other implementations, it is also possible that at least one of the monitoring pipeline sections 122 is adapted to a contact pressure sensor on the blood purification device 200. Therefore, the contact pressure sensor on the blood purification device 200 may directly abut against the diaphragm 12223 or a flexible region of the monitoring pipeline section 122, to monitor the pressure parameter of the corresponding blood or medical liquid, which is not limited in the embodiment of the application.

[**0145]** In some implementations, the second interface 204 is fixedly arranged relative to the device housing 201.

[**0146]** For example, the second interface 204 is fixedly arranged relative to the device housing 201 in the process of mounting the monitoring panel 121 to the device housing 201. Therefore, in the process of mounting the monitoring panel 121, it may avoid that the second interface 204 moves relative to the device housing 201, and thus it may avoid that the second interface 204 interferes with the first interface 1221 and cannot be mounted in place. Of course, when the second interface 204 is prevented from moving relative to the device housing 201, it may also avoid that the second interface 204 is shaken during operation of the blood purification device 200 to cause the second interface 204 to loosen and fall off relative to the first interface 1221, therefore accuracy and reliability of the detection result of the blood purification device 200 may be improved.

[**0147]** In some implementations, the second interface 204 is movably arranged relative to the device housing 201.

[**0148]** For example, in the process of mounting the monitoring panel 121 to the device housing 201, the second interface 204 is movable relative to the device housing 201 to compensate for a tolerance of at least one of the first interface 1221, the monitoring panel 121 and the second interface 204.

[**0149]** Therefore, when the second interface 204 is movable relative to the device housing 201, it may avoid that the second interface 204 cannot be connected to the first interface 1221 due to a geometric tolerance accumulated in processes of manufacturing and assembling each part of the first interface 1221, the monitoring panel 121 and the second interface 204. Furthermore, the second interface 204 is controlled to be movable in a small range only to compensate for the tolerance, it may also avoid that there is an excessive movement amplitude of the second interface 204 relative to the monitoring panel 121, and thus it may avoid that the second interface 204 collides with the first interface 1221 and cannot be mounted in place. Of course, when the excessive movement amplitude of the second interface 204 relative to the monitoring panel 121 is avoided, it may also avoid that the second interface 204 is shaken during operation of the blood purification device 200 to cause the first interface 1221 to loosen and fall off relative to the second interface 204, therefore accuracy and reliability of the detection result of the blood purification device 200 may be improved.

[**0150]** The above descriptions are some introductions of modes of mounting the monitoring component 12 in the embodiments of the application, and the technical solutions in the embodiments of the application will be continuously introduced below with reference to some modes of mounting the power component 11 in the embodiments of the application.

[**0151]** In some implementations, the pump pipeline sections 112 include a first flexible pipeline, the first flexible pipeline abuts against a pump head of the corresponding blood pump 2031 or medical liquid pump 2032 if the pump pipeline sections 112 are adapted to the corresponding blood pump 2031 or medical liquid pump 2032, such that rotation of the pump head is capable of driving the first flexible pipeline to be wound around the pump head. Therefore, in a process of mounting the power component 11, it only needs the first flexible pipeline to come into contact with the pump head of the pump component 203, and it is unnecessary for the user to manually wind the pump pipeline section 112 around the pump head of the pump component 203, thereby making installation of the pump pipeline section 112 simpler and more convenient.

[**0152]** For example, the blood pump 2031 and/or the medical liquid pump 2032 may include a peristaltic pump, and when the first flexible pipeline comes into contact with a pump head of the peristaltic pump, the pump head of the peristaltic pump rotates first to drive the first flexible pipeline to be wound around the pump head, and then the peristaltic pump continues to squeeze the first flexible pipeline by rotation of the pump head, thereby driving the medical liquid and/or the blood in the pump pipeline section 112 to flow.

[**0153]** Optionally, the pump pipeline sections 112 include a first flexible pipeline, the first flexible pipeline is wound around a pump head of the corresponding blood pump 2031 or medical liquid pump 2032 if the pump pipeline sections 112 are adapted to the corresponding blood pump 2031 or medical liquid pump 2032, such that rotation of the pump head directly drives the corresponding medical liquid and/or blood in the first flexible pipeline to flow.

[**0154]** It may be understood that if the pump pipeline sections 112 are adapted to the pump component 203, no matter the first flexible pipeline is directly wound around the pump head of the pump component 203 or the pump component 203 needs to operate such that the first flexible pipeline is wound around the pump head of the pump component 203, it means that after the power panel 111 is mounted at the first target position, it is unnecessary for the user to connect the pump pipeline sections 112 to the pump component 203, which may make installation of the power component 11 simpler.

[**0155]** In a first mode of mounting the power component 11, if the power panel 111 is mounted at a first preset position of the device housing 201, the power panel 111 is capable of being driven by the blood purification device 200 to move to the first target position, such that the pump pipeline sections 112 are adapted to the pump component 203.

[**0156]** Therefore, for installation of the power component 11, the user only needs to mount the power panel 111 at the first preset position of the device housing 201, and then the blood purification device 200 automatically drives the power panel 111 to the first target position so as to achieve automatic installation of the power panel 111, such that installation of the power component 11 is simpler.

[**0157]** Correspondingly, if the power panel 111 is mounted at the first preset position, the pump pipeline sections 112 may not be adapted to the pump component 203. Therefore, if the power panel 111 is mounted at the first preset position, a certain gap may be reserved between the pump pipeline section 112 and the pump head of the corresponding blood pump 2031 or medical liquid pump 2032, such that the pump pipeline section 112 may move toward the pump head correspondingly when the power panel 111 moves toward the first target position.

[**0158]** Furthermore, when the user needs to replace the power component 11 on the blood purification device 200 (that is, when the user needs to remove the power component 11 from the blood purification device 200), the blood purification device 200 may drive the power panel 111 to return from the first target position to the first preset position so as to separate the pump pipeline section 112 from the pump head of the corresponding blood pump 2031 or medical liquid pump 2032, such that the user may remove the power component 11 from the blood purification device 200 more conveniently.

[**0159]** Of course, in some other implementations, if the power panel 111 is mounted at the first preset position, the pump pipeline section 112 and the pump head of the corresponding blood pump 2031 or medical liquid pump 2032 may also come into contact with each other. For example, if the power panel 111 is mounted at the first preset position, the first flexible pipeline of the pump pipeline sections 112 may abut against the pump head of the corresponding blood pump 2031 or medical liquid pump 2032, and the first flexible pipeline may be deformed adaptively when the power panel 111 moves toward the first target position.

[**0160]** In a second mode of mounting the power component 11, the power component 11 is capable of being manually mounted such that the power panel 111 is mounted at the first target position and each of the pump pipeline sections 112 is synchronously adapted to the corresponding blood pump 2031 or medical liquid pump 2032. Therefore, after the power panel 111 is mounted at the first target position, it is unnecessary for the user to manually mount the pump pipeline section 112 to the pump head of the corresponding blood pump 2031 or medical liquid pump 2032, which may reduce difficulty of mounting the power component 11 by the user.

[**0161]** It should also be noted that the power component 11 may be manually mounted once such that the power panel 111 is mounted at the first target position and the pump pipeline section 112 is synchronously adapted to the corresponding blood pump 2031 or medical liquid pump 2032, or the power component 11 may be manually mounted at least twice such that the power panel 111 is mounted at the first target position and the pump pipeline section 112 is synchronously adapted to the corresponding blood pump 2031 or medical liquid pump 2032, which is not limited in the embodiment of the application.

[**0162]** The above descriptions are an overall introduction of two modes of mounting the power component 11 in the embodiments of the application.

[**0163]** With reference to FIG. 1 and FIG. 5, in some implementations, the first assembly and disassembly member 2021 includes a first claw 20211, the first claw 20211 is clamped to a first connection part 1111 of the power panel 111 to achieve detachable connection.

[**0164]** Correspondingly, the first connection part 1111 may include a first clamping hole, and the first claw 20211 may be clamped into the first clamping hole. Of course, it is also possible that the first claw 20211 includes at least two first clamping arms, and the first connection part 1111 is a convex structure on the power panel 111, such that the first connection part 1111 may be clamped between two first clamping arms, which is not limited in the embodiment of the application.

[**0165]** In some implementations, the first claw 20211 is fixedly arranged on the device housing 201.

[**0166]** For example, in the first mode of mounting the power component 11, with the first claw 20211 fixedly arranged on the device housing 201, it may avoid that the power component 11 is mounted out of place due to shake of the first claw 20211 when the user directly mounts the power component 11 at the first target position, and thus it may improve accuracy of the detection result of the blood purification device 200.

[**0167]** In some implementations, the assembly and disassembly component 202 further includes a first driving mechanism 20212, the first driving mechanism 20212 is transmissively connected to the first claw 20211, and if the power panel 111 is mounted at a first preset position by the first claw 20211, the first driving mechanism 20212 is configured to drive the first claw 20211 to move so as to drive the power panel 111 to be mounted at the first target position. Therefore, the user only needs to mount the power panel 111 to the first claw 20211 simply, and then the first driving mechanism 20212 of the blood purification device 200 automatically drives the first claw 20211 to move such that the power component 11 is accurately mounted at the first target position of the device housing 201, and thus the power component 11 has advantages of convenient installation, accuracy and reliability.

[**0168]** In some implementations, the blood purification device 200 further includes a first position detection mechanism 206. When the first claw 20211 is fixedly arranged on the device housing 201, the first position detection mechanism 206 is configured to detect whether the power panel 111 is mounted at the first target position. When the first claw 20211 is transmissively connected to the first driving mechanism 20212, the first position detection mechanism 206 is configured to detect whether the monitoring panel 121 is mounted at the first preset position and/or the first target position.

[**0169]** Therefore, the blood purification device 200 may determine whether the power panel 111 is mounted in place when the power panel 111 is mounted at the first preset position, based on a detection result of the first position detection mechanism 206, thereby determining whether it needs to drive the power panel 111 to move from the first preset position to the first target position by the first driving mechanism 20212; and after the blood purification device 200 drives the power panel 111 to move from the first preset position to the first target position by the first driving mechanism 20212, the blood purification device 200 may further determine whether the power panel 111 is mounted in place, based on the detection result of the first position detection mechanism 206, thereby avoiding abnormal installation of the power panel 111.

[**0170]** Furthermore, when the first claw 20211 is fixedly arranged on the device housing 201, the first position detection mechanism 206 may further detect whether the power panel 111 is mounted in place when the power panel 111 is mounted at the first target position.

[**0171]** In some implementations, if the power panel 111 is mounted at the first preset position and/or the first target position, the power panel 111 blocks the first position detection mechanism 206 or comes into contact with the first position detection mechanism 206, such that the first position detection mechanism 206 may detect position of the power panel 111.

[**0172]** Exemplarily, the first position detection mechanism 206 may include a photoelectric sensor such as an infrared ranging sensor, a laser ranging sensor, a photoelectric gate or the like, such that the first position detection mechanism 206 may detect the position of the power panel 111 by detecting a distance of the power panel 111, detecting whether the power panel 111 blocks the corresponding photoelectric gate, etc.

[**0173]** Optionally, the first position detection mechanism 206 may further include a pressure sensor, to detect the position of the power panel 111 by a pressure applied to the first position detection mechanism 206 when the power panel 111 is mounted at the first preset position and/or the first target position.

[**0174]** Of course, in some other implementations, the first position detection mechanism 206 may further include a travel switch, a Hall sensor, a proximity switch or the like, which is not limited in the embodiment of the application.

[**0175]** It may also be understood that when the first position detection mechanism 206 is configured to detect whether the power panel 111 is mounted at the first preset position and the first target position, the first position detection mechanism 206 may include at least two first sensors, at least one of the first sensors is configured to detect whether the power panel 111 is mounted at the first preset position, and at least another one of the first sensors is configured to detect whether the power panel 111 is mounted at the first target position.

[**0176]** The technical solutions in the embodiments of the application will be continuously introduced below with reference to some other structures of the panel kit 100.

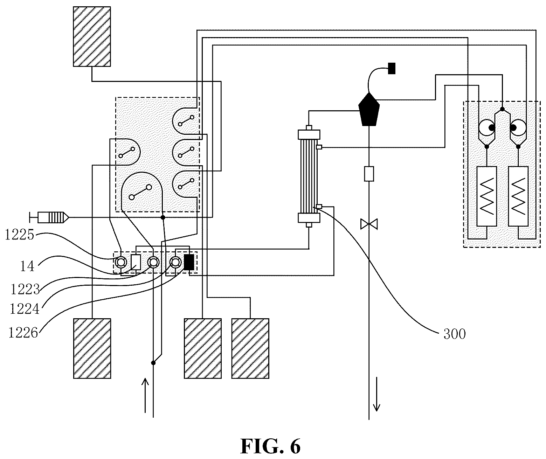

[**0177]** With continuous reference to FIG. 6, in some implementations, the blood purification system further includes a filter 300. The filter 300 is configured to filter blood flowing through the pump pipeline sections 112.

[**0178]** The filter 300 may be arranged on the blood purification device 200, and after the panel kit 100 is mounted to the device housing 201, the panel kit 100 is connected to the filter 300 on the blood purification device 200. Alternatively, the filter 300 may be arranged on the panel kit 100, and the filter 300 is fixed to the blood purification device 200 in a process of mounting the panel kit 100 to the blood purification device 200, which is not limited in the embodiment of the application.

[**0179]** In some implementations, at least two of the monitoring pipeline sections 122 are provided with the first interfaces 1221 respectively, and the at least two second interfaces 204 include at least two pressure interfaces, and if the pressure interfaces are connected to the first interfaces 1221 respectively, the blood purification device 200 is capable of monitoring the pressure parameter through the monitoring pipeline sections 122. Therefore, connection of at least two pressure interfaces for measuring the pressure parameter may be achieved through the monitoring panel 121, such that installation of the monitoring component 12 is more convenient.

[**0180]** In some implementations, the at least two second interfaces 204 include at least one pressure interface, and if the pressure interface is connected to the first interface 1221, the blood purification device 200 is capable of monitoring the pressure parameter through the monitoring pipeline sections 122. The blood purification device 200 further includes a blood leakage monitoring module, the blood leakage monitoring module is capable of monitoring, through the monitoring pipeline sections 122, whether the blood at one side of a filtration membrane of a filter 300 permeates to the other side of the filtration membrane. Therefore, connection of at least one pressure interface for measuring the pressure parameter and installation of the monitoring pipeline sections 122 for monitoring blood leakage may be achieved through the monitoring panel 121, such that installation of the monitoring component 12 is more convenient.

[**0181]** In some implementations, the monitoring pipeline sections 122 include at least one of a blood drawing pressure monitoring pipeline section 1223, a pressure monitoring pipeline section before filtering 1224, a waste liquid pressure monitoring pipeline section 1225 and a blood leakage monitoring pipeline section 1226.

[**0182]** The blood drawing pressure monitoring pipeline section 1223 is configured to measure the pressure parameter of the blood drawn out of a patient's blood vessel (hereinafter, referred to as a blood drawing pressure simply). Therefore, the blood purification device 200 may determine whether the blood is drawn out of the patient's blood vessel normally, by monitoring the blood drawing pressure.

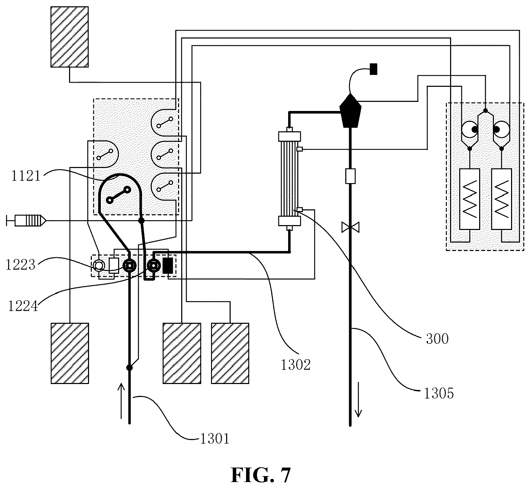

[**0183]** With continuous reference to FIG. 7, for example, the pump pipeline sections 112 include a blood pump pipeline section 1121, the blood pump pipeline section 1121 is configured to cooperate with the blood pump 2031 to drive the blood to flow. The panel kit 100 further includes a blood drawing flow path 1301, the blood drawing flow path 1301 is configured to connect an input end of the blood pump pipeline section 1121 to the patient's blood vessel, and the blood drawing flow path 1301 is provided with the blood drawing pressure monitoring pipeline section 1223.

[**0184]** Therefore, when the blood pump 2031 operates, the blood pump pipeline section 1121 may drive the blood from the patient's blood vessel to flow into the blood drawing flow path 1301 and then flow through the blood drawing pressure monitoring pipeline section 1223 when the blood travels along the blood drawing flow path 1301, such that the blood drawing pressure is monitored in the blood drawing pressure monitoring pipeline section 1223.

[**0185]** The pressure monitoring pipeline section before filtering 1224 is configured to measure a pressure parameter of blood flowing from the pump pipeline sections 112 into the filter 300 (hereinafter, referred to as a pressure before filtering simply). Therefore, whether the blood may flow into the filter 300 normally may be determined by measuring the pressure before filtering. For example, when the pressure before filtering is too high, there may be a situation where blockage, coagulation or the like occurs to the filter 300.

[**0186]** Exemplarily, the pump pipeline sections 112 include a blood pump pipeline section 1121, the blood pump pipeline section 1121 is configured to cooperate with the blood pump 2031 to drive the blood to flow, the panel kit 100 further includes a blood pumping flow path 1302, the blood pumping flow path 1302 is connected to an output end of the blood pump pipeline section 1121 and a blood input end of the filter 300 respectively, and the blood pumping flow path 1302 is provided with the pressure monitoring pipeline section before filtering 1224.

[**0187]** Therefore, when the pump component 203 operates, the blood from the patient's blood vessel may flow into the filter 300 by passing through the blood drawing flow path 1301, the blood pump pipeline section 1121 and the blood pumping flow path 1302 sequentially, such that the blood flowing into the filter 300 may flow through the pressure monitoring pipeline section before filtering 1224.

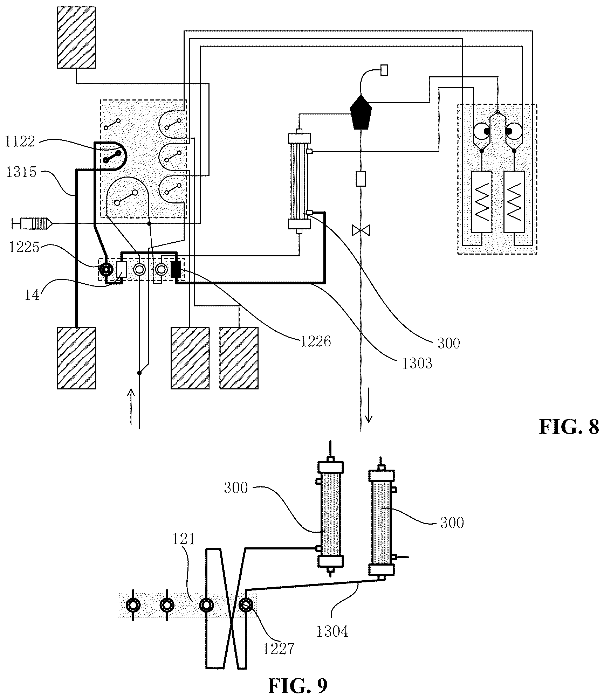

[**0188]** With continuous reference to FIG. 8, the waste liquid pressure monitoring pipeline section 1225 is configured to measure a pressure parameter of the medical liquid discharged from the filter 300 (hereinafter, referred to as a waste liquid pressure simply). Therefore, whether the filtration membrane of the filter 300 is blocked may be determined by measuring the waste liquid pressure.

[**0189]** Exemplarily, the pump pipeline sections 112 include a waste liquid pressure pump pipeline section 1122, the waste liquid pressure pump pipeline section 1122 is configured to cooperate with the medical liquid pump 2032 to drive the medical liquid to discharge from the filter 300, the panel kit 100 further includes a waste liquid flow path 1303, the waste liquid flow path 1303 is connected to a medical liquid output end of the filter 300 and an input end of the waste liquid pressure pump pipeline section 1122 respectively, and the waste liquid flow path 1303 is provided with the waste liquid pressure monitoring pipeline section 1225.

[**0190]** Therefore, when the corresponding medical liquid pump 2032 operates, the medical liquid in the filter 300 may flow along the waste liquid flow path 1303 to the waste liquid pressure pump pipeline section 1122, to flow through the waste liquid pressure monitoring pipeline section 1225.

[**0191]** It should be noted here that compared to arranging the waste liquid pressure monitoring pipeline section 1225 downstream of the waste liquid pressure pump pipeline section 1122, in the embodiment of the application, the waste liquid pressure monitoring pipeline section 1225 is arranged between the waste liquid pressure pump pipeline section 1122 and the filter 300, which may avoid that other faults downstream of the waste liquid pressure pump pipeline section 1122 interfere with the waste liquid pressure monitoring pipeline section 1225 monitoring whether the filtration membrane of the filter 300 is blocked.

[**0192]** The blood leakage monitoring pipeline section 1226 is configured to cooperate with the blood leakage monitoring module of the blood purification device 200, so as to monitor whether the blood at one side of the filtration membrane of the filter 300 permeates to the other side of the filtration membrane. Therefore, the blood leakage monitoring module may determine whether the filtration membrane of the filter 300 is damaged, causing the blood to permeate the filtration membrane.

[**0193]** Exemplarily, the pump pipeline sections 112 include a waste liquid pressure pump pipeline section 1122, the waste liquid pressure pump pipeline section 1122 is configured to cooperate with the medical liquid pump 2032 to drive the medical liquid to discharge from the filter 300. The panel kit 100 further includes a waste liquid flow path 1303 and a collection flow path 1315, the waste liquid flow path 1303 is connected to a medical liquid output end of the filter 300 and an input end of the waste liquid pressure pump pipeline section 1122 respectively, an input end of the collection flow path 1315 is connected to an output end of the waste liquid pressure pump pipeline section 1122, and the blood leakage monitoring pipeline section 1226 is arranged in the waste liquid flow path 1303 or the collection flow path 1315.

[**0194]** Therefore, when the corresponding medical liquid pump 2032 operates, the medical liquid in the filter 300 may be collected by an external waste liquid collection container after flowing through the waste liquid flow path 1303, the waste liquid pressure pump pipeline section 1122 and the collection flow path 1315 sequentially. In this process, monitoring may be performed through the blood leakage monitoring pipeline section 1226 on the waste liquid flow path 1303 or the collection flow path 1315.

[**0195]** In some implementations, the blood leakage monitoring module on the blood purification device 200 may include a photoelectric sensor such as a color sensor, to determine whether there is a blood composition by detecting color of the medical liquid discharged from the filter 300.

[**0196]** Correspondingly, at least a portion of the blood leakage monitoring pipeline section 1226 may be made of a light-shielding material, or the blood leakage monitoring pipeline section 1226 may be provided with a light-shielding member to prevent ambient light from affecting a detection result of the blood leakage monitoring module.

[**0197]** Of course, the blood leakage monitoring module may be any other modules for detecting blood compositions, which is not limited in the embodiment of the application.

[**0198]** It may also be understood that an input end of the blood leakage monitoring pipeline section 1226 may face downward, such that the medical liquid flows through the blood leakage monitoring pipeline section 1226 from bottom to top, thereby avoiding that bubbles are accumulated when the medical liquid flows through the blood leakage monitoring pipeline section 1226 from top to bottom and affect the detection result of the blood leakage monitoring module. Of course, in some other implementations, the input end of the blood leakage monitoring pipeline section 1226 may also face other directions, which is not limited in the embodiment of the application.

[**0199]** Similarly, an input end of at least one of the monitoring pipeline sections 122 may face downward, such that the corresponding medical liquid or blood flows through the monitoring pipeline section 122 from bottom to top, thereby avoiding that bubbles are accumulated in the monitoring pipeline section 122 and affect the detection result.

[**0200]** Optionally, in some other implementations, the panel kit 100 includes a waste liquid branch, the waste liquid branch is configured to be communicated with the medical liquid output end of the filter 300. The waste liquid branch is provided with the blood leakage monitoring pipeline section 1226, the waste liquid branch may allow the medical liquid flowing out of the filter 300 to flow through the blood leakage monitoring pipeline section 1226, the blood purification device 200 further includes a blood leakage monitoring module, and the blood leakage monitoring module is capable of monitoring, through the blood leakage monitoring pipeline section 1226, whether the blood at one side of the filtration membrane of the filter 300 permeates to the other side of the filtration membrane.

[**0201]** For example, the waste liquid branch includes the waste liquid flow path 1303 and the collection flow path 1315. Therefore, the blood leakage monitoring pipeline section 1226 may be arranged in the waste liquid flow path 1303 or the collection flow path 1315, which is not limited in the embodiment of the application.

[**0202]** In some implementations, the waste liquid branch may allow the medical liquid flowing out of the filter 300 to flow through the blood leakage monitoring pipeline section 1226 from bottom to top, thereby avoiding that bubbles are accumulated in the blood leakage monitoring pipeline section 1226 and affect the detection result.

[**0203]** It may also be understood that the blood leakage monitoring pipeline section 1226 may be arranged on the monitoring panel 121, to be used as one of the monitoring pipeline sections 122; or, the blood leakage monitoring pipeline section 1226 may not be arranged on the monitoring panel 121, for example, the blood leakage monitoring pipeline section 1226 may be arranged between the power panel 111 and the monitoring panel 121, which is not limited in the embodiment of the application.

[ **0204]** Exemplarily, the blood leakage monitoring pipeline section 1226 may be arranged in the waste liquid flow path 1303 and located between the input end of the waste liquid pressure pump pipeline section 1122 and an output end of the waste liquid pressure monitoring pipeline section 1225.

[**0205]** Therefore, on one hand, it may avoid that it needs to reserve a large space on the monitoring panel 121 to mount the blood leakage monitoring pipeline section 1226, therefore it is beneficial for miniaturization design of the monitoring panel 121; on the other hand, it may also reduce the number of the monitoring pipeline sections 122 on the monitoring panel 121, such that it is unnecessary for pipelines of the panel kit 100 to bend multiple times on the monitoring panel 121 to configure more monitoring pipeline sections 122, therefore it may not only reduce difficulty of manufacturing the panel kit 100, but also allow the corresponding blood or medical liquid to flow more smoothly in the panel kit 100.

[**0206]** Furthermore, when an input end of the waste liquid pressure monitoring pipeline section 1225 also faces downward, the input end of the blood leakage monitoring pipeline section 1226 may also face downward, to be connected to the output end of the waste liquid pressure monitoring pipeline section 1225 arranged to face upward, thereby avoiding that bubbles are accumulated when the medical liquid flows through the blood leakage monitoring pipeline section 1226 from top to bottom and affect the detection result of the blood leakage monitoring module.

[**0207]** In some implementations, the monitoring component 12 includes at least two monitoring components, any two of the monitoring components 12 are arranged separately from each other, such that each of the monitoring components 12 can be assembled and disassembled separately, and each of the monitoring components 12 is provided with at least two of the blood leakage monitoring pipeline section 1226, the blood drawing pressure monitoring pipeline section 1223, the pressure monitoring pipeline section before filtering 1224 and the waste liquid pressure monitoring pipeline section 1225. Therefore, with the at least two monitoring components 12, the panel kit 100 may be adapted to installation requirements of more different types of blood purification devices 200.

[**0208]** Exemplarily, one of the monitoring components 12 may be provided with the blood drawing pressure monitoring pipeline section 1223 and the pressure monitoring pipeline section before filtering 1224, and the other of the monitoring components 12 may be provided with the blood leakage monitoring pipeline section 1226 and the waste liquid pressure monitoring pipeline section 1225, which is not limited in the embodiment of the application.

[**0209]** With continuous reference to FIG. 9, in some implementations, the monitoring pipeline sections 122 further include a secondary membrane pressure monitoring pipeline section 1227, the secondary membrane pressure monitoring pipeline section 1227 is configured to communicate a blood output end of one filter 300 with a blood input end of another filter 300, and the secondary membrane pressure monitoring pipeline section 1227 is configured to measure the pressure parameter of the blood.

[**0210]** For example, the panel kit 100 may further include a connection flow path 1304, the connection flow path 1304 is communicated with the blood output end of one filter 300 and the blood input end of another filter 300 respectively, and the secondary membrane pressure monitoring pipeline section 1227 is arranged in the connection flow path 1304.

[**0211]** Correspondingly, when the monitoring component 12 includes at least two monitoring components, any two of the monitoring components 12 are arranged separately from each other, such that each of the monitoring components 12 can be assembled and disassembled separately, then each of the monitoring components 12 is provided with at least two of the blood leakage monitoring pipeline section 1226, the blood drawing pressure monitoring pipeline section 1223, the pressure monitoring pipeline section before filtering 1224, the waste liquid pressure monitoring pipeline section 1225 and the secondary membrane pressure monitoring pipeline section 1227. Therefore, with the at least two monitoring components 12, the panel kit 100 may be adapted to installation requirements of more different types of blood purification devices 200.

[**0212]** In some implementations, the input end of the collection flow path 1315 is connected to the output end of the waste liquid pressure pump pipeline section 1122, and an output end of the collection flow path 1315 is configured to be connected to the secondary membrane pressure monitoring pipeline section 1227. Therefore, the medical liquid in the filter 300 may flow along the waste liquid flow path 1303, the waste liquid pressure pump pipeline section 1122 and the collection flow path 1315 sequentially, and then flow into the secondary membrane pressure monitoring pipeline section 1227.

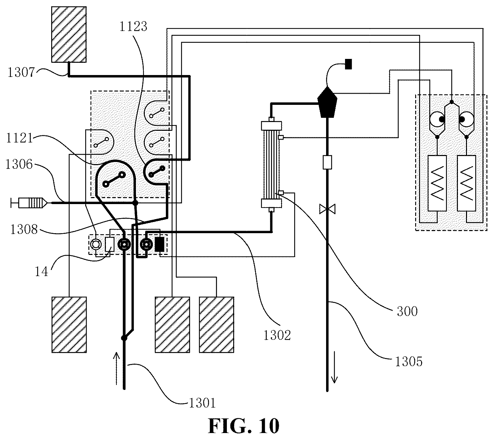

[**0213]** With continuous reference to FIG. 10, in some implementations, the panel kit 100 further includes an electrostatic discharge (ESD) element 14, the ESD element 14 is configured to cooperate with the blood purification device 200 to discharge static electricity from the panel kit 100. It may be understood that static electricity may also be generated when the pump component 203 drives the pump pipeline sections 112 to move, then the static electricity is discharged by the ESD element 14, to avoid interference with other devices used simultaneously, such as an electrocardiograph (ECG) monitor.

[**0214]** In some implementations, the ESD element 14 is arranged on at least one of the power panel 111 or the monitoring panel 121, such that with the ESD element 14 integrated into the corresponding power panel 111 and/or monitoring panel 121, adaptation of the ESD element 14 to the blood purification device 200 may be achieved synchronously through installation of the corresponding power panel 111 and/or monitoring panel 121, thereby facilitating installation of the panel kit 100.

[**0215]** Of course, in some other implementations, it is also possible that the ESD element 14 is not arranged on the power panel 111 and is not arranged on the monitoring panel 121, which is not limited in the embodiment of the application.

[**0216]** In some implementations, the blood purification device 200 is provided with a conductive member, and the ESD element 14 is configured to be connected to the conductive member to discharge static electricity. For example, the ESD element 14 may come into contact with the conductive member to form electric connection, such that the ESD element 14 is grounded through the conductive member.

[**0217]** In some implementations, a portion of pipeline sections of the panel kit 100 is made of a conductive material to form the ESD element 14. Of course, the ESD element 14 may also be a conductive element connected to one of the pipeline sections of the panel kit 100, which is not limited in the embodiment of the application.

[**0218]** In some implementations, the pump pipeline sections 112 include the blood pump pipeline section 1121. The panel kit 100 further includes the blood drawing flow path 1301, the blood pumping flow path 1302 and a blood return flow path 1305. An input end of the blood drawing flow path 1301 is configured to be connected to a blood drawing site of the patient's blood vessel, and an output end of the blood drawing flow path 1301 is connected to the input end of the blood pump pipeline section 1121. An input end of the blood pumping flow path 1302 is connected to the output end of the blood pump pipeline section 1121, and an output end of the blood pumping flow path 1302 is configured to be connected to the blood input end of the filter 300. An input end of the blood return flow path 1305 is configured to be connected to a blood output end of the filter 300, and an output end of the blood return flow path 1305 is configured to be connected to a blood return site of the patient's blood vessel.

[**0219]** Therefore, during operation of the blood purification device 200, when the pump component 203 (such as the blood pump 2031 of the pump component 203) operates, the blood pump pipeline section 1121 drives the blood to flow through the blood drawing flow path 1301, the blood pump pipeline section 1121, the blood pumping flow path 1302, the filter 300 and the blood return flow path 1305 sequentially from the blood drawing site of the patient's blood vessel, and then flow back to the patient's blood vessel from the blood return site of the patient's blood vessel, thereby achieving treatment of the patient with extracorporeal blood circulation.

[**0220]** In some implementations, the panel kit 100 further includes an injection flow path 1306, an input end of the injection flow path 1306 is configured to be connected to an anticoagulant drug syringe pump, and an output end of the injection flow path 1306 is connected to the blood drawing flow path 1301 or the blood pumping flow path 1302. Therefore, the anticoagulant drug syringe pump provided on the blood purification device 200 may inject the anticoagulant drug into the blood in the blood drawing flow path 1301 or the blood pumping flow path 1302 through the injection flow path 1306, to avoid coagulation of the blood in the blood drawing flow path 1301 or the blood pumping flow path 1302.

[**0221]** The anticoagulant drug may include heparin, citric acid or the like, which is not limited in the embodiment of the application.

[**0222]** It may also be understood that when the injection flow path 1306 is connected to the blood pumping flow path 1302, it may avoid that the pump component 203 generates a large negative pressure in the blood drawing flow path 1301 when the pump component 203 operates, to suck the anticoagulant drug out of the anticoagulant drug syringe pump to a certain extent.

[**0223]** In some implementations, the pump pipeline sections 112 further include a pump pipeline section of a front pump that is before the blood pump 1123, the panel kit 100 includes a first liquid inlet flow path 1307 and a second liquid inlet flow path 1308, the first liquid inlet flow path 1307, the pump pipeline section of the front pump 1123 and the second liquid inlet flow path 1308 are connected sequentially, an input end of the first liquid inlet flow path 1307 is at least configured to be connected to an anticoagulant drug supply container, and an output end of the second liquid inlet flow path 1308 is connected to the blood drawing flow path 1301, the blood pumping flow path 1302 or the blood return flow path 1305. Therefore, the blood purification device 200 may drive the pump pipeline section of the front pump 1123 through the pump component 203 (such as the medical liquid pump 2032 in the pump component 203) to at least allow the anticoagulant drug in the anticoagulant drug supply container to flow into the blood drawing flow path 1301, the blood pumping flow path 1302 or the blood return flow path 1305 after passing through the first inlet flow path 1307, the pump pipeline section of the front pump 1123 and the second inlet flow path 1308 sequentially, so as to avoid coagulation of the blood in the blood drawing flow path 1301, the blood pumping flow path 1302 or the blood return flow path 1305.

[**0224]** It may be understood that when the panel kit 100 is provided with the injection flow path 1306, the first liquid inlet flow path 1307, the pump pipeline section of the front pump 1123 and the second liquid inlet flow path 1308 simultaneously, it is possible that the anticoagulant drug syringe pump connected to the injection flow path 1306 and the anticoagulant drug supply container connected to the first liquid inlet flow path 1307 are configured to supply different anticoagulant drugs. For example, the anticoagulant drug syringe pump is configured to supply heparin, and the anticoagulant drug supply container is configured to supply citric acid, to meet different anticoagulant requirements during extracorporeal blood purification.

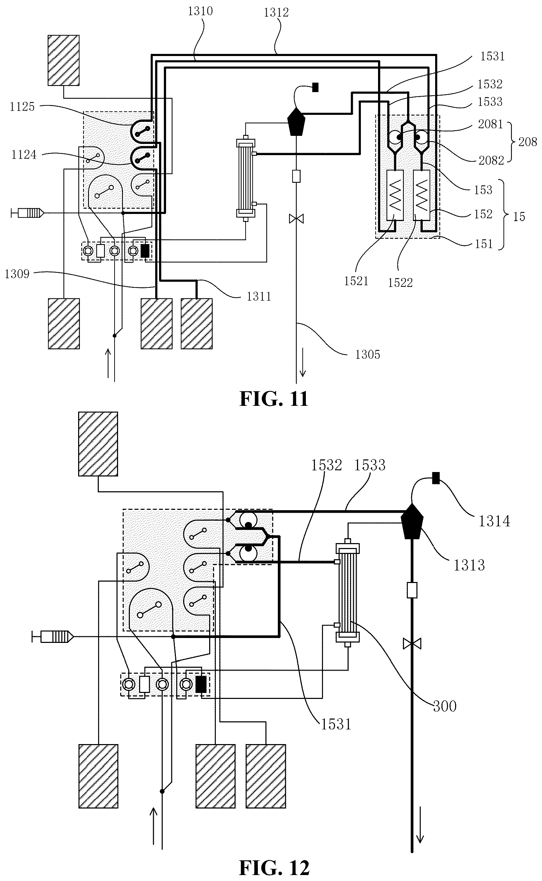

[**0225]** With continuous reference to FIG. 11, in some implementations, the pump pipeline sections 112 include a dialysate pump pipeline section 1124, and the panel kit 100 further includes a third liquid inlet flow path 1309 and a fourth liquid inlet flow path 1310, the third liquid inlet flow path 1309, the dialysate pump pipeline section 1124 and the fourth liquid inlet flow path 1310 are connected sequentially, an input end of the third liquid inlet flow path 1309 is configured to be connected to a dialysate supply container, and an output end of the fourth liquid inlet flow path 1310 is configured to be connected to a medical liquid input end of the filter 300.

[**0226]** Therefore, when the pump component 203 (such as the medical liquid pump 2032 corresponding to the pump component 203) on the blood purification device 200 operates, the pump component 203 may drive dialysate in the dialysate supply container through the dialysate pump pipeline section 1124, to flow into the filter 300 by passing through the third liquid inlet flow path 1309, the dialysate pump pipeline section 1124, the fourth liquid inlet flow path 1310 and the medical liquid input end of the filter 300 sequentially.

[**0227]** In some implementations, the pump pipeline sections 112 further include a replacement fluid pump pipeline section 1125, the panel kit 100 further includes a fifth liquid inlet flow path 1311 and a sixth liquid inlet flow path 1312, the fifth liquid inlet flow path 1311, the replacement fluid pump pipeline section 1125 and the sixth liquid inlet flow path 1312 are connected sequentially or connected sequentially and directly, an input end of the fifth liquid inlet flow path 1311 is configured to be connected to a replacement fluid supply container, and an output end of the sixth liquid inlet flow path 1312 is configured to be connected to the blood drawing flow path 1301, the blood pumping flow path 1302 or the blood return flow path 1305.

[**0228]** Therefore, when the pump component 203 (such as the medical liquid pump 2032 corresponding to the pump component 203) on the blood purification device 200 operates, the pump component 203 may drive a replacement fluid in the replacement fluid supply container through the replacement fluid pump pipeline section 1125, to flow into the blood drawing flow path 1301, the blood pumping flow path 1302 or the blood return flow path 1305 after passing through the fifth liquid inlet flow path 1311, the replacement fluid pump pipeline section 1125 and the sixth liquid inlet flow path 1312 sequentially.

[**0229]** The replacement fluid may include a commercially available replacement fluid, such as an online replacement fluid produced on-line by a hemodiafiltration (HDF) machine, a manually prepared replacement fluid or the like, which is not limited in the embodiment of the application.

[**0230]** In some implementations, the panel kit 100 further includes a heating component 15, the heating component 15 is configured to be mounted on a heating region 207 of the device housing 201 such that the medical liquid and/or the blood in the heating component 15 is heated by a heating unit of the blood purification device 200. Therefore, heating requirements of the medical liquid and/or the blood may be met. For example, the medical liquid may be heated to prevent the medical liquid with a too low temperature from affecting the blood, for example, the medical liquid with a too low temperature causes a too low temperature of the blood flowing back to a human body, to cause discomfort of the human.

[**0231]** The heating component 15 may include a heating panel 151 and a heating container 152. The heating panel 151 is configured to be detachably mounted on the heating region 207 of the device housing 201. The heating container 152 is mounted to the heating panel 151 so as to be mounted on the heating region 207 by the heating panel 151, such that the heating container 152 may be conveniently mounted to the device housing 201 by the heating panel 151.

[**0232]** It may be understood that the heating container 152 may include a coil-type heating container, a bladder-type heating container, a bottle-type heating container or the like, which is not limited in the embodiment of the application.

[**0233]** The heating component 15 may further include a liquid outlet flow path 153. An input end of the liquid outlet flow path 153 is communicated with an output end of the heating container 152. The input end of the liquid outlet flow path 153 may be mounted to the heating panel 151. Therefore, the heating container 152, the input end of the liquid outlet flow path 153 or the like may be conveniently mounted to the blood purification device 200 through the heating panel 151.

[**0234]** In some implementations, the pump pipeline sections 112 include the dialysate pump pipeline section 1124, the heating container 152 includes a first heating container 1521, an input end connected to the first heating container 1521 is connected to the output end of the fourth liquid inlet flow path 1310, the liquid outlet flow path 153 connected to the first heating container 1521 includes a first liquid outlet flow path 1531 and a second liquid outlet flow path 1532, the first liquid outlet flow path 1531 is connected to the blood return flow path 1305, and the second liquid outlet flow path 1532 is connected to the medical liquid input end of the filter 300.

[**0235]** Therefore, during actual usage, the input end of the third liquid inlet flow path 1309 may be selectively connected to the dialysate supply container or the replacement fluid supply container, such that the blood purification device 200 may meet more different use requirements.

[**0236]** For example, when the input end of the third liquid inlet flow path 1309 is connected to the dialysate supply container, the first liquid outlet flow path 1531 may be controlled to open and the second liquid outlet flow path 1532 may be controlled to be in communication by a first pipeline switching unit 2081 on the blood purification device 200, such that the dialysate in the dialysate supply container flows into the filter 300 after passing through the third liquid inlet flow path 1309, the dialysate pump pipeline section 1124, the fourth liquid inlet flow path 1310, the first heating container 1521, the second liquid outlet flow path 1532 and the medical liquid input end of the filter 300 sequentially.

[**0237]** Alternatively, when the input end of the third liquid inlet flow path 1309 is connected to the replacement fluid supply container, the first liquid outlet flow path 1531 may be controlled to be in communication and the second liquid outlet flow path 1532 may be controlled to open by the first pipeline switching unit 2081 on the blood purification device 200, such that the replacement fluid in the replacement fluid supply container flows into the blood return flow path 1305 after passing through the third liquid inlet flow path 1309, the dialysate pump pipeline section 1124, the fourth liquid inlet flow path 1310, the first heating container 1521 and the first liquid outlet flow path 1531 sequentially.

[**0238]** In some implementations, the pump pipeline sections 112 include the replacement fluid pump pipeline section 1125, the heating container 152 includes a second heating container 1522, an input end connected to the second heating container 1522 is connected to the output end of the sixth liquid inlet flow path 1312, the liquid outlet flow path 153 connected to the second heating container 1522 includes the first liquid outlet flow path 1531 and a third liquid outlet flow path 1533, the first liquid outlet flow path 1531 is connected to the blood return flow path 1305, and the third liquid outlet flow path 1533 is connected to the blood drawing flow path 1301 or the blood pumping flow path 1302.

[**0239]** Therefore, during actual usage, the replacement fluid in the second heating container 1522 may selectively flow into the blood return flow path 1305, the blood drawing flow path 1301 or the blood pumping flow path 1302.

[**0240]** Exemplarily, the first liquid outlet flow path 1531 may be controlled to be in communication and the third liquid outlet flow path 1533 may be controlled to open by a second pipeline switching unit 2082 on the blood purification device 200, such that the replacement fluid in the replacement fluid supply container flows to the blood return flow path 1305 after passing through the fifth liquid inlet flow path 1311, the replacement fluid pump pipeline section 1125, the sixth liquid inlet flow path 1312, the second heating container 1522 and the first liquid outlet flow path 1531 sequentially, thereby achieving that the replacement fluid is injected into the blood filtered by the filter 300.

[**0241]** Alternatively, the first liquid outlet flow path 1531 may be controlled to open and the third liquid outlet flow path 1533 may be controlled to be in communication by the second pipeline switching unit 2082 on the blood purification device 200, such that the replacement fluid in the replacement fluid supply container flows to the blood drawing flow path 1301 or the blood pumping flow path 1302 after passing through the fifth liquid inlet flow path 1311, the replacement fluid pump pipeline section 1125, the sixth liquid inlet flow path 1312, the second heating container 1522 and the third liquid outlet flow path 1533 sequentially, thereby achieving that the replacement fluid is injected into the blood that has not been filtered by the filter 300.

[**0242]** In some implementations, both the power panel 111 and the monitoring panel 121 are arranged separately from the heating panel 151, such that the heating panel 151 may be assembled and disassembled independently relative to the power panel 111, and the heating panel 151 may be assembled and disassembled independently relative to the monitoring panel 121.

[**0243]** With continuous reference to FIG. 12, in some implementations, it is also possible that an output end of the first liquid outlet flow path 1531 is connected to the blood drawing flow path 1301 or the blood pumping flow path 1302, an output end of the second liquid outlet flow path 1532 is connected to the medical liquid input end of the filter 300, and an output end of the third liquid outlet flow path 1533 is connected to the blood return flow path 1305.

[**0244]** Therefore, the replacement fluid in the replacement fluid supply container may be selectively injected into the blood drawing flow path 1301 or the blood pumping flow path 1302 through the first liquid outlet flow path 1531, or injected into the blood return flow path 1305 through the third liquid outlet flow path. Furthermore, the input end of the third liquid inlet flow path 1309 may be selectively connected to the dialysate supply container and injected into the filter 300 through the second liquid outlet flow path 1532, or the input end of the third liquid inlet flow path 1309 may be selectively connected to the replacement fluid supply container and injected into the blood drawing flow path 1301 or the blood pumping flow path 1302 through the first liquid outlet flow path 1531.

[**0245]** In some implementations, it is also possible that the panel kit 100 is not provided with the heating panel 151 and the heating container 152. For example, the output end of the fourth liquid inlet flow path 1310 is connected to an input end of the first liquid outlet flow path 1531 and an input end of the second liquid outlet flow path 1532 respectively through a three-way joint or the like, and the output end of the sixth liquid inlet flow path 1312 is connected to the input end of the first liquid outlet flow path 1531 and an input end of the third liquid outlet flow path 1533 respectively through a three-way joint or the like.

[**0246]** At this time, the input end of the first liquid outlet flow path 1531, the input end of the second liquid outlet flow path 1532 and the input end of the third liquid outlet flow path 1533 may be mounted to the power panel 111.

[**0247]** In some implementations, the blood return flow path 1305 is provided with a degassing pot 1313 and a degassing pot monitoring interface 1314, the degassing pot monitoring interface 1314 is communicated with the degassing pot 1313, and if the degassing pot monitoring interface 1314 is connected to a degassing pot monitoring element 209 on the blood purification device 200, the degassing pot monitoring interface 1314 is used for the blood purification device 200 to monitor a pressure in the degassing pot 1313. For example, the degassing pot monitoring interface 1314 may include a Luer connector.

[**0248]** In some implementations, each of the pump pipeline sections 112 is arc-shaped. Therefore, the pump pipeline section 112 may be wound around the pump head of the pump component 203 more conveniently through its arc-shaped structure.

[**0249]** In some implementations, openings formed at two ends of each of the pump pipeline sections 112 face toward an outer circumferential side of the power panel 111, therefore two ends of each of the pump pipeline sections 112 may be connected to other corresponding flow paths in the panel kit 100 conveniently by two ends of each of the pump pipeline sections 112 facing outward.

[**0250]** Optionally, openings formed at two ends of at least a portion of the pump pipeline sections 112 face an inner circumferential side of the power panel 111, which is not limited in the embodiment of the application.

[**0251]** The above descriptions are introductions of some other structures of the panel kit 100 in the embodiments of the application, and introductions will be continuously made below with reference to some other structures of the blood purification device 200.

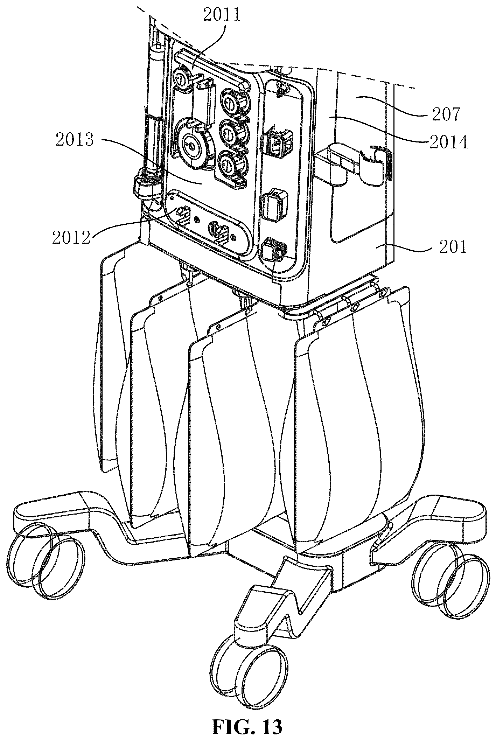

[**0252]** With continuous reference to FIG. 13, in some implementations, the device housing 201 is provided with a first surface 2013, each of the parameter region 2012 and the power region 2011 is arranged on the first surface 2013, and the parameter region 2012 is located at a lower side of the power region 2011 along a vertical direction.

[**0253]** Therefore, compared to arranging the parameter region 2012 and the power region 2011 on different surfaces of the device housing 201, in the embodiment of the application, the parameter region 2012 and the power region 2011 are arranged on the same surface of the device housing 201 collectively, such that a distance between the monitoring component 12 and the power component 11 mounted to the device housing 201 and the power component 11 may be closer, and thus pipeline sections connected between the power component 11 and the monitoring component 12 may be shorter. Furthermore, with the parameter region 2012 arranged at the lower side of the power region 2011, the second interfaces 204 may also be arranged below the power region 2011 collectively, corresponding monitoring pipeline sections 122 on the monitoring panel 121 may also be arranged collectively, and installation of the monitoring pipeline sections 122 is facilitated.

[**0254]** In some implementations, the device housing 201 is further provided with a second surface 2014, the second surface 2014 faces toward a direction different from the first surface 2013, the second surface 2014 is located at a side of the first surface 2013 along a horizontal direction, the second surface 2014 is provided with a heating region 207, a heating component 15 of the panel kit 100 is configured to be detachably mounted on the heating region 207. If the heating component 15 is mounted on the heating region 207, the medical liquid and/or the blood in the heating component 15 are capable of being heated by the blood purification device 200 (such as the heating unit on the blood purification device 200).

[**0255]** Therefore, compared to further arranging the heating region 207 on the first surface 2013, in the embodiment of the application, the second surface 2014 of the device housing 201 may be fully utilized to arrange the heating region 207, to avoid an excessive large area of the first surface 2013, therefore it is beneficial for miniaturization design of the blood purification device 200.

[**0256]** It may also be understood that the first surface 2013 may be a front surface of the device housing 201 (that is, a side facing toward the user during usage) and the second surface 2014 may be a side surface of the device housing 201 (such as a left side surface or a right side surface). Of course, in some other implementations, it is also possible that the first surface 2013 is a side surface of the device housing 201 and the second surface 2014 is a front surface of the device housing 201, which is not limited in the embodiment of the application.

[**0257]** In some implementations, the heating component 15 is provided with multiple liquid flow paths, the liquid flow paths are configured to deliver the medical liquid. The blood purification device 200 further includes a pipeline switching unit 208, the pipeline switching unit 208 is arranged on the device housing 201, and if the heating component 15 is mounted on the heating region 207, the pipeline switching unit 208 is capable of controlling on/off of at least two of the liquid flow paths.

[**0258]** For example, the liquid flow paths may include the first liquid outlet flow path 1531, the second liquid outlet flow path 1532 and the third liquid outlet flow path 1533 as described above or the like, and the pipeline switching unit 208 may include the first pipeline switching unit 2081 and/or the second pipeline switching unit 2082 correspondingly.

[**0259]** Therefore, on one hand, installation of at least two of the liquid flow paths may be assisted by the heating panel 151, such that the panel kit 100 has advantages of convenient installation; on the other hand, with the pipeline switching unit 208, the blood purification device 200 may meet more different extracorporeal blood purification treatment scenarios.

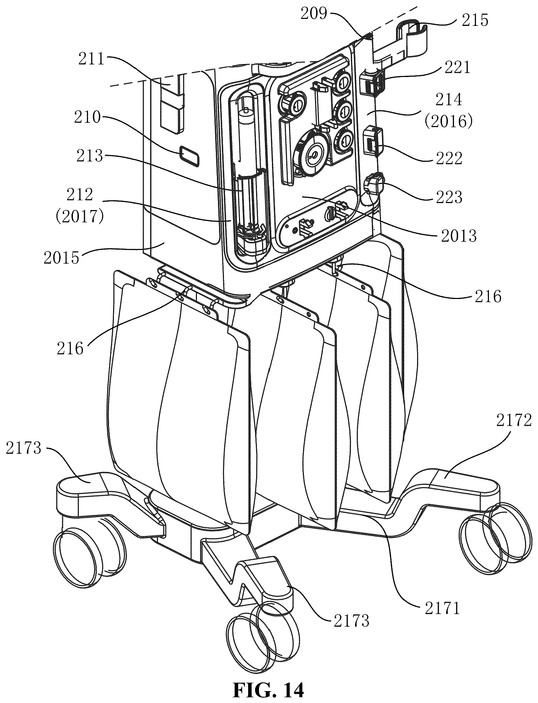

[**0260]** With continuous reference to FIG. 14, in some implementations, the device housing 201 is further provided with a third surface 2015, the third surface 2015 and the second surface 2014 are located at opposite sides of the first surface 2013. The blood purification device 200 further includes at least one of a blood composition analysis component 210 and an infusion pump 211, the blood composition analysis component 210 is configured to detect blood compositions in a blood sample, the blood composition analysis component 210 is provided with a blood composition analysis detection interface, the blood composition analysis detection interface is arranged on the third surface 2015, and the infusion pump 211 is arranged on the third surface 2015.

[**0261]** When it is mentioned that the third surface 2015 and the second surface 2014 are located at opposite sides of the first surface 2013, it means that the third surface 2015 is located at a side opposite to the second surface 2014, the third surface 2015 and the second surface 2014 are located at two sides of the first surface 2013. For example, the first surface 2013 is located at a front side of the device housing 201, one of the third surface 2015 and the second surface 2014 is located at a left side of the device housing 201, and the other of the third surface 2015 and the second surface 2014 is located at a right side of the device housing 201.

[**0262]** It may be understood that on one hand, during operation of the blood purification device 200, the blood sample may be acquired from pipeline sections of the panel kit 100 and transferred to a blood composition detection test piece, and then the blood composition detection test piece carrying the blood sample may be inserted into the blood composition analysis detection interface to detect the blood compositions in the blood sample, such that the blood purification device 200 may control the operation of the blood purification device 200 based on the blood compositions in the blood sample. On the other hand, the infusion pump 211 may infuse the medical liquid into the pipeline sections of the panel kit 100 according to actual requirements, or may infuse the medical liquid into the patient directly, such that the blood purification device 200 according to the embodiment of the application may meet more treatment requirements.

[**0263]** In some implementations, the blood composition analysis component 210 is capable of detecting one or more of a calcium ion concentration, a sodium ion concentration, a potassium ion concentration, a chloride ion concentration and a magnesium ion concentration in the blood sample. Taking the sodium ion concentration as an example, during treatment of the patient with the blood purification device 200, for the patient suffering from hypernatremia or hyponatremia, the sodium ion concentration in the blood sample is detected by the blood composition analysis component 210, to facilitate monitoring the sodium ion concentration in the blood and facilitate finding and correcting the above various diseases timely. Similarly, during treatment of the patient with the blood purification device 200, for the patient suffering from hypernatremia, hyponatremia or other electrolyte level abnormalities, it also needs to monitor a corresponding electrolyte concentration in the blood at a regular time.

[**0264]** Furthermore, the blood composition analysis component 210 may also be configured to detect one or more of a hydrogenion concentration (PH) value, a partial pressure of carbon dioxide (PCO2) value, a partial pressure of oxygen (PO2) value, a glucose (Glu) value, a Lactate (Lac) value and a Hematocrit (Hct) value of the blood sample. With corresponding detection operations, it facilitates an operator to perform corresponding processing based on detection results, which is not limited in the embodiment of the application.

[**0265]** It may also be understood that compared to further arranging the blood composition analysis component 210 and the infusion pump 211 on the first surface 2013, in the embodiment of the application, the third surface 2015 of the device housing 201 may be fully utilized to arrange the blood composition analysis component 210 and the infusion pump 211, to avoid an excessive large area of the first surface 2013, therefore it is beneficial for miniaturization design of the blood purification device 200.

[**0266]** In some implementations, the blood purification device 200 further includes a syringe pump mounting region 212, a syringe pump 213 (such as the above anticoagulant drug syringe pump) is configured to be mounted on the syringe pump mounting region 212, and the syringe pump 213 is configured to inject the medical liquid into the panel kit 100. Therefore, the syringe pump 213 may be mounted to the blood purification device 200 more stably.

[**0267]** In some implementations, the blood purification device 200 further includes a safety monitoring region 214, at least two of a degassing pot fixing seat 221, a degassing pot monitoring element 209, a bubble monitoring element 222 and a liquid stopping clamp 223 are arranged on the safety monitoring region 214, a degassing pot 1313 of the panel kit 100 is configured to be detachably mounted and fixed to the degassing pot fixing seat 221, the bubble monitoring element 222 is configured to monitor whether there are bubbles in a downstream pipeline connected to the degassing pot 1313, and the liquid stopping clamp 223 is configured to control on/off of the downstream pipeline connected to the degassing pot 1313.

[**0268]** It may be understood that at least two of the degassing pot fixing seat 221, the degassing pot monitoring element 209, the bubble monitoring element 222 and the liquid stopping clamp 223 are arranged on the safety monitoring region 214 collectively, such that the downstream pipeline of the degassing pot 1313 in the panel kit 100 may be shorter.

[**0269]** In some implementations, the device housing 201 is further provided with a third surface 2015, a fourth surface 2016 and a fifth surface 2017; the second surface 2014, the fourth surface 2016, the first surface 2013, the fifth surface 2017 and the third surface 2015 are sequentially and directly connected along the horizontal direction, the third surface 2015 is arranged opposite to the second surface 2014, each of the third surface 2015 and the first surface 2013 is bent and connected to the fifth surface 2017, and each of the second surface 2014 and the first surface 2013 is bent and connected to the fourth surface 2016; one of the syringe pump mounting region 212 and the safety monitoring region 214 is arranged on the fourth surface 2016, and the other of the syringe pump mounting region 212 and the safety monitoring region 214 is arranged on the fifth surface 2017.

[**0270]** It may be understood that the second surface 2014, the fourth surface 2016, the first surface 2013, the fifth surface 2017 and the third surface 2015 are sequentially and directly connected along the horizontal direction, and no other surfaces are present between any two adjacent surfaces of the second surface 2014, the fourth surface 2016, the first surface 2013, the fifth surface 2017 and the third surface 2015.

[**0271]** Exemplarily, the first surface 2013, the second surface 2014, the third surface 2015, the fourth surface 2016 and the fifth surface 2017 face toward different sides in a circumferential direction of the device housing 201.

[**0272]** For example, it is possible that the first surface 2013 is a front surface of the device housing 201, the second surface 2014 is a right side surface of the device housing 201, the third surface 2015 is a left side surface of the device housing 201, the fourth surface 2016 is a first connection side surface connected between the front surface and the right side surface of the device housing 201, and the fifth surface 2017 is a second connection side surface connected between the front surface and the left side surface of the device housing 201. Therefore, with one of the syringe pump mounting region 212 and the safety monitoring region 214 arranged on the first connection side surface, and the other of the syringe pump mounting region 212 and the safety monitoring region 214 arranged on the second connection side surface, outer surfaces of the device housing 201 may be fully utilized to arrange the syringe pump mounting region 212 and the safety monitoring region 214, such that structures of the blood purification device 200 are more compact.

[**0273]** It may be understood that the fourth surface 2016 may be a flat surface, or may be a curved surface such as a convex curved surface, a concave curved surface or an irregular curved surface, or the like; the fifth surface 2017 may be a flat surface, or may be a curved surface such as a convex curved surface, a concave curved surface or an irregular curved surface, or the like, which is not limited in the embodiment of the application.

[**0274]** Optionally, in some other implementations, each of the syringe pump mounting region 212 and the safety monitoring region 214 is arranged on the first surface 2013, and the syringe pump mounting region 212 and the safety monitoring region 214 are arranged at a same side or different sides of the power region 2011, which is not limited in the embodiment of the application.

[**0275]** When each of the syringe pump mounting region 212 and the safety monitoring region 214 is arranged on the first surface 2013, the device housing 201 may not be provided with the fourth surface 2016 and the fifth surface 2017, that is, one of side edges of the first surface 2013 along the horizontal direction is directly connected to the second surface 2014, and the other of side edges of the first surface 2013 along the horizontal direction is directly connected to the third surface 2015.

[**0276]** In some implementations, the blood purification device 200 further includes a mounting arm 215, the mounting arm 215 is mounted on the second surface 2014, a filter 300 can be detachably connected to the mounting arm 215. Therefore, the filter 300 may be fixed to the blood purification device 200 more stably and reliably by the mounting arm 215.

[**0277]** Furthermore, compared to further arranging the mounting arm 215 on the first surface 2013, in the embodiment of the application, the second surface 2014 of the device housing 201 may be fully utilized to arrange the mounting arm 215, to avoid an excessive large area of the first surface 2013, therefore it is beneficial for miniaturization design of the blood purification device 200.

[**0278]** In some implementations, when the mounting arm 215 is mounted on the second surface 2014, if the heating component 15 is mounted on the heating region 207, the medical liquid in the heating component 15 is capable of being heated by the blood purification device 200 (such as the heating unit on the blood purification device 200).

[**0279]** Alternatively, in some other implementations, the mounting arm 215 is mounted on the third surface 2015, and if the heating component 15 is mounted on the heating region 207, the blood in the heating component 15 is capable of being heated by the blood purification device 200 (such as the heating unit on the blood purification device 200).

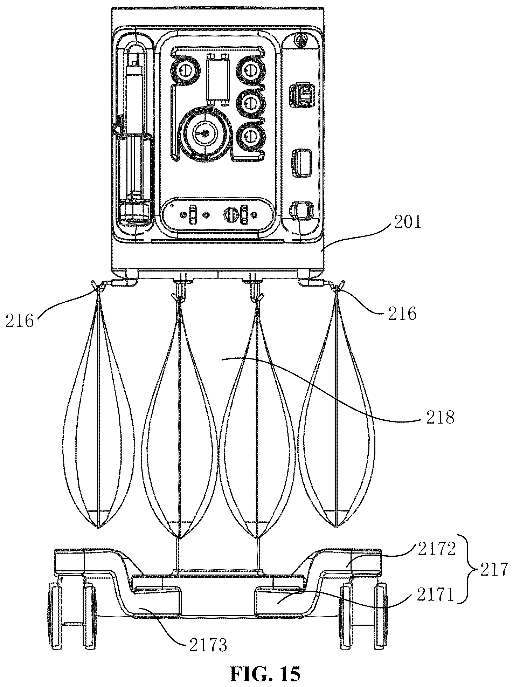

[**0280]** With continuous reference to FIG. 15, in some implementations, the blood purification device 200 further includes multiple first weighing elements 216, the first weighing elements 216 are arranged at a bottom part of the device housing 201 along a vertical direction.

[**0281]** Each of the first weighing elements 216 may be configured to monitor weight of a medical liquid container. For example, the first weighing elements 216 may be configured to monitor weights of the replacement fluid supply container, the dialysate supply container, the anticoagulant drug supply container or the like, to control supply of the replacement fluid, the dialysate, the anticoagulant drug or the like based on variations in the weights of the replacement fluid supply container, the dialysate supply container, the anticoagulant drug supply container or the like.

[**0282]** Each of the first weighing elements 216 may be configured to hang a medical liquid container to monitor weight of the medical liquid container. Therefore, each of the first weighing elements 216 may also be reused for carrying and fixing the medical liquid container. Of course, each of the first weighing elements 216 may also be configured to support a medical liquid container to monitor weight of the medical liquid container, which is not limited in the embodiment of the application.

[**0283]** In some implementations, the first weighing elements 216 may include at least three first weighing elements, and at least three side edges at the bottom part of the device housing 201 are provided with the first weighing elements 216 respectively, such that space at the bottom part of the device housing 201 may be fully utilized to provide a larger number of medical liquid containers and/or provide medical liquid containers with greater volumes.

[**0284]** In some implementations, the first weighing elements 216 may include at least four first weighing elements. Therefore, variations in the weights of the replacement fluid supply container, the dialysate supply container, the anticoagulant drug supply container and an effluent collection container, or the like may be monitored by the at least four first weighing elements 216 respectively.

[**0285]** In some implementations, at least one of the first weighing elements 216 is movably connected to the device housing 201. Therefore, during operation of the blood purification device 200, the first weighing element 216 may be moved to a suitable position for usage according to actual requirements, to meet more usage scenarios of the blood purification device 200.

[**0286]** For example, when the first weighing elements 216 include at least four first weighing elements, it is possible that the at least four first weighing elements 216 are arranged in sequence, two first weighing elements 216 located at outer sides are movably connected to the device housing 201, and other first weighing elements 216 may be movably connected to the device housing 201 or may be fixedly connected to the device housing 201.

[**0287]** Of course, in some other implementations, it is also possible that all the first weighing elements 216 are fixedly connected to the device housing 201, which is not limited in the embodiments of the application.

[**0288]** In some implementations, the blood purification device 200 further includes a base 217 and a support frame 218, the support frame 218 is mounted to the base 217 and is fixedly connected to the device housing 201, such that the device housing 201 is arranged above the base 217 along the vertical direction, and thus there is enough space below the device housing 201 to hang the medical liquid containers.

[**0289]** In some implementations, the base 217 includes an avoidance part 2171 and a distal end 2172, the distal end 2172 is located at a side of the avoidance part 2171, which side is away from the support frame 218 along a horizontal direction, a height of the avoidance part 2171 is less than that of the distal end 2172, and the avoidance part 2171 is located below at least one of the first weighing elements 216 along the vertical direction, so as to avoid a medical liquid container hung by the at least one of the first weighing elements 216 above the avoidance part 2171, such that there is enough space below the device housing 201 to hang the medical liquid containers.

[**0290]** For example, the base 217 may include multiple extension parts 2173, the multiple extension parts 2173 are arranged at intervals along an axial direction of the support frame 218. Therefore, the multiple extension parts 2173 may be arranged as extending in a radial pattern. An end of each of the extension parts 2173 is connected to the support frame 218, and another end of at least a portion of the extension parts 2173 forms the distal end 2172.

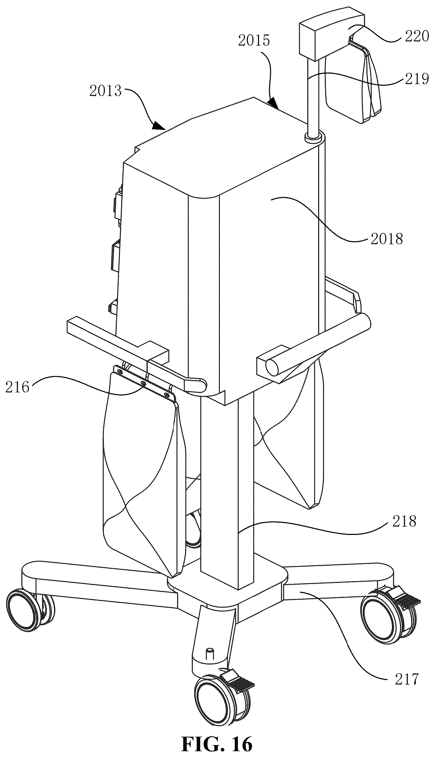

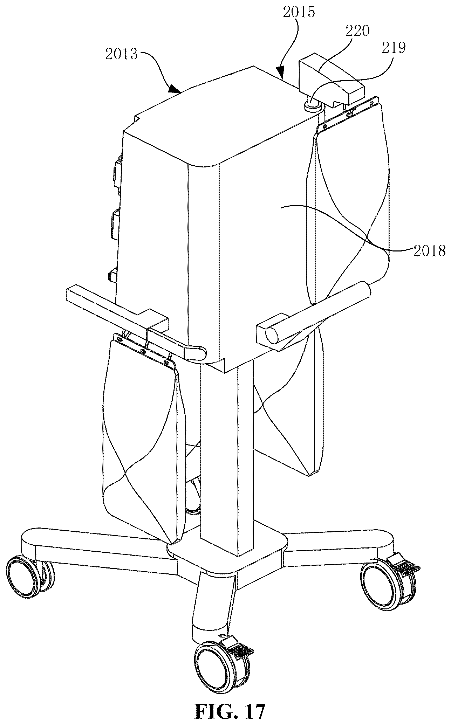

[**0291]** With continuous reference to FIG. 16 and FIG. 17, in some implementations, the blood purification device 200 further includes a weighing bar 219 and a second weighing element 220, the weighing bar 219 is liftably mounted on the device housing 201, the weighing bar 219 is adjustable to a specified height, the second weighing element 220 is arranged at an end of the weighing bar 219, which end is away from the device housing 201, and the weighing bar 219 and/or the second weighing element 220 are rotatable to a specified angle or direction relative to the device housing 201. Therefore, the second weighing element 220 may be configured to weigh a medical liquid container at different heights and/or angles, to meet more usage requirements of the blood purification device 200.

[**0292]** The second weighing element 220 may be configured to hang a medical liquid container to monitor weight of the medical liquid container. Therefore, the second weighing element 220 may also be reused for carrying and fixing the medical liquid container. Of course, the second weighing element 220 may also be configured to support a medical liquid container to monitor weight of the medical liquid container, which is not limited in the embodiment of the application.

[**0293]** In some implementations, the device housing 201 includes a first surface 2013, a third surface 2015 and a sixth surface 2018. The first surface 2013 and the sixth surface 2018 are located at opposite sides of the third surface 2015 along a horizontal direction, the first surface 2013 is arranged opposite to the sixth surface 2018, and the power region 2011 is located on the first surface 2013. The second weighing element 220 is movable to a first state and a second state. In the first state, the second weighing element 220 is rotated to a normal direction facing toward the third surface 2015, and the weighing bar 219 is adjusted to a first height. In the second state, the second weighing element 220 is rotated to a normal direction facing toward the sixth surface 2018, the weighing bar 219 is adjusted to a second height, and the second height is less than the first height.

[**0294]** For example, the second weighing element 220 may be adjusted to the first state when the second weighing element 220 needs to carry a lighter medical liquid container, and the second weighing element 220 may be adjusted to the second state when the second weighing element 220 needs to carry a heavier medical liquid container.

[**0295]** It may also be understood that the blood purification device 200 may be provided with the first weighing elements 216 and the second weighing element 220 simultaneously, or may be provided with only one of the first weighing elements 216 and the second weighing element 220, which is not limited in the embodiment of the application.

[**0296]** For example, when the blood purification device 200 may be provided with the first weighing elements 216 and the second weighing element 220 simultaneously, the first weighing elements 216 may include at least three first weighing elements.

[**0297]** In some implementations, if the second weighing element 220 is moved to the second state, the second weighing element 220 and the first weighing elements 216 are located at different sides of the device housing 201 along the horizontal direction. Therefore, when the second weighing element 220 is moved to the second state where it has a lower height, it may avoid that the medical liquid container hung by the second weighing element 220 interferes with medical liquid containers hung by the first weighing elements 216.

[**0298]** For example, the first weighing elements 216 include multiple first weighing elements, and each of the first weighing elements 216 is arranged at a bottom part of the first surface 2013, the second surface 2014 or the third surface 2015 of the device housing 201. Therefore, if the second weighing element 220 is moved to the second state, the medical liquid container hung by the second weighing element 220 may be located on the sixth surface 2018.

[**0299]** In some implementations, the weighing bar 219 may be protruded from an upper side of the device housing 201 along the vertical direction, and the second weighing element 220 is arranged at an upper end of the weighing bar 219 along the vertical direction. Therefore, space at the lower side of the device housing 201 may be fully utilized by the first weighing elements 216, and space at the upper side of the device housing 201 may be fully utilized by the second weighing element 220.

[**0300]** In some implementations, the device housing 201 is further provided with an effluent discharge port, the effluent discharge port is configured to discharge effluents from the panel kit 100, and the effluent discharge port and the power region 2011 are arranged at different sides of the device housing 201.

[**0301]** Therefore, compared to further arranging the effluent discharge port on the first surface 2013, in the embodiment of the application, other surfaces of the device housing 201 may be fully utilized to arrange the effluent discharge port, to avoid an excessive large area of the first surface 2013, therefore it is beneficial for miniaturization design of the blood purification device 200.

[**0302]** For example, the effluent discharge port may be arranged at a side of the device housing 201 facing away from the power region 2011; alternatively, the effluent discharge port and the power region 2011 may be arranged at adjacent sides of the device housing 201, which is not limited in the embodiment of the application.

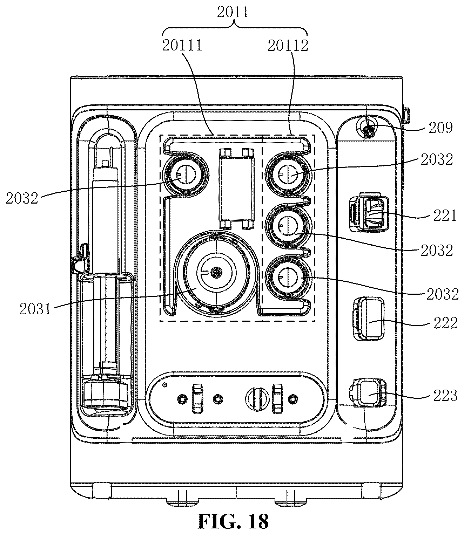

[**0303]** With continuous reference to FIG. 18, in some implementations, the power region 2011 includes a first pump body region 20111 and a second pump body region 20112 that are adjacent to each other, and the first pump body region 20111 is located at a side of the second pump body region 20112 along a horizontal direction. The medical liquid pump 2032 includes multiple medical liquid pumps, one of the medical liquid pumps 2032 and the blood pump 2031 are arranged substantially along a vertical direction and disposed in the first pump body region 20111, and other medical liquid pumps 2032 are arranged substantially along the vertical direction and disposed in the second pump body region 20112. Therefore, with the blood pump 2031 and the multiple medical liquid pumps 2032 arranged in two rows, space in the power region 2011 may be fully utilized.

[**0304]** When it is mentioned that the first pump body region 20111 is located at a side of the second pump body region 20112 along the horizontal direction, it is possible that an upper end of the first pump body region 20111 is flush with an upper end of the second pump body region 20112, and a lower end of the first pump body region 20111 is flush with a lower end of the second pump body region 20112.

[**0305]** When it is mentioned that the first pump body region 20111 is located at a side of the second pump body region 20112 along the horizontal direction, it is also possible that the upper end of the first pump body region 20111 is higher than the upper end of the second pump body region 20112. At this time, the lower end of the first pump body region 20111 may be lower than the lower end of the second pump body region 20112, or the lower end of the first pump body region 20111 may be higher than the lower end of the second pump body region 20112, or the lower end of the first pump body region 20111 may be flush with the lower end of the second pump body region 20112.

[**0306]** When it is mentioned that the first pump body region 20111 is located at a side of the second pump body region 20112 along the horizontal direction, it is also possible that the upper end of the first pump body region 20111 is lower than the upper end of the second pump body region 20112. At this time, the lower end of the first pump body region 20111 may be lower than the lower end of the second pump body region 20112, or the lower end of the first pump body region 20111 may be higher than the lower end of the second pump body region 20112, or the lower end of the first pump body region 20111 may be flush with the lower end of the second pump body region 20112.

[**0307]** That is, in the embodiment of the application, when it is mentioned that the first pump body region 20111 is located at a side of the second pump body region 20112 along the horizontal direction, it is possible that the upper end of the first pump body region 20111 is not flush with the upper end of the second pump body region 20112, and the lower end of the first pump body region 20111 is not flush with the lower end of the second pump body region 20112.

[**0308]** In some embodiments, when it is mentioned that the first pump body region 20111 is located at a side of the second pump body region 20112 along the horizontal direction, it is possible that at least half of a region of the first pump body region 20111 along the vertical direction is arranged directly opposite to the second pump body region 20112 along the horizontal direction, and/or at least half of a region of the second pump body region 20112 along the vertical direction is arranged directly opposite to the first pump body region 20111 along the horizontal direction.

[**0309]** In some implementations, the at least one medical liquid pump 2032 includes a front pump that is before the blood pump, a dialysate pump, a replacement fluid pump and an effluent pump. The front pump is at least configured to be adapted to one of the pump pipeline sections 112 (such as the pump pipeline section of the front pump 1123) so as to drive an anticoagulant drug to flow along said one of the pump pipeline sections 112, the dialysate pump is configured to be adapted to one of the pump pipeline sections 112 (such as the dialysate pump pipeline section 1124) so as to drive dialysate to flow along said one of the pump pipeline sections 112, the replacement fluid pump is configured to be adapted to one of the pump pipeline sections 112 (such as the replacement fluid pump pipeline section 1125) so as to drive a replacement fluid to flow along said one of the pump pipeline sections 112, the effluent pump is configured to be adapted to one of the pump pipeline sections 112 (such as the waste liquid pressure pump pipeline section 1122) so as to drive the medical liquid flowing out of a filter 300 to flow along said one of the pump pipeline sections 112.

[**0310]** In some implementations, one of the front pump and the effluent pump is arranged in the first pump body region 20111, and the other of the front pump and the effluent pump is arranged at a bottom or middle part of the second pump body region 20112.

[**0311]** Therefore, on one hand, no matter the medical liquid pump 2032 located in the first pump body region 20111 or the medical liquid pump 2032 located at the bottom or middle part of the second pump body region 20112 is the waste liquid pressure pump pipeline section 1122, if the waste liquid pressure pump pipeline section 1122 is damaged, it is not easy for waste liquid of the medical liquid leaked from the waste liquid pressure pump pipeline section 1122 to contaminate other medical pump pipeline sections 112 located above the second pump body region 20112.

[**0312]** On the other hand, no matter the medical liquid pump 2032 located in the first pump body region 20111 or the medical liquid pump 2032 located at the bottom or middle part of the second pump body region 20112 is the front pump, since the pump pipeline section of the front pump 1123 is adjacent to the blood pump 2031, the blood drawing flow path 1301 or the blood pumping flow path 1302 connected to the blood pump pipeline section 1121 may be closer to the second liquid inlet flow path 1308 connected to the pump pipeline section of the front pump 1123, such that the second liquid inlet flow path 1308 may be shorter.

[**0313]** It may be understood that when it is mentioned that the other of the front pump and the effluent pump is arranged at the bottom part of the second pump body region 20112, it means that among multiple pumps in the second pump body region 20112, a corresponding front pump or effluent pump is a pump arranged at the bottom part along the vertical direction. For example, three pumps arranged along the vertical direction are disposed in the second pump body region 20112, a lowermost pump in the second pump body region 20112 is a corresponding front pump or effluent pump.

[**0314]** Similarly, when it is mentioned that the other of the front pump and the effluent pump is arranged at the middle part of the second pump body region 20112, it means that among multiple pumps in the second pump body region 20112, a corresponding front pump or effluent pump is a pump arranged in the middle along the vertical direction. For example, three pumps arranged along the vertical direction are disposed in the second pump body region 20112, a pump in the middle of the second pump body region 20112 is a corresponding front pump or effluent pump.

[**0315]** Exemplarily, pumps in the first pump body region 20111 are the front pump and the blood pump 2031 arranged in sequence from top to bottom, and pumps in the second pump body region 20112 are the dialysate pump, the replacement fluid pump and the effluent pump arranged in sequence from top to bottom.

[**0316]** Alternatively, the pumps in the first pump body region 20111 are the front pump and the blood pump 2031 arranged in sequence from top to bottom, and the pumps in the second pump body region 20112 are the dialysate pump, the effluent pump and the replacement fluid pump arranged in sequence from top to bottom.

[**0317]** Alternatively, the pumps in the first pump body region 20111 are the front pump and the blood pump 2031 arranged in sequence from top to bottom, and the pumps in the second pump body region 20112 are the replacement fluid pump, the dialysate pump and the effluent pump arranged in sequence from top to bottom.

[**0318]** Alternatively, the pumps in the first pump body region 20111 are the front pump and the blood pump 2031 arranged in sequence from top to bottom, and the pumps in the second pump body region 20112 are the replacement fluid pump, the effluent pump and the dialysate pump arranged in sequence from top to bottom.

[**0319]** Alternatively, the pumps in the first pump body region 20111 are the effluent pump and the blood pump 2031 arranged in sequence from top to bottom, and the pumps in the second pump body region 20112 are the dialysate pump, the replacement fluid pump and the front pump arranged in sequence from top to bottom.

[**0320]** Alternatively, the pumps in the first pump body region 20111 are the effluent pump and the blood pump 2031 arranged in sequence from top to bottom, and the pumps in the second pump body region 20112 are the dialysate pump, the front pump and the replacement fluid pump arranged in sequence from top to bottom.

[**0321]** Alternatively, the pumps in the first pump body region 20111 are the effluent pump and the blood pump 2031 arranged in sequence from top to bottom, and the pumps in the second pump body region 20112 are the replacement fluid pump, the dialysate pump and the front pump arranged in sequence from top to bottom.

[**0322]** Alternatively, the pumps in the first pump body region 20111 are the effluent pump and the blood pump 2031 arranged in sequence from top to bottom, and the pumps in the second pump body region 20112 are the replacement fluid pump, the front pump and the dialysate pump arranged in sequence from top to bottom.

[**0323]** In some implementations, the blood pump 2031 and the medical liquid pump 2032 in the first pump body region 20111 form a first pump group, and all the medical liquid pumps 2032 in the second pump body region 20112 form a second pump group. A ratio of a length of the first pump group in the vertical direction to a length of the second pump group in the vertical direction is 0.8 to 1.2. For example, the ratio of the length of the first pump group in the vertical direction to the length of the second pump group in the vertical direction is 0.8, 0.85, 0.894, 0.9, 0.934, 0.95, 0.97, 1, 1.06, 1.08, 1.1, 1.15 or 1.2, which is not limited in the embodiment of the application.

[**0324]** Furthermore, it may also be understood that the length of the first pump group in the vertical direction is substantially the same as the length of the second pump group in the vertical direction, such that the space in the power region 2011 is fully utilized by two rows of pump bodies with substantially the same length.

[**0325]** It may also be understood that the length of the first pump group in the vertical direction is a distance from a top end of an uppermost pump body of the first pump group to a bottom end of a lowermost pump body of the first pump group; the length of the second pump group in the vertical direction is a distance from a top end of an uppermost pump body of the second pump group to a bottom end of a lowermost pump body of the second pump group.

[**0326]** In some implementations, the blood pump 2031 and the medical liquid pump 2032 in the first pump body region 20111 form a first pump group, and the medical liquid pumps 2032 in the second pump body region 20112 form a second pump group. At least one end of the first pump group along the vertical direction is substantially flush with at least one end of the second pump group along the vertical direction. Therefore, the space in the power region 2011 may be fully utilized.

[**0327]** Of course, in some other implementations, each end of the first pump group along the vertical direction is not flush with the corresponding end of the second pump group along the vertical direction, which is not limited in the embodiment of the application.

[**0328]** In some implementations, the first assembly and disassembly member 2021 is located at least in the first pump body region 20111. For example, at least a portion of the first assembly and disassembly member 2021 and the medical liquid pump 2032 in the first pump body region 20111 are arranged side by side along the horizontal direction and are both located above the blood pump 2031, therefore space above the blood pump 2031 is fully utilized.

[**0329]** In some implementations, if the user faces toward the first surface 2013 of the device housing 201, the first pump body region 20111 is located to a left side of the second pump body region 20112 within the user's visual field. Alternatively, it may be simply understood as if the user faces toward the first surface 2013 of the device housing 201, the first pump body region 20111 is located at the user's left front side, and the second pump body region 20112 is located at the user's right front side.

[**0330]** Of course, in some other implementations, if the user faces toward the first surface 2013 of the device housing 201, the first pump body region 20111 is located to a right side of the second pump body region 20112 within the user's visual field. Alternatively, it may be simply understood as if the user faces toward the first surface 2013 of the device housing 201, the first pump body region 20111 is located at the user's right front side, and the second pump body region 20112 is located at the user's left front side.

[**0331]** In some implementations, the second pump body region 20112 is located at a side of the first pump body region 20111 close to the second surface 2014.

[**0332]** Therefore, when the second surface 2014 is provided with the heating region 207 and the heating region 207 is used for heating the medical liquid, it may also be understood that the first pump body region 20111 where the blood pump 2031 is arranged is farther away from the heating region 207, such that the medical liquid in the pump pipeline section 112 at a position where the panel kit 100 is mounted in the second pump body region 20112 may flow to the heating region 207 through a shorter flow path to heat the medical liquid, and thus the panel kit 100 may have advantages of smooth pipeline arrangement and shorter pipelines.

[**0333]** Optionally, it is also possible that the first pump body region 20111 is located at a side of the second pump body region 20112 close to the second surface 2014, which is not limited in the embodiment of the application.

[**0334]** According to a second aspect, an embodiment of the application further provides a blood purification system, the blood purification system includes a blood purification device 200 and a panel kit 100.

[**0335]** The blood purification device 200 includes a device housing 201, a pump component 203 and a second interface 204. The panel kit 100 includes a power component 11 and a monitoring component 12.

[**0336]** The power component 11 includes a power panel 111 and at least two pump pipeline sections 112, the power panel 111 is configured to be detachably mounted to the device housing 201, the at least two pump pipeline sections 112 are mounted to the power panel 111, at least one of the pump pipeline sections 112 is configured to cooperate with the pump component 203 to drive blood to flow along the at least one of the pump pipeline sections 112, and at least another one of the pump pipeline sections 112 is configured to cooperate with the pump component 203 to drive a medical liquid to flow along the at least another one of the pump pipeline sections 112. If the power panel 111 is mounted at a first target position of the device housing 201, the pump pipeline sections 112 are adapted to the pump component 203, and if the pump pipeline sections 112 are adapted to the pump component 203, the pump pipeline sections 112 can be driven by the pump component 203 to drive the corresponding blood or medical liquid to flow.

[**0337]** The monitoring component 12 includes a monitoring panel 121 and at least two monitoring pipeline sections 122, the monitoring panel 121 is configured to be detachably mounted to the device housing 201, the monitoring panel 121 is arranged separately from the power panel 111 such that the monitoring panel 121 and the power panel 111 can be assembled and disassembled separately, that is, the power panel 111 and the monitoring panel 121 are independent of each other and can be assembled and disassembled separately, the at least two monitoring pipeline sections 122 are mounted to the monitoring panel 121, the at least two monitoring pipeline sections 122 are configured to monitor parameter information of the medical liquid and/or the blood, and the parameter information at least includes a pressure parameter. At least one of the monitoring pipeline sections 122 is provided with a first interface 1221; if the second interface 204 is connected to the first interface 1221, the blood purification device 200 is capable of monitoring the parameter information through the monitoring pipeline sections 122.

[**0338]** The first interface 1221 is fixedly arranged relative to the monitoring panel 121 in a process of mounting the monitoring panel 121 to the device housing 201. Alternatively, the first interface 1221 is movably arranged relative to the monitoring panel 121, and the movable arrangement allows a user to achieve connection of the first interface 1221 to the second interface 204 by operating the first interface 1221.

[**0339]** Therefore, on one hand, when the first interface 1221 is fixedly arranged relative to the monitoring panel 121, since it may avoid that the first interface 1221 moves relative to the monitoring panel 121 in a process of mounting the monitoring panel 121, it may avoid that the second interface 204 collides with the first interface 1221 and cannot be mounted in place. Of course, when the first interface 1221 is prevented from moving relative to the monitoring panel 121, it may also avoid that the first interface 1221 is shaken during operation of the blood purification device 200 to cause the second interface 204 to loosen and fall off relative to the first interface 1221, therefore accuracy and reliability of the detection result of the blood purification device 200 may be improved.

[**0340]** On the other hand, when the first interface 1221 is movably arranged relative to the monitoring panel 121, after the monitoring panel 121 is mounted in place, it may facilitate the user to manually connect the first interface 1221 to the second interface 204, to avoid failure to align and seal between the first interface 1221 and the second interface 204.

[**0341]** In some implementations, specific structures of the blood purification device 200 and the panel kit 100 may refer to specific structures of the blood purification device 200 and the panel kit 100 according to the first aspect respectively, which are not elaborated here in the embodiment of the application.

[**0342]** According to a third aspect, an embodiment of the application further provides a blood purification device 200. The blood purification device 200 is configured to be adapted to a panel kit 100 so as to perform extracorporeal blood purification.

[**0343]** The panel kit 100 includes a power component 11 and a monitoring component 12, the power component 11 includes a power panel 111 and at least two pump pipeline sections 112 mounted to the power panel 111. The monitoring component 12 includes a monitoring panel 121 and at least two monitoring pipeline sections 122 mounted to the monitoring panel 121. The at least two monitoring pipeline sections 122 are configured to monitor parameter information of a medical liquid and/or a blood, the parameter information at least includes a pressure parameter, and at least one of the monitoring pipeline sections 122 is provided with a first interface 1221.

[**0344]** The blood purification device 200 includes a device housing 201, an assembly and disassembly component 202, a pump component 203 and at least two second interfaces 204.

[**0345]** The device housing 201 is provided with a power region 2011 and a parameter region 2012.

[**0346]** The assembly and disassembly component 202 includes a first assembly and disassembly member 2021 and a second assembly and disassembly member 2022, the power panel 111 can be detachably connected to the first assembly and disassembly member 2021 so as to achieve that the power panel 111 is mounted at a first target position in the power region 2011, the monitoring panel 121 can be detachably connected to the second assembly and disassembly member 2022 so as to achieve that the monitoring panel 121 is mounted at a second target position in the parameter region 2012, the power panel 111 and the monitoring panel 121 may be assembled and disassembled separately, that is, the power panel 111 and the monitoring panel 121 are independent of each other and can be assembled and disassembled separately.

[**0347]** The pump component 203 is arranged in the power region 2011, the pump component 203 includes a blood pump 2031 and at least one medical liquid pump 2032, and if the power panel 111 is mounted at the first target position, the blood pump 2031 is capable of being adapted to at least one of the pump pipeline sections 112 so as to drive the blood to flow along the at least one of the pump pipeline sections 112, and the at least one medical liquid pump 2032 is capable of being adapted to other pump pipeline sections 112 so as to drive the medical liquid to flow along the other pump pipeline sections 112.

[**0348]** The second interfaces 204 are arranged in the parameter region 2012. The monitoring component 12 is capable of being manually mounted such that the monitoring panel 121 is mounted at the second target position and the second interfaces 204 are synchronously connected to the first interfaces 1221 respectively, and if the second interfaces 204 are connected to the first interfaces 1221 respectively, the blood purification device 200 is capable of monitoring the parameter information through the monitoring pipeline sections 122.

[**0349]** Therefore, operations of mounting the first interfaces 1221 independently may be reduced, and it may avoid that the user mounts the first interfaces 1221 at wrong positions when the user mounts the first interfaces 1221 manually, thereby improving accuracy of mounting the first interfaces 1221.

[**0350]** It should be noted that the monitoring component 12 is capable of being manually mounted such that the monitoring panel 121 is mounted at the second target position of the device housing 201 and the first interfaces 1221 are synchronously connected to the second interfaces 204 respectively, and it may also be understood that the user may allow the monitoring panel 121 to synchronously drive the first interfaces 1221 to connect to the second interfaces 204 respectively by an action of mounting the monitoring panel 121 at the second target position. In this process, two actions of mounting the monitoring panel 121 at the second target position and connecting the first interfaces 1221 to the second interfaces 204 respectively may be completed simultaneously, or there may be a certain time difference between the two actions, for example, when the first interfaces 1221 are movably arranged relative to the monitoring panel 121, it is possible that the monitoring panel 121 still continues to move by a certain distance after the first interfaces 1221 are connected to the second interfaces 204 respectively, and then may be mounted at the second target position of the device housing 201, which is not limited in the embodiment of the application.

[**0351]** It should also be noted that the monitoring component 12 may be manually mounted once such that the monitoring panel 121 is mounted at the second target position and the first interfaces 1221 are synchronously connected to the second interfaces 204 respectively, or the monitoring component 12 may be manually mounted at least twice such that the monitoring panel 121 is mounted at the second target position and the first interfaces 1221 are synchronously connected to the second interfaces 204 respectively, which is not limited in the embodiment of the application.

[**0352]** In some implementations, specific structures of the blood purification device 200 and the panel kit 100 may refer to specific structures of the blood purification device 200 and the panel kit 100 according to the first aspect respectively, which are not elaborated here in the embodiment of the application.

[**0353]** According to a fourth aspect, an embodiment of the application further provides a blood purification device 200. The blood purification device 200 is configured to be adapted to a panel kit 100 so as to perform extracorporeal blood purification.

[**0354]** The panel kit 100 includes a power component 11 and a monitoring component 12, the power component 11 includes a power panel 111 and at least two pump pipeline sections 112 mounted to the power panel 111. The monitoring component 12 includes a monitoring panel 121 and at least two monitoring pipeline sections 122 mounted to the monitoring panel 121. The at least two monitoring pipeline sections 122 are configured to monitor parameter information of a medical liquid and/or a blood, the parameter information at least includes a pressure parameter, and at least one of the monitoring pipeline sections 122 is provided with a first interface 1221.

[**0355]** The blood purification device 200 includes a device housing 201, an assembly and disassembly component 202, a pump component 203 and at least two second interfaces 204.

[**0356]** The device housing 201 is provided with a power region 2011 and a parameter region 2012.

[**0357]** The assembly and disassembly component 202 includes a first assembly and disassembly member 2021 and a second assembly and disassembly member 2022, the power panel 111 can be detachably connected to the first assembly and disassembly member 2021 so as to achieve that the power panel 111 is mounted at a first target position in the power region 2011, the monitoring panel 121 can be detachably connected to the second assembly and disassembly member 2022 so as to achieve that the monitoring panel 121 is mounted at a second target position in the parameter region 2012, the power panel 111 and the monitoring panel 121 can be assembled and disassembled separately, that is, the power panel 111 and the monitoring panel 121 are independent of each other and can be assembled and disassembled separately.

[**0358]** The pump component 203 is arranged in the power region 2011, the pump component 203 includes a blood pump 2031 and at least one medical liquid pump 2032, and if the power panel 111 is mounted at the first target position, the blood pump 2031 is capable of being adapted to at least one of the pump pipeline sections 112 so as to drive the blood to flow along the at least one of the pump pipeline sections 112, and the at least one medical liquid pump 2032 is capable of being adapted to other pump pipeline sections 112 so as to drive the medical liquid to flow along the other pump pipeline sections 112.

[**0359]** The second interfaces 204 are arranged in the parameter region 2012. If the monitoring panel 121 is mounted at the second target position, the second interfaces 204 can be extended to be connected to the first interfaces 1221 respectively, and if the second interfaces 204 are connected to the first interfaces 1221 respectively, the blood purification device 200 is capable of monitoring the parameter information through the monitoring pipeline sections 122.

[**0360]** Therefore, the user only needs to mount the monitoring panel 121 at the second target position of the device housing 201 simply, the second interfaces 204 on the blood purification device 200 may be extended to be connected to the first interfaces 1221 of the monitoring pipeline sections 122 respectively, and it is unnecessary for the user to mount the monitoring panel 121 at the second target position of the device housing 201 and then manually connect the second interfaces 204 to the first interfaces 1221 respectively, such that installation of the panel kit 100 is simpler.

[**0361]** In some implementations, specific structures of the blood purification device 200 and the panel kit 100 may refer to specific structures of the blood purification device 200 and the panel kit 100 according to the first aspect respectively, which are not elaborated here in the embodiment of the application.

[**0362]** According to a fifth aspect, an embodiment of the application further provides a blood purification device 200. The blood purification device 200 is configured to be adapted to a panel kit 100 so as to perform extracorporeal blood purification.

[**0363]** The panel kit 100 includes a power component 11 and a monitoring component 12, the power component 11 includes a power panel 111 and at least two pump pipeline sections 112 mounted to the power panel 111. The monitoring component 12 includes a monitoring panel 121 and at least two monitoring pipeline sections 122 mounted to the monitoring panel 121. The at least two monitoring pipeline sections 122 are configured to monitor parameter information of a medical liquid and/or a blood, the parameter information at least includes a pressure parameter, and at least one of the monitoring pipeline sections 122 is provided with a first interface 1221.

[**0364]** The blood purification device 200 includes a device housing 201, an assembly and disassembly component 202, a pump component 203 and at least two second interfaces 204.

[**0365]** The device housing 201 is provided with a power region 2011 and a parameter region 2012.

[**0366]** The assembly and disassembly component 202 includes a first assembly and disassembly member 2021 and a second assembly and disassembly member 2022, the power panel 111 can be detachably connected to the first assembly and disassembly member 2021 so as to achieve that the power panel 111 is mounted at a first target position in the power region 2011, the monitoring panel 121 can be detachably connected to the second assembly and disassembly member 2022 so as to achieve that the monitoring panel 121 is mounted at a second target position in the parameter region 2012, the power panel 111 and the monitoring panel 121 can be assembled and disassembled separately, that is, the power panel 111 and the monitoring panel 121 are independent of each other and can be assembled and disassembled separately.

[**0367]** The pump component 203 is arranged in the power region 2011, the pump component 203 includes a blood pump 2031 and at least one medical liquid pump 2032, and if the power panel 111 is mounted at the first target position, the blood pump 2031 is capable of being adapted to at least one of the pump pipeline sections 112 so as to drive the blood to flow along the at least one of the pump pipeline sections 112, and the at least one medical liquid pump 2032 is capable of being adapted to other pump pipeline sections 112 so as to drive the medical liquid to flow along the other pump pipeline sections 112.

[**0368]** The second interfaces 204 are arranged in the parameter region 2012. If the monitoring panel 121 is mounted at a second preset position of the device housing 201, the second assembly and disassembly member 2022 is capable of driving the monitoring panel 121 to move to the second target position, such that the second interfaces 204 are connected to the first interfaces 1221 respectively, and if the second interfaces 204 are connected to the first interfaces 1221 respectively, the blood purification device 200 is capable of monitoring the parameter information through the monitoring pipeline sections 122.

[**0369]** Therefore, for installation of the panel kit 100, the user only needs to mount the monitoring panel 121 at the second preset position of the device housing 201, and then the device housing 201 automatically drives the monitoring panel 121 to the second target position so as to achieve automatic installation of the monitoring panel 121, such that installation of the panel kit 100 is simpler.

[**0370]** In some implementations, specific structures of the blood purification device 200 and the panel kit 100 may refer to specific structures of the blood purification device 200 and the panel kit 100 according to the first aspect respectively, which are not elaborated here in the embodiment of the application.

[**0371]** According to a sixth aspect, an embodiment of the application further provides a blood purification device 200. The blood purification device 200 is configured to be adapted to a panel kit 100 so as to perform extracorporeal blood purification.

[**0372]** The panel kit 100 includes a power component 11 and a monitoring component 12, the power component 11 includes a power panel 111 and at least two pump pipeline sections 112 mounted to the power panel 111. The monitoring component 12 includes a monitoring panel 121 and at least two monitoring pipeline sections 122 mounted to the monitoring panel 121. The at least two monitoring pipeline sections 122 are configured to monitor parameter information of a medical liquid and/or a blood, the parameter information at least includes a pressure parameter, and at least one of the monitoring pipeline sections 122 is provided with a first interface 1221.

[**0373]** The blood purification device 200 includes a device housing 201, an assembly and disassembly component 202, a pump component 203 and at least two second interfaces 204.

[**0374]** The device housing 201 is provided with a power region 2011 and a parameter region 2012.

[**0375]** The assembly and disassembly component 202 includes a first assembly and disassembly member 2021 and a second assembly and disassembly member 2022, the power panel 111 can be detachably connected to the first assembly and disassembly member 2021 so as to achieve that the power panel 111 is mounted at a first target position in the power region 2011, the monitoring panel 121 can be detachably connected to the second assembly and disassembly member 2022 so as to achieve that the monitoring panel 121 is mounted at a second target position in the parameter region 2012, the power panel 111 and the monitoring panel 121 are independent of each other and can be assembled and disassembled separately.

[**0376]** The pump component 203 is arranged in the power region 2011, the pump component 203 includes a blood pump 2031 and at least one medical liquid pump 2032, and if the power panel 111 is mounted at the first target position, the blood pump 2031 is capable of being adapted to at least one of the pump pipeline sections 112 so as to drive the blood to flow along the at least one of the pump pipeline sections 112, and the at least one medical liquid pump 2032 is capable of being adapted to other pump pipeline sections 112 so as to drive the medical liquid to flow along the other pump pipeline sections 112.

[**0377]** The second interfaces 204 are arranged in the parameter region 2012. If the monitoring panel 121 is mounted at the second target position, the first interfaces 1221 are capable of being manually operated to achieve connection of the first interfaces 1221 to the second interfaces 204, and if the second interfaces 204 are connected to the first interfaces 1221 respectively, the blood purification device 200 is capable of monitoring the parameter information through the monitoring pipeline sections 122.

[**0378]** In the embodiment of the application, installation of the at least two pump pipeline sections 112 may be assisted by the power panel 111, and installation of the at least two monitoring pipeline sections 122 may be assisted by the monitoring panel 121, to reduce difficulty of mounting the panel kit 100. On this basis, when the second interface 204 is movably arranged relative to the monitoring panel 121, after the monitoring panel 121 is mounted in place, it may facilitate the user to manually connect the first interface 1221 to the second interface 204, to avoid failure to align and seal between the first interface 1221 and the second interface 204.

[**0379]** In some implementations, specific structures of the blood purification device 200 and the panel kit 100 may refer to specific structures of the blood purification device 200 and the panel kit 100 according to the first aspect respectively, which are not elaborated here in the embodiment of the application.

[**0380]** According to a seventh aspect, an embodiment of the application further provides a blood purification device 200. The blood purification device 200 is configured to be adapted to a panel kit 100 so as to perform extracorporeal blood purification.

[**0381]** The panel kit 100 includes a power component 11, a monitoring component 12, a heating component 15 and a degassing pot 1313, the power component 11 includes a power panel 111 and at least two pump pipeline sections 112 mounted to the power panel 111. The monitoring component 12 includes a monitoring panel 121 and at least two monitoring pipeline sections 122 mounted to the monitoring panel 121. The at least two monitoring pipeline sections 122 are configured to monitor parameter information of a medical liquid and/or a blood. The parameter information at least includes a pressure parameter, and at least one of the monitoring pipeline sections 122 is provided with a first interface 1221.

[**0382]** The blood purification device 200 includes a device housing 201, an assembly and disassembly component 202, a pump component 203 and at least two second interfaces 204.

[**0383]** The device housing 201 includes a first surface 2013, a second surface 2014, a third surface 2015, a fourth surface 2016 and a fifth surface 2017; the second surface 2014, the fourth surface 2016, the first surface 2013, the fifth surface 2017 and the third surface 2015 are sequentially and directly connected along a horizontal direction, the third surface 2015 is arranged opposite to the second surface 2014, the first surface 2013 is provided with a power region 2011 and a parameter region 2012, the second surface 2014 is provided with a heating region 207, the heating component 15 is configured to be detachably mounted on the heating region 207, the medical liquid and/or the blood in the heating component 15 are capable of being heated by the blood purification device 200 if the heating component 15 is mounted on the heating region 207, each of the third surface 2015 and the first surface 2013 is bent and connected to the fifth surface 2017, and each of the second surface 2014 and the first surface 2013 is bent and connected to the fourth surface 2016. A syringe pump mounting region 212 is arranged on one of the fourth surface 2016 and the fifth surface 2017, a safety monitoring region 214 is arranged on the other of the fourth surface 2016 and the fifth surface 2017, a syringe pump 213 is configured to be mounted on the syringe pump mounting region 212, the syringe pump 213 is configured to inject the medical liquid into pipelines of the panel kit 100, at least two of a degassing pot fixing seat 221, a degassing pot monitoring element 20, a bubble monitoring element 222 and a liquid stopping clamp 223 are arranged on the safety monitoring region 214, the degassing pot 1313 is configured to be detachably mounted and fixed to the degassing pot fixing seat 221, the bubble monitoring element 222 is configured to monitor whether there are bubbles in a downstream pipeline connected to the degassing pot 1313, and the liquid stopping clamp 223 is configured to control on/off of the downstream pipeline connected to the degassing pot 1313.

[**0384]** Alternatively, the device housing 201 includes a first surface 2013, a second surface 2014 and a third surface 2015; the third surface 2015, the first surface 2013 and the second surface 2014 are sequentially and directly connected along a horizontal direction, the third surface 2015 is arranged opposite to the second surface 2014, the first surface 2013 is provided with a power region 2011 and a parameter region 2012, the second surface 2014 is provided with a heating region 207, the heating component 15 is configured to be detachably mounted on the heating region 207, the medical liquid and/or the blood in the heating component 15 are capable of being heated by the blood purification device 200 if the heating component 15 is mounted on the heating region 207. The first surface 2013 is further provided with a syringe pump mounting region 212 and a safety monitoring region 214, a syringe pump 213 is configured to be mounted on the syringe pump mounting region 212, the syringe pump 213 is configured to inject the medical liquid into pipelines of the panel kit 100, at least two of a degassing pot fixing seat 221, a degassing pot monitoring element 209, a bubble monitoring element 222 and a liquid stopping clamp 223 are arranged on the safety monitoring region 214, the degassing pot 1313 is configured to be detachably mounted and fixed to the degassing pot fixing seat 221, the bubble monitoring element 222 is configured to monitor whether there are bubbles in a downstream pipeline connected to the degassing pot 1313, and the liquid stopping clamp 223 is configured to control on/off of the downstream pipeline connected to the degassing pot 1313.

[**0385]** The assembly and disassembly component 202 includes a first assembly and disassembly member 2021 and a second assembly and disassembly member 2022, the power panel 111 can be detachably connected to the first assembly and disassembly member 2021 so as to achieve that the power panel 111 is mounted at a first target position in the power region 2011, the monitoring panel 121 can be detachably connected to the second assembly and disassembly member 2022 so as to achieve that the monitoring panel 121 is mounted at a second target position in the parameter region 2012, the power panel 111 and the monitoring panel 121 can be assembled and disassembled separately, that is, the power panel 111 and the monitoring panel 121 are independent of each other and can be assembled and disassembled separately.

[**0386]** The pump component 203 is arranged in the power region 2011, the pump component 203 includes a blood pump 2031 and at least one medical liquid pump 2032, and if the power panel 111 is mounted at the first target position, the blood pump 2031 is capable of being adapted to at least one of the pump pipeline sections 112 so as to drive the blood to flow along the at least one of the pump pipeline sections 112, and the at least one medical liquid pump 2032 is capable of being adapted to other pump pipeline sections 112 so as to drive the medical liquid to flow along the other pump pipeline sections 112.

[**0387]** The second interfaces 204 are arranged in the parameter region 2012. If the monitoring panel 121 is mounted at the second target position, the second interfaces 204 are connected to the first interfaces 1221 respectively, and if the second interfaces 204 are connected to the first interfaces 1221 respectively, the blood purification device 200 is capable of monitoring the parameter information through the monitoring pipeline sections 122.

[**0388]** Therefore, compared to arranging all of the power region 2011, the parameter region 2012, the heating region 207, the syringe pump mounting region 212 and the safety monitoring region 214 on the first surface 2013, in the embodiment of the application, the parameter region 2012 and the power region 2011 are arranged on the first surface 2013, the heating region 207 is arranged on the second surface 2014, and the syringe pump mounting region 212 and the safety monitoring region 214 are arranged on the fourth surface 2016 and the fifth surface 2017 respectively, space on outer surfaces of the device housing 201 may be fully utilized to avoid that it needs to reserve a large space on the first surface 2013 of the device housing 201, therefore it is beneficial for miniaturization design of the device housing 201 or the blood purification device 200.

[**0389]** In some implementations, specific structures of the blood purification device 200 and the panel kit 100 may refer to specific structures of the blood purification device 200 and the panel kit 100 according to the first aspect respectively, which are not elaborated here in the embodiment of the application.

[**0390]** The following is a list of enumerated example embodiments.
1. A blood purification system, comprising a panel kit and a blood purification device, wherein the panel kit comprises a power component and a monitoring component, the power component comprises a power panel and at least two pump pipeline sections mounted to the power panel; the monitoring component comprises a monitoring panel, and at least two monitoring pipeline sections mounted to the monitoring panel and configured to monitor parameter information of a medical liquid and/or a blood, the parameter information at least comprises a pressure parameter, and at least one of the monitoring pipeline sections is provided with a first interface; wherein the blood purification device comprises:
   a device housing, provided with a power region and a parameter region;
   an assembly and disassembly component, comprising a first assembly and disassembly member and a second assembly and disassembly member, wherein the power panel can be detachably connected to the first assembly and disassembly member so as to achieve that the power panel is mounted at a first target position in the power region, the monitoring panel can be detachably connected to the second assembly and disassembly member so as to achieve that the monitoring panel is mounted at a second target position in the parameter region, the power panel and the monitoring panel are independent of each other and can be assembled and disassembled separately;
   a pump component, arranged in the power region and comprising a blood pump and at least one medical liquid pump, wherein if the power panel is mounted at the first target position, the blood pump is capable of being adapted to at least one of the pump pipeline sections so as to drive the blood to flow along the at least one of the pump pipeline sections, and the at least one medical liquid pump is capable of being adapted to other pump pipeline sections so as to drive the medical liquid to flow along the other pump pipeline sections; and
   at least two second interfaces, arranged in the parameter region, wherein the monitoring component is capable of being manually mounted such that the monitoring panel is mounted at the second target position and the second interfaces are synchronously connected to the first interfaces respectively, and if the second interfaces are connected to the first interfaces respectively, the blood purification device is capable of monitoring the parameter information through the monitoring pipeline sections.
2. A blood purification system, comprises a panel kit and a blood purification device, wherein the panel kit comprises a power component and a monitoring component, the power component comprises a power panel and at least two pump pipeline sections mounted to the power panel; the monitoring component comprises a monitoring panel, and at least two monitoring pipeline sections mounted to the monitoring panel and configured to monitor parameter information of a medical liquid and/or a blood, the parameter information at least comprises a pressure parameter, and at least one of the monitoring pipeline sections is provided with a first interface; wherein the blood purification device comprises:
   a device housing, provided with a power region and a parameter region;
   an assembly and disassembly component, comprising a first assembly and disassembly member and a second assembly and disassembly member, wherein the power panel can be detachably connected to the first assembly and disassembly member so as to achieve that the power panel is mounted at a first target position in the power region, the monitoring panel can be detachably connected to the second assembly and disassembly member so as to achieve that the monitoring panel is mounted at a second target position in the parameter region, the power panel and the monitoring panel are independent of each other and can be assembled and disassembled separately;
   a pump component, arranged in the power region and comprising a blood pump and at least one medical liquid pump, wherein if the power panel is mounted at the first target position, the blood pump is capable of being adapted to at least one of the pump pipeline sections so as to drive the blood to flow along the at least one of the pump pipeline sections, and the at least one medical liquid pump is capable of being adapted to other pump pipeline sections so as to drive the medical liquid to flow along the other pump pipeline sections; and
   at least two second interfaces, arranged in the parameter region, wherein if the monitoring panel is mounted at the second target position, the second interfaces can be extended to be connected to the first interfaces respectively, and if the second interfaces are connected to the first interfaces respectively, the blood purification device is capable of monitoring the parameter information through the monitoring pipeline sections.
3. A blood purification system, comprises a panel kit and a blood purification device, wherein the panel kit comprises a power component and a monitoring component, the power component comprises a power panel and at least two pump pipeline sections mounted to the power panel; the monitoring component comprises a monitoring panel, and at least two monitoring pipeline sections mounted to the monitoring panel and configured to monitor parameter information of a medical liquid and/or a blood, the parameter information at least comprises a pressure parameter, and at least one of the monitoring pipeline sections is provided with a first interface; wherein the blood purification device comprises:
   a device housing, provided with a power region and a parameter region;
   an assembly and disassembly component, comprising a first assembly and disassembly member and a second assembly and disassembly member, wherein the power panel can be detachably connected to the first assembly and disassembly member so as to achieve that the power panel is mounted at a first target position in the power region, the monitoring panel can be detachably connected to the second assembly and disassembly member so as to achieve that the monitoring panel is mounted at a second target position in the parameter region, the power panel and the monitoring panel are independent of each other and can be assembled and disassembled separately;
   a pump component, arranged in the power region and comprising a blood pump and at least one medical liquid pump, wherein if the power panel is mounted at the first target position, the blood pump is capable of being adapted to at least one of the pump pipeline sections so as to drive the blood to flow along the at least one of the pump pipeline sections, and the at least one medical liquid pump is capable of being adapted to other pump pipeline sections so as to drive the medical liquid to flow along the other pump pipeline sections; and
   at least two second interfaces, arranged in the parameter region, wherein if the monitoring panel is mounted at a second preset position of the device housing, the second assembly and disassembly member is capable of driving the monitoring panel to move to the second target position, such that the second interfaces are connected to the first interfaces respectively, and if the second interfaces are connected to the first interfaces respectively, the blood purification device is capable of monitoring the parameter information through the monitoring pipeline sections.
4. A blood purification system, comprises a blood purification device and a panel kit, wherein the blood purification device comprises a device housing, a pump component and a second interface; the panel kit comprises:
   a power component, comprising a power panel and at least two pump pipeline sections, wherein the power panel is configured to be detachably mounted to the device housing, the at least two pump pipeline sections are mounted to the power panel, at least one of the pump pipeline sections is configured to cooperate with the pump component to drive blood to flow along the at least one of the pump pipeline sections, and at least another one of the pump pipeline sections is configured to cooperate with the pump component to drive a medical liquid to flow along the at least another one of the pump pipeline sections; if the power panel is mounted at a first target position of the device housing, the pump pipeline sections are adapted to the pump component, and if the pump pipeline sections are adapted to the pump component, the pump pipeline sections can be driven by the pump component to drive the corresponding blood or medical liquid to flow; and
   a monitoring component, comprising a monitoring panel and at least two monitoring pipeline sections, wherein the monitoring panel is configured to be detachably mounted to the device housing, the monitoring panel is arranged separately from the power panel such that the monitoring panel and the power panel are independent of each other and can be assembled and disassembled separately, the at least two monitoring pipeline sections are mounted to the monitoring panel and are configured to monitor parameter information of the medical liquid and/or the blood, and the parameter information at least comprises a pressure parameter; at least one of the monitoring pipeline sections is provided with a first interface; if the second interface is connected to the first interface, the blood purification device is capable of monitoring the parameter information through the monitoring pipeline sections; the first interface is fixedly arranged relative to the monitoring panel in a process of mounting the monitoring panel to the device housing.
5. A blood purification system, comprises a blood purification device and a panel kit, wherein the blood purification device comprises a device housing, a pump component and a second interface; the panel kit comprises:
   a power component, comprising a power panel and at least two pump pipeline sections, wherein the power panel is configured to be detachably mounted to the device housing, the at least two pump pipeline sections are mounted to the power panel, at least one of the pump pipeline sections is configured to cooperate with the pump component to drive blood to flow along the at least one of the pump pipeline sections, and at least another one of the pump pipeline sections is configured to cooperate with the pump component to drive a medical liquid to flow along the at least another one of the pump pipeline sections; if the power panel is mounted at a first target position of the device housing, the pump pipeline sections are adapted to the pump component, and if the pump pipeline sections are adapted to the pump component, the pump pipeline sections can be driven by the pump component to drive the corresponding blood or medical liquid to flow; and
   a monitoring component, comprising a monitoring panel and at least two monitoring pipeline sections, wherein the monitoring panel is configured to be detachably mounted to the device housing, the monitoring panel is arranged separately from the power panel such that the monitoring panel and the power panel are independent of each other and can be assembled and disassembled separately, the at least two monitoring pipeline sections are mounted to the monitoring panel and are configured to monitor parameter information of the medical liquid and/or the blood, and the parameter information at least comprises a pressure parameter; at least one of the monitoring pipeline sections is provided with a first interface; if the second interface is connected to the first interface, the blood purification device is capable of monitoring the parameter information through the monitoring pipeline sections; the first interface is movably arranged relative to the monitoring panel, and the movable arrangement allows a user to achieve connection of the first interface to the second interface by operating the first interface.
6. A blood purification system, comprises a panel kit and a blood purification device, wherein the panel kit comprises a power component and a monitoring component, the power component comprises a power panel and at least two pump pipeline sections mounted to the power panel; the monitoring component comprises a monitoring panel, and at least two monitoring pipeline sections mounted to the monitoring panel and configured to monitor parameter information of a medical liquid and/or a blood, the parameter information at least comprises a pressure parameter, and at least one of the monitoring pipeline sections is provided with a first interface; wherein the blood purification device comprises:
   a device housing, provided with a power region and a parameter region;
   an assembly and disassembly component, comprising a first assembly and disassembly member and a second assembly and disassembly member, wherein the power panel can be detachably connected to the first assembly and disassembly member so as to achieve that the power panel is mounted at a first target position in the power region, the monitoring panel can be detachably connected to the second assembly and disassembly member so as to achieve that the monitoring panel is mounted at a second target position in the parameter region, the power panel and the monitoring panel are independent of each other and can be assembled and disassembled separately;
   a pump component, arranged in the power region and comprising a blood pump and at least one medical liquid pump, wherein if the power panel is mounted at the first target position, the blood pump is capable of being adapted to at least one of the pump pipeline sections so as to drive the blood to flow along the at least one of the pump pipeline sections, and the at least one medical liquid pump is capable of being adapted to other pump pipeline sections so as to drive the medical liquid to flow along the other pump pipeline sections; and
   at least two second interfaces, arranged in the parameter region, wherein if the monitoring panel is mounted at the second target position, the first interfaces are capable of being manually operated to achieve connection of the first interfaces to the second interfaces, and if the second interfaces are connected to the first interfaces respectively, the blood purification device is capable of monitoring the parameter information through the monitoring pipeline sections.
7. The blood purification system of any one of examples 1 to 3 and 6, wherein the first assembly and disassembly member comprises a first claw clamped to a first connection part of the power panel to achieve detachable connection;
   the second assembly and disassembly member comprises a second claw clamped to a second connection part of the monitoring panel to achieve detachable connection.
8. The blood purification system of example 7, wherein the assembly and disassembly component further comprises a first driving mechanism that is transmissively connected to the first claw, and if the power panel is mounted at a first preset position by the first claw, the first driving mechanism is configured to drive the first claw to move so as to drive the power panel to be mounted at the first target position; or
   the first claw is fixedly arranged on the device housing, and the power component is capable of being manually mounted such that the power panel is mounted at the first target position and each of the pump pipeline sections is synchronously adapted to the corresponding blood pump or medical liquid pump.
9. The blood purification system of example 8, wherein the blood purification device further comprises a first position detection mechanism;
   when the first claw is transmissively connected to the first driving mechanism, the first position detection mechanism is configured to detect whether the power panel is mounted at the first preset position and/or the first target position;
   when the first claw is fixedly arranged on the device housing, the first position detection mechanism is configured to detect whether the power panel is mounted at the first target position.
10. The blood purification system of any one of examples 1 to 3 and 6, wherein the second assembly and disassembly member comprises a second claw clamped to a second connection part of the monitoring panel to achieve detachable connection;
   wherein the second claw is fixedly arranged on the device housing; or, the assembly and disassembly component further comprises a second driving mechanism transmissively connected to the second claw, and if the monitoring panel is mounted at a second preset position by the second claw, the second driving mechanism is configured to drive the second claw to move so as to drive the monitoring panel to be mounted at the second target position.
11. The blood purification system of example 10, wherein the blood purification device further comprises a second position detection mechanism;
   when the second claw is fixedly arranged on the device housing, the second position detection mechanism is configured to detect whether the monitoring panel is mounted at the second target position;
   when the second claw is transmissively connected to the second driving mechanism, the second position detection mechanism is configured to detect whether the monitoring panel is mounted at the second preset position and/or the second target position.
12. The blood purification system of any one of examples 1 to 6, wherein the second interface is fixedly arranged relative to the device housing; or
   the second interface is movably arranged relative to the device housing.
13. The blood purification system of any one of examples 1 to 6, wherein at least two of the monitoring pipeline sections are provided with the first interfaces respectively, and when the blood purification device comprises at least two second interfaces, the at least two second interfaces comprise at least two pressure interfaces, and if the pressure interfaces are connected to the first interfaces respectively, the blood purification device is capable of monitoring the pressure parameter through the monitoring pipeline sections; or
   the at least two second interfaces comprise at least one pressure interface, and if the pressure interface is connected to the first interface, the blood purification device is capable of monitoring the pressure parameter through the monitoring pipeline sections; the blood purification device further comprises a blood leakage monitoring module which is capable of monitoring, through the monitoring pipeline sections, whether the blood at one side of a filtration membrane of a filter permeates to the other side of the filtration membrane.
14. The blood purification system of any one of examples 1 to 3 and 6, wherein the device housing is provided with a first surface, each of the parameter region and the power region is arranged on the first surface, and the parameter region is located at a lower side of the power region along a vertical direction.
15. The blood purification system of example 14, wherein the device housing is further provided with a second surface that faces toward a direction different from the first surface, is located at a side of the first surface along a horizontal direction, and is provided with a heating region where a heating component of the panel kit is configured to be detachably mounted;
   if the heating component is mounted on the heating region, the medical liquid and/or the blood in the heating component are capable of being heated by the blood purification device.
16. The blood purification system of example 15, wherein the heating component is provided with a plurality of liquid flow paths configured to deliver the medical liquid;
   the blood purification device further comprises a pipeline switching unit arranged on the device housing, and if the heating component is mounted on the heating region, the pipeline switching unit is capable of controlling on/off of at least two of the liquid flow paths.
17. The blood purification system of example 16, wherein the device housing is further provided with a third surface located at a side opposite to the second surface, the third surface and the second surface are located at two sides of the first surface;
   the blood purification device further comprises at least one of a blood composition analysis component and an infusion pump, the blood composition analysis component is configured to detect blood compositions in a blood sample, the blood composition analysis component is provided with a blood composition analysis detection interface, the blood composition analysis detection interface is arranged on the third surface, and the infusion pump is arranged on the third surface.
18. The blood purification system of example 15, wherein the blood purification device further comprises a syringe pump mounting region and a safety monitoring region, a syringe pump is configured to be mounted on the syringe pump mounting region and is configured to inject the medical liquid into the panel kit, at least two of a degassing pot fixing seat, a degassing pot monitoring element, a bubble monitoring element and a liquid stopping clamp are arranged on the safety monitoring region, a degassing pot of the panel kit is configured to be detachably mounted to the degassing pot fixing seat, the bubble monitoring element is configured to monitor whether there are bubbles in a downstream pipeline connected to the degassing pot, and the liquid stopping clamp is configured to control on/off of the downstream pipeline connected to the degassing pot,wherein the device housing is further provided with a third surface, a fourth surface and a fifth surface; the second surface, the fourth surface, the first surface, the fifth surface and the third surface are sequentially and directly connected along the horizontal direction, the third surface is arranged opposite to the second surface, each of the second surface and the first surface is bent and connected to the fourth surface, and each of the third surface and the first surface is bent and connected to the fifth surface; one of the syringe pump mounting region and the safety monitoring region is arranged on the fourth surface, and the other of the syringe pump mounting region and the safety monitoring region is arranged on the fifth surface; or
   each of the syringe pump mounting region and the safety monitoring region is arranged on the first surface, and the syringe pump mounting region and the safety monitoring region are arranged at a same side or different sides of the power region.
19. The blood purification system of example 15, wherein the blood purification device further comprises a mounting arm mounted on the second surface, and a filter can be detachably connected to the mounting arm, and if the heating component is mounted on the heating region, the medical liquid in the heating component is capable of being heated by the blood purification device; or
   the device housing is further provided with a third surface located at a side opposite to the second surface, the third surface and the second surface are located at two sides of the first surface; the blood purification device further comprises a mounting arm mounted on the third surface, and a filter can be detachably connected to the mounting arm, and if the heating component is mounted on the heating region, the blood in the heating component is capable of being heated by the blood purification device.
20. The blood purification system of any one of examples 1 to 3 and 6, wherein the power region comprises a first pump body region and a second pump body region that are adjacent to each other, and the first pump body region is located at a side of the second pump body region along a horizontal direction;
   the medical liquid pump comprises a plurality of medical liquid pumps, one of the medical liquid pumps and the blood pump are arranged substantially along a vertical direction and disposed in the first pump body region, and other medical liquid pumps are arranged substantially along the vertical direction and disposed in the second pump body region.
21. The blood purification system of example 20, wherein the at least one medical liquid pump comprises a front pump that is before the blood pump, a dialysate pump, a replacement fluid pump and an effluent pump, the front pump is at least configured to be adapted to one of the pump pipeline sections so as to drive an anticoagulant drug to flow along said one of the pump pipeline sections, the dialysate pump is configured to be adapted to one of the pump pipeline sections so as to drive dialysate to flow along said one of the pump pipeline sections, the replacement fluid pump is configured to be adapted to one of the pump pipeline sections so as to drive a replacement fluid to flow along said one of the pump pipeline sections, the effluent pump is configured to be adapted to one of the pump pipeline sections so as to drive the medical liquid flowing out of a filter to flow along said one of the pump pipeline sections;
   wherein one of the front pump and the effluent pump is arranged in the first pump body region, and the other of the front pump and the effluent pump is arranged at a bottom or middle part of the second pump body region.
22. The blood purification system of example 21, wherein pumps in the first pump body region are the front pump and the blood pump arranged in sequence from top to bottom, and pumps in the second pump body region are the dialysate pump, the replacement fluid pump and the effluent pump arranged in sequence from top to bottom; or
   the pumps in the first pump body region are the front pump and the blood pump arranged in sequence from top to bottom, and the pumps in the second pump body region are the dialysate pump, the effluent pump and the replacement fluid pump arranged in sequence from top to bottom; or
   the pumps in the first pump body region are the front pump and the blood pump arranged in sequence from top to bottom, and the pumps in the second pump body region are the replacement fluid pump, the dialysate pump and the effluent pump arranged in sequence from top to bottom; or
   the pumps in the first pump body region are the front pump and the blood pump arranged in sequence from top to bottom, and the pumps in the second pump body region are the replacement fluid pump, the effluent pump and the dialysate pump arranged in sequence from top to bottom; or
   the pumps in the first pump body region are the effluent pump and the blood pump arranged in sequence from top to bottom, and the pumps in the second pump body region are the dialysate pump, the replacement fluid pump and the front pump arranged in sequence from top to bottom; or
   the pumps in the first pump body region are the effluent pump and the blood pump arranged in sequence from top to bottom, and the pumps in the second pump body region are the dialysate pump, the front pump and the replacement fluid pump arranged in sequence from top to bottom; or
   the pumps in the first pump body region are the effluent pump and the blood pump arranged in sequence from top to bottom, and the pumps in the second pump body region are the replacement fluid pump, the dialysate pump and the front pump arranged in sequence from top to bottom; or
   the pumps in the first pump body region are the effluent pump and the blood pump arranged in sequence from top to bottom, and the pumps in the second pump body region are the replacement fluid pump, the front pump and the dialysate pump arranged in sequence from top to bottom.
23. The blood purification system of example 20, wherein the blood pump and the medical liquid pump in the first pump body region form a first pump group, and all the medical liquid pumps in the second pump body region form a second pump group;
   wherein a ratio of a length of the first pump group in the vertical direction to a length of the second pump group in the vertical direction is 0.8 to 1.2; and/or
   at least one end of the first pump group along the vertical direction is substantially flush with at least one end of the second pump group along the vertical direction, or each end of the first pump group along the vertical direction is not flush with the corresponding end of the second pump group along the vertical direction.
24. The blood purification system of any one of examples 1 to 6, wherein the blood purification device further comprises a plurality of first weighing elements arranged at a bottom part of the device housing along a vertical direction.
25. The blood purification system of example 24, wherein at least one of the first weighing elements is movably connected to the device housing; and/or
   the first weighing elements comprise at least three first weighing elements; and/or
   the blood purification device further comprises a base and a support frame mounted to the base and fixedly connected to the device housing, such that the device housing is arranged above the base along the vertical direction; the base comprises an avoidance part and a distal end located at a side of the avoidance part, which side is away from the support frame along a horizontal direction, a height of the avoidance part is less than that of the distal end, and the avoidance part is located below at least one of the first weighing elements along the vertical direction, so as to avoid a medical liquid container hung by the at least one of the first weighing elements above the avoidance part.
26. The blood purification system of any one of examples 1 to 6, wherein the blood purification device further comprises a weighing bar and a second weighing element, the weighing bar is liftably mounted on the device housing and is adjustable to a specified height, the second weighing element is arranged at an end of the weighing bar, which end is away from the device housing, and the weighing bar and/or the second weighing element are rotatable to a specified angle or direction relative to the device housing.
27. The blood purification system of example 26, wherein the blood purification device further comprises a plurality of first weighing elements arranged at a bottom part of the device housing along a vertical direction; and/or
   the device housing comprises a first surface, a third surface and a sixth surface; the first surface and the sixth surface are located at opposite sides of the third surface along a horizontal direction, the first surface is arranged opposite to the sixth surface, and the power region is located on the first surface; wherein the second weighing element is movable to a first state and a second state; in the first state, the second weighing element is rotated to a normal direction facing toward the third surface, and the weighing bar is adjusted to a first height; in the second state, the second weighing element is rotated to a normal direction facing toward the sixth surface, the weighing bar is adjusted to a second height, and the second height is less than the first height.
28. The blood purification system of any one of examples 1 to 3 and 6, wherein the device housing is further provided with an effluent discharge port configured to discharge effluents from the panel kit, and the effluent discharge port and the power region are arranged at different sides of the device housing.
29. A blood purification device, characterized in that the blood purification device is configured to be adapted to a panel kit so as to perform extracorporeal blood purification, wherein the panel kit comprises a power component and a monitoring component, the power component comprises a power panel and at least two pump pipeline sections mounted to the power panel; the monitoring component comprises a monitoring panel, and at least two monitoring pipeline sections mounted to the monitoring panel and configured to monitor parameter information of a medical liquid and/or a blood, the parameter information at least comprises a pressure parameter, and at least one of the monitoring pipeline sections is provided with a first interface; wherein the blood purification device comprises:
   a device housing, provided with a power region and a parameter region;
   an assembly and disassembly component, comprising a first assembly and disassembly member and a second assembly and disassembly member, wherein the power panel can be detachably connected to the first assembly and disassembly member so as to achieve that the power panel is mounted at a first target position in the power region, the monitoring panel can be detachably connected to the second assembly and disassembly member so as to achieve that the monitoring panel is mounted at a second target position in the parameter region, the power panel and the monitoring panel are independent of each other and can be assembled and disassembled separately;
   a pump component, arranged in the power region and comprising a blood pump and at least one medical liquid pump, wherein if the power panel is mounted at the first target position, the blood pump is capable of being adapted to at least one of the pump pipeline sections so as to drive the blood to flow along the at least one of the pump pipeline sections, and the at least one medical liquid pump is capable of being adapted to other pump pipeline sections so as to drive the medical liquid to flow along the other pump pipeline sections; and
   at least two second interfaces, arranged in the parameter region, wherein the monitoring component is capable of being manually mounted such that the monitoring panel is mounted at the second target position and the second interfaces are synchronously connected to the first interfaces respectively, and if the second interfaces are connected to the first interfaces respectively, the blood purification device is capable of monitoring the parameter information through the monitoring pipeline sections.
30. A blood purification device, characterized in that the blood purification device is configured to be adapted to a panel kit so as to perform extracorporeal blood purification, wherein the panel kit comprises a power component and a monitoring component, the power component comprises a power panel and at least two pump pipeline sections mounted to the power panel; the monitoring component comprises a monitoring panel, and at least two monitoring pipeline sections mounted to the monitoring panel and configured to monitor parameter information of a medical liquid and/or a blood, the parameter information at least comprises a pressure parameter, and at least one of the monitoring pipeline sections is provided with a first interface; wherein the blood purification device comprises:
   a device housing, provided with a power region and a parameter region;
   an assembly and disassembly component, comprising a first assembly and disassembly member and a second assembly and disassembly member, wherein the power panel can be detachably connected to the first assembly and disassembly member so as to achieve that the power panel is mounted at a first target position in the power region, the monitoring panel can be detachably connected to the second assembly and disassembly member so as to achieve that the monitoring panel is mounted at a second target position in the parameter region, the power panel and the monitoring panel are independent of each other and can be assembled and disassembled separately;
   a pump component, arranged in the power region and comprising a blood pump and at least one medical liquid pump, wherein if the power panel is mounted at the first target position, the blood pump is capable of being adapted to at least one of the pump pipeline sections so as to drive the blood to flow along the at least one of the pump pipeline sections, and the at least one medical liquid pump is capable of being adapted to other pump pipeline sections so as to drive the medical liquid to flow along the other pump pipeline sections; and
   at least two second interfaces, arranged in the parameter region telescopically, wherein if the monitoring panel is mounted at the second target position, the second interfaces can be extended to be connected to the first interfaces respectively, and if the second interfaces are connected to the first interfaces respectively, the blood purification device is capable of monitoring the parameter information through the monitoring pipeline sections.
31. A blood purification device, characterized in that the blood purification device is configured to be adapted to a panel kit so as to perform extracorporeal blood purification, wherein the panel kit comprises a power component and a monitoring component, the power component comprises a power panel and at least two pump pipeline sections mounted to the power panel; the monitoring component comprises a monitoring panel, and at least two monitoring pipeline sections mounted to the monitoring panel and configured to monitor parameter information of a medical liquid and/or a blood, the parameter information at least comprises a pressure parameter, and at least one of the monitoring pipeline sections is provided with a first interface; wherein the blood purification device comprises:
   a device housing, provided with a power region and a parameter region;
   an assembly and disassembly component, comprising a first assembly and disassembly member and a second assembly and disassembly member, wherein the power panel can be detachably connected to the first assembly and disassembly member so as to achieve that the power panel is mounted at a first target position in the power region, the monitoring panel can be detachably connected to the second assembly and disassembly member so as to achieve that the monitoring panel is mounted at a second target position in the parameter region, the power panel and the monitoring panel are independent of each other and can be assembled and disassembled separately;
   a pump component, arranged in the power region and comprising a blood pump and at least one medical liquid pump, wherein if the power panel is mounted at the first target position, the blood pump is capable of being adapted to at least one of the pump pipeline sections so as to drive the blood to flow along the at least one of the pump pipeline sections, and the at least one medical liquid pump is capable of being adapted to other pump pipeline sections so as to drive the medical liquid to flow along the other pump pipeline sections; and
   at least two second interfaces, arranged in the parameter region, wherein if the monitoring panel is mounted at a second preset position of the device housing, the second assembly and disassembly member is capable of driving the monitoring panel to move to the second target position, such that the second interfaces are connected to the first interfaces respectively, and if the second interfaces are connected to the first interfaces respectively, the blood purification device is capable of monitoring the parameter information through the monitoring pipeline sections.
32. A blood purification device, characterized in that the blood purification device is configured to be adapted to a panel kit so as to perform extracorporeal blood purification, wherein the panel kit comprises a power component and a monitoring component, the power component comprises a power panel and at least two pump pipeline sections mounted to the power panel; the monitoring component comprises a monitoring panel, and at least two monitoring pipeline sections mounted to the monitoring panel and configured to monitor parameter information of a medical liquid and/or a blood, the parameter information at least comprises a pressure parameter, and at least one of the monitoring pipeline sections is provided with a first interface; wherein the blood purification device comprises:
   a device housing, provided with a power region and a parameter region;
   an assembly and disassembly component, comprising a first assembly and disassembly member and a second assembly and disassembly member, wherein the power panel can be detachably connected to the first assembly and disassembly member so as to achieve that the power panel is mounted at a first target position in the power region, the monitoring panel can be detachably connected to the second assembly and disassembly member so as to achieve that the monitoring panel is mounted at a second target position in the parameter region, the power panel and the monitoring panel are independent of each other and can be assembled and disassembled separately;
   a pump component, arranged in the power region and comprising a blood pump and at least one medical liquid pump, wherein if the power panel is mounted at the first target position, the blood pump is capable of being adapted to at least one of the pump pipeline sections so as to drive the blood to flow along the at least one of the pump pipeline sections, and the at least one medical liquid pump is capable of being adapted to other pump pipeline sections so as to drive the medical liquid to flow along the other pump pipeline sections; and
   at least two second interfaces, arranged in the parameter region, wherein if the monitoring panel is mounted at the second target position, the first interfaces are capable of being manually operated to achieve connection of the first interfaces to the second interfaces, and if the second interfaces are connected to the first interfaces respectively, the blood purification device is capable of monitoring the parameter information through the monitoring pipeline sections.
33. The blood purification device of any one of examples 29 to 32, wherein the first assembly and disassembly member comprises a first claw clamped to a first connection part of the power panel to achieve detachable connection;
   the second assembly and disassembly member comprises a second claw clamped to a second connection part of the monitoring panel to achieve detachable connection.
34. The blood purification device of example 33, wherein the assembly and disassembly component further comprises a first driving mechanism transmissively connected to the first claw, and if the power panel is mounted at a first preset position by the first claw, the first driving mechanism is configured to drive the first claw to move so as to drive the power panel to be mounted at the first target position; or
   the first claw is fixedly arranged on the device housing, and the power component is capable of being manually mounted such that the power panel is mounted at the first target position and each of the pump pipeline sections is synchronously adapted to the corresponding blood pump or medical liquid pump.
35. The blood purification device of example 34, further comprising a first position detection mechanism;
   wherein when the first claw is transmissively connected to the first driving mechanism, the first position detection mechanism is configured to detect whether the power panel is mounted at the first preset position and/or the first target position;
   when the first claw is fixedly arranged on the device housing, the first position detection mechanism is configured to detect whether the power panel is mounted at the first target position.
36. The blood purification device of example 30, wherein the second assembly and disassembly member comprises a second claw clamped to a second connection part of the monitoring panel to achieve detachable connection;
   wherein the second claw is fixedly arranged on the device housing; or, the assembly and disassembly component further comprises a second driving mechanism transmissively connected to the second claw, and if the monitoring panel is mounted at a second preset position by the second claw, the second driving mechanism is configured to drive the second claw to move so as to drive the monitoring panel to be mounted at the second target position.
37. The blood purification device of example 36, further comprising a second position detection mechanism;
   wherein when the second claw is fixedly arranged on the device housing, the second position detection mechanism is configured to detect whether the monitoring panel is mounted at the second target position;
   when the second claw is transmissively connected to the second driving mechanism, the second position detection mechanism is configured to detect whether the monitoring panel is mounted at the second preset position and/or the second target position.
38. The blood purification device of any one of examples 29 to 32, wherein the second interface is fixedly arranged relative to the device housing; or
   the second interface is movably arranged relative to the device housing.
39. The blood purification device of any one of examples 29 to 32, wherein at least two of the monitoring pipeline sections are provided with the first interfaces respectively, and the at least two second interfaces comprise at least two pressure interfaces, and if the pressure interfaces are connected to the first interfaces respectively, the blood purification device is capable of monitoring the pressure parameter through the monitoring pipeline sections; or
   the at least two second interfaces comprise at least one pressure interface, and if the pressure interface is connected to the first interface, the blood purification device is capable of monitoring the pressure parameter through the monitoring pipeline sections; the blood purification device further comprises a blood leakage monitoring module which is capable of monitoring, through the monitoring pipeline sections, whether the blood at one side of a filtration membrane of a filter permeates to the other side of the filtration membrane.
40. The blood purification device of any one of examples 29 to 32, wherein the device housing is provided with a first surface, each of the parameter region and the power region is arranged on the first surface, and the parameter region is located at a lower side of the power region along a vertical direction.
41. The blood purification device of example 40, wherein the device housing is further provided with a second surface that faces toward a direction different from the first surface, is located at a side of the first surface along a horizontal direction, and is provided with a heating region where a heating component of the panel kit is configured to be detachably mounted;
   if the heating component is mounted on the heating region, the medical liquid and/or the blood in the heating component are capable of being heated by the blood purification device.
42. The blood purification device of example 41, wherein the heating component is provided with a plurality of liquid flow paths configured to deliver the medical liquid;
   the blood purification device further comprises a pipeline switching unit arranged on the device housing, and if the heating component is mounted on the heating region, the pipeline switching unit is capable of controlling on/off of at least two of the liquid flow paths.
43. The blood purification device of example 41, wherein the device housing is further provided with a third surface located at a side opposite to the second surface, the third surface and the second surface are located at two sides of the first surface;
   the blood purification device further comprises at least one of a blood composition analysis component and an infusion pump, the blood composition analysis component is configured to detect blood compositions in a blood sample, the blood composition analysis component is provided with a blood composition analysis detection interface arranged on the third surface, and the infusion pump is arranged on the third surface.
44. The blood purification device of example 41, further comprising a syringe pump mounting region and a safety monitoring region, wherein a syringe pump is configured to be mounted on the syringe pump mounting region and is configured to inject the medical liquid into the panel kit, at least two of a degassing pot fixing seat, a degassing pot monitoring element, a bubble monitoring element and a liquid stopping clamp are arranged on the safety monitoring region, a degassing pot of the panel kit is configured to be detachably mounted and fixed to the degassing pot fixing seat, the bubble monitoring element is configured to monitor whether there are bubbles in a downstream pipeline connected to the degassing pot, and the liquid stopping clamp is configured to control on/off of the downstream pipeline connected to the degassing pot;
   wherein the device housing is further provided with a third surface, a fourth surface and a fifth surface; the third surface, the fourth surface, the first surface, the fifth surface and the second surface are sequentially and directly connected along the horizontal direction, the third surface is arranged opposite to the second surface, each of the second surface and the first surface is bent and connected to the fourth surface, and each of the third surface and the first surface is bent and connected to the fifth surface; one of the syringe pump mounting region and the safety monitoring region is arranged on the fourth surface, and the other of the syringe pump mounting region and the safety monitoring region is arranged on the fifth surface; or
   each of the syringe pump mounting region and the safety monitoring region is arranged on the first surface, and the syringe pump mounting region and the safety monitoring region are arranged at a same side or different sides of the power region.
45. The blood purification device of example 41, further comprising a mounting arm mounted on the second surface, wherein a filter can be detachably connected to the mounting arm, and if the heating component is mounted on the heating region, the medical liquid in the heating component is capable of being heated by the blood purification device; or
   the device housing is further provided with a third surface located at a side opposite to the second surface, the third surface and the second surface are located at two sides of the first surface; the blood purification device further comprises a mounting arm mounted on the third surface, a filter can be detachably connected to the mounting arm, and if the heating component is mounted on the heating region, the blood in the heating component is capable of being heated by the blood purification device.
46. The blood purification device of any one of examples 29 to 32, wherein the power region comprises a first pump body region and a second pump body region that are adjacent to each other, and the first pump body region is located at a side of the second pump body region along a horizontal direction;
   the medical liquid pump comprises a plurality of medical liquid pumps, one of the medical liquid pumps and the blood pump are arranged substantially along a vertical direction and disposed in the first pump body region, and other medical liquid pumps are arranged substantially along the vertical direction and disposed in the second pump body region.
47. The blood purification device of example 46, wherein the at least one medical liquid pump comprises a front pump that is before the blood pump, a dialysate pump, a replacement fluid pump and an effluent pump, the front pump is configured to be adapted to one of the pump pipeline sections so as to drive an anticoagulant drug to flow along said one of the pump pipeline sections, the dialysate pump is configured to be adapted to one of the pump pipeline sections so as to drive dialysate to flow along said one of the pump pipeline sections, the replacement fluid pump is configured to be adapted to one of the pump pipeline sections so as to drive a replacement fluid to flow along said one of the pump pipeline sections, the effluent pump is configured to be adapted to one of the pump pipeline sections so as to drive the medical liquid flowing out of a filter to flow along said one of the pump pipeline sections;
   wherein one of the front pump and the effluent pump is arranged in the first pump body region, and the other of the front pump and the effluent pump is arranged at a bottom or middle part of the second pump body region.
48. The blood purification device of example 47, wherein pumps in the first pump body region are the front pump and the blood pump arranged in sequence from top to bottom, and pumps in the second pump body region are the dialysate pump, the replacement fluid pump and the effluent pump arranged in sequence from top to bottom; or
   the pumps in the first pump body region are the front pump and the blood pump arranged in sequence from top to bottom, and the pumps in the second pump body region are the dialysate pump, the effluent pump and the replacement fluid pump arranged in sequence from top to bottom; or
   the pumps in the first pump body region are the front pump and the blood pump arranged in sequence from top to bottom, and the pumps in the second pump body region are the replacement fluid pump, the dialysate pump and the effluent pump arranged in sequence from top to bottom; or
   the pumps in the first pump body region are the front pump and the blood pump arranged in sequence from top to bottom, and the pumps in the second pump body region are the replacement fluid pump, the effluent pump and the dialysate pump arranged in sequence from top to bottom; or
   the pumps in the first pump body region are the effluent pump and the blood pump arranged in sequence from top to bottom, and the pumps in the second pump body region are the dialysate pump, the replacement fluid pump and the front pump arranged in sequence from top to bottom; or
   the pumps in the first pump body region are the effluent pump and the blood pump arranged in sequence from top to bottom, and the pumps in the second pump body region are the dialysate pump, the front pump and the replacement fluid pump arranged in sequence from top to bottom; or
   the pumps in the first pump body region are the effluent pump and the blood pump arranged in sequence from top to bottom, and the pumps in the second pump body region are the replacement fluid pump, the dialysate pump and the front pump arranged in sequence from top to bottom; or
   the pumps in the first pump body region are the effluent pump and the blood pump arranged in sequence from top to bottom, and the pumps in the second pump body region are the replacement fluid pump, the front pump and the dialysate pump arranged in sequence from top to bottom.
49. The blood purification device of example 46, wherein the blood pump and the medical liquid pump in the first pump body region form a first pump group, and all the medical liquid pumps in the second pump body region form a second pump group;
   wherein a ratio of a length of the first pump group in the vertical direction to a length of the second pump group in the vertical direction is 0.8 to 1.2; and/or
   at least one end of the first pump group along the vertical direction is substantially flush with at least one end of the second pump group along the vertical direction, or each end of the first pump group along the vertical direction is not flush with the corresponding end of the second pump group along the vertical direction.
50. The blood purification device of any one of examples 29 to 32, further comprising a plurality of first weighing elements arranged at a bottom part of the device housing along a vertical direction.
51. The blood purification device of example 50, wherein at least one of the first weighing elements is movably connected to the device housing; and/or
   the first weighing elements comprise at least three first weighing elements; and/or
   the blood purification device further comprises a base and a support frame mounted to the base and fixedly connected to the device housing, such that the device housing is arranged above the base along the vertical direction; the base comprises an avoidance part and a distal end located at a side of the avoidance part, which side is away from the support frame along a horizontal direction, a height of the avoidance part is less than that of the distal end, and the avoidance part is located below at least one of the first weighing elements along the vertical direction, so as to avoid a medical liquid container hung by the at least one of the first weighing elements above the avoidance part.
52. The blood purification device of any one of examples 29 to 31, further comprising a weighing bar and a second weighing element, wherein the weighing bar is liftably mounted on the device housing and is adjustable to a specified height, the second weighing element is arranged at an end of the weighing bar, which end is away from the device housing, and the weighing bar and/or the second weighing element are rotatable to a specified angle or direction relative to the device housing.
53. The blood purification device of example 52, further comprising a plurality of first weighing elements arranged at a bottom part of the device housing along a vertical direction; and/or
   wherein the device housing comprises a first surface, a third surface and a sixth surface; the first surface and the sixth surface are located at opposite sides of the third surface along a horizontal direction, the first surface is arranged opposite to the sixth surface, and the power region is located on the first surface; wherein the second weighing element is movable to a first state and a second state; in the first state, the second weighing element is rotated to a normal direction facing toward the third surface, and the weighing bar is adjusted to a first height; in the second state, the second weighing element is rotated to a normal direction facing toward the sixth surface, the weighing bar is adjusted to a second height, and the second height is less than the first height.
54. The blood purification device of any one of examples 29 to 32, wherein the device housing is further provided with an effluent discharge port configured to discharge effluents from the panel kit, and the effluent discharge port and the power region are arranged at different sides of the device housing.
55. A blood purification device, characterized in that the blood purification device is configured to be adapted to a panel kit so as to perform extracorporeal blood purification, wherein the panel kit comprises a power component, a monitoring component, a heating component and a degassing pot, the power component comprises a power panel and at least two pump pipeline sections mounted to the power panel; the monitoring component comprises a monitoring panel, and at least two monitoring pipeline sections mounted to the monitoring panel and configured to monitor parameter information of a medical liquid and/or a blood, the parameter information at least comprises a pressure parameter, and at least one of the monitoring pipeline sections is provided with a first interface; wherein the blood purification device comprises:
   a device housing, comprising a first surface, a second surface, a third surface, a fourth surface and a fifth surface, wherein the second surface, the fourth surface, the first surface, the fifth surface and the third surface are sequentially and directly connected along a horizontal direction, the third surface is arranged opposite to the second surface, the first surface is provided with a power region and a parameter region, the second surface is provided with a heating region where the heating component is configured to be detachably mounted, the medical liquid and/or the blood in the heating component are capable of being heated by the blood purification device if the heating component is mounted on the heating region, each of the second surface and the first surface is bent and connected to the fourth surface, and each of the third surface and the first surface is bent and connected to the fifth surface; a syringe pump mounting region is arranged on one of the fourth surface and the fifth surface, a safety monitoring region is arranged on the other of the fourth surface and the fifth surface, a syringe pump is configured to be mounted on the syringe pump mounting region and is configured to inject the medical liquid into pipelines of the panel kit, at least two of a degassing pot fixing seat, a degassing pot monitoring element, a bubble monitoring element and a liquid stopping clamp are arranged on the safety monitoring region, the degassing pot is configured to be detachably mounted and fixed to the degassing pot fixing seat, the bubble monitoring element is configured to monitor whether there are bubbles in a downstream pipeline connected to the degassing pot, and the liquid stopping clamp is configured to control on/off of the downstream pipeline connected to the degassing pot; or, the device housing comprises a first surface, a second surface and a third surface, the third surface, the first surface and the second surface are sequentially and directly connected along a horizontal direction, the third surface is arranged opposite to the second surface, the first surface is provided with a power region and a parameter region, the second surface is provided with a heating region where the heating component is configured to be detachably mounted, the medical liquid and/or the blood in the heating component are capable of being heated by the blood purification device if the heating component is mounted on the heating region, the first surface is further provided with a syringe pump mounting region and a safety monitoring region, a syringe pump is configured to be mounted on the syringe pump mounting region and is configured to inject the medical liquid into pipelines of the panel kit, at least two of a degassing pot fixing seat, a degassing pot monitoring element, a bubble monitoring element and a liquid stopping clamp are arranged on the safety monitoring region, the degassing pot is configured to be detachably mounted and fixed to the degassing pot fixing seat, the bubble monitoring element is configured to monitor whether there are bubbles in a downstream pipeline connected to the degassing pot, and the liquid stopping clamp is configured to control on/off of the downstream pipeline connected to the degassing pot;
   an assembly and disassembly component, comprising a first assembly and disassembly member and a second assembly and disassembly member, wherein the power panel can be detachably connected to the first assembly and disassembly member so as to achieve that the power panel is mounted at a first target position in the power region, the monitoring panel can be detachably connected to the second assembly and disassembly member so as to achieve that the monitoring panel is mounted at a second target position in the parameter region, the power panel and the monitoring panel are independent of each other and can be assembled and disassembled separately;
   a pump component, arranged in the power region and comprising a blood pump and at least one medical liquid pump, wherein if the power panel is mounted at the first target position, the blood pump is capable of being adapted to at least one of the pump pipeline sections so as to drive the blood to flow along the at least one of the pump pipeline sections, and the at least one medical liquid pump is capable of being adapted to other pump pipeline sections so as to drive the medical liquid to flow along the other pump pipeline sections; and
   at least two second interfaces, arranged in the parameter region, wherein if the monitoring panel is mounted at the second target position, the second interfaces are connected to the first interfaces respectively, and if the second interfaces are connected to the first interfaces respectively, the blood purification device is capable of monitoring the parameter information through the monitoring pipeline sections.
56. The blood purification device of example 55, wherein the monitoring component is capable of being manually mounted such that the monitoring panel is mounted at the second target position and the second interfaces are synchronously connected to the first interfaces respectively; or
   if the monitoring panel is mounted at the second target position, the second interfaces can be extended to be connected to the first interfaces respectively; or
   if the monitoring panel is mounted at a second preset position of the device housing, the second assembly and disassembly member is capable of driving the monitoring panel to move to the second target position, such that the second interfaces are connected to the first interfaces respectively; or
   if the monitoring panel is mounted at the second target position, the first interfaces are capable of being manually operated to achieve connection of the first interfaces to the second interfaces.
57. The blood purification device of example 55, wherein when the device housing comprises the first surface, the second surface, the third surface, the fourth surface and the fifth surface, the first surface, the second surface, the third surface, the fourth surface and the fifth surface face toward different sides in a circumferential direction of the device housing respectively.

[**0447]** In the above embodiments, description of each of the embodiments has its own emphasis, and portions that are not described in detail in a certain embodiment may refer to relevant descriptions of other embodiments.

[**0448]** The blood purification system and the blood purification device 200 provided in the embodiments of the application have been described in detail as above, principles and implementations of the application have been described here by using specific examples, and descriptions of the above embodiments are only intended to help to understand methods and core ideas of the application; furthermore, variations of specific implementations and scope of the application may be made by those skilled in the art according to the ideas of the application, and in summary, contents of the description should not be understood as limiting the application.

## Claims

1. A blood purification system, **characterized in that** the blood purification system comprises a panel kit (100) and a blood purification device (200), wherein the panel kit (100) comprises a power component (11) and a monitoring component (12), the power component (11) comprises a power panel (111) and at least two pump pipeline sections (112) mounted to the power panel (111); the monitoring component (12) comprises a monitoring panel (121), and at least two monitoring pipeline sections (122) mounted to the monitoring panel (121) and configured to monitor parameter information of a medical liquid and/or a blood, the parameter information at least comprises a pressure parameter, and at least one of the monitoring pipeline sections (122) is provided with a first interface (1221); wherein the blood purification device (200) comprises:
a device housing (201), provided with a power region (2011) and a parameter region (2012);
an assembly and disassembly component (202), comprising a first assembly and disassembly member (2021) and a second assembly and disassembly member (2022), wherein the power panel (111) can be detachably connected to the first assembly and disassembly member (2021) so as to achieve that the power panel (111) is mounted at a first target position in the power region (2011), the monitoring panel (121) can be detachably connected to the second assembly and disassembly member (2022) so as to achieve that the monitoring panel (121) is mounted at a second target position in the parameter region (2012), the power panel (111) and the monitoring panel (121) are independent of each other and can be assembled and disassembled separately;
a pump component (203), arranged in the power region (2011) and comprising a blood pump (2031) and at least one medical liquid pump (2032), wherein if the power panel (111) is mounted at the first target position, the blood pump (2031) is capable of being adapted to at least one of the pump pipeline sections (112) so as to drive the blood to flow along the at least one of the pump pipeline sections (112), and the at least one medical liquid pump (2032) is capable of being adapted to other pump pipeline sections (112) so as to drive the medical liquid to flow along the other pump pipeline sections (112); and
at least two second interfaces (204), arranged in the parameter region (2012), wherein the monitoring component (12) is capable of being manually mounted such that the monitoring panel (121) is mounted at the second target position and the second interfaces (204) are synchronously connected to the first interfaces (1221) respectively, and if the second interfaces (204) are connected to the first interfaces (1221) respectively, the blood purification device (200) is capable of monitoring the parameter information through the monitoring pipeline sections (122).

2. A blood purification system, **characterized in that** the blood purification system comprises a panel kit (100) and a blood purification device (200), wherein the panel kit (100) comprises a power component (11) and a monitoring component (12), the power component (11) comprises a power panel (111) and at least two pump pipeline sections (112) mounted to the power panel (111); the monitoring component (12) comprises a monitoring panel (121), and at least two monitoring pipeline sections (122) mounted to the monitoring panel (121) and configured to monitor parameter information of a medical liquid and/or a blood, the parameter information at least comprises a pressure parameter, and at least one of the monitoring pipeline sections (122) is provided with a first interface (1221); wherein the blood purification device (200) comprises:
a device housing (201), provided with a power region (2011) and a parameter region (2012);
an assembly and disassembly component (202), comprising a first assembly and disassembly member (2021) and a second assembly and disassembly member (2022), wherein the power panel (111) can be detachably connected to the first assembly and disassembly member (2021) so as to achieve that the power panel (111) is mounted at a first target position in the power region (2011), the monitoring panel (121) can be detachably connected to the second assembly and disassembly member (2022) so as to achieve that the monitoring panel (121) is mounted at a second target position in the parameter region (2012), the power panel (111) and the monitoring panel (121) are independent of each other and can be assembled and disassembled separately;
a pump component (203), arranged in the power region (2011) and comprising a blood pump (2031) and at least one medical liquid pump (2032), wherein if the power panel (111) is mounted at the first target position, the blood pump (2031) is capable of being adapted to at least one of the pump pipeline sections (112) so as to drive the blood to flow along the at least one of the pump pipeline sections (112), and the at least one medical liquid pump (2032) is capable of being adapted to other pump pipeline sections (112) so as to drive the medical liquid to flow along the other pump pipeline sections (112); and
at least two second interfaces (204), arranged in the parameter region (2012), wherein if the monitoring panel (121) is mounted at the second target position, the second interfaces (204) can be extended to be connected to the first interfaces (1221) respectively, and if the second interfaces (204) are connected to the first interfaces (1221) respectively, the blood purification device (200) is capable of monitoring the parameter information through the monitoring pipeline sections (122).

3. A blood purification system, **characterized in that** the blood purification system comprises a panel kit (100) and a blood purification device (200), wherein the panel kit (100) comprises a power component (11) and a monitoring component (12), the power component (11) comprises a power panel (111) and at least two pump pipeline sections (112) mounted to the power panel (111); the monitoring component (12) comprises a monitoring panel (121), and at least two monitoring pipeline sections (122) mounted to the monitoring panel (121) and configured to monitor parameter information of a medical liquid and/or a blood, the parameter information at least comprises a pressure parameter, and at least one of the monitoring pipeline sections (122) is provided with a first interface (1221); wherein the blood purification device (200) comprises:
a device housing (201), provided with a power region (2011) and a parameter region (2012);
an assembly and disassembly component (202), comprising a first assembly and disassembly member (2021) and a second assembly and disassembly member (2022), wherein the power panel (111) can be detachably connected to the first assembly and disassembly member (2021) so as to achieve that the power panel (111) is mounted at a first target position in the power region (2011), the monitoring panel (121) can be detachably connected to the second assembly and disassembly member (2022) so as to achieve that the monitoring panel (121) is mounted at a second target position in the parameter region (2012), the power panel (111) and the monitoring panel (121) are independent of each other and can be assembled and disassembled separately;
a pump component (203), arranged in the power region (2011) and comprising a blood pump (2031) and at least one medical liquid pump (2032), wherein if the power panel (111) is mounted at the first target position, the blood pump (2031) is capable of being adapted to at least one of the pump pipeline sections (112) so as to drive the blood to flow along the at least one of the pump pipeline sections (112), and the at least one medical liquid pump (2032) is capable of being adapted to other pump pipeline sections (112) so as to drive the medical liquid to flow along the other pump pipeline sections (112); and
at least two second interfaces (204), arranged in the parameter region (2012), wherein if the monitoring panel (121) is mounted at a second preset position of the device housing (201), the second assembly and disassembly member (2022) is capable of driving the monitoring panel (121) to move to the second target position, such that the second interfaces (204) are connected to the first interfaces (1221) respectively, and if the second interfaces (204) are connected to the first interfaces (1221) respectively, the blood purification device (200) is capable of monitoring the parameter information through the monitoring pipeline sections (122).

4. A blood purification system, **characterized in that** the blood purification system comprises a blood purification device (200) and a panel kit (100), wherein the blood purification device (200) comprises a device housing (201), a pump component (203) and a second interface (204); the panel kit (100) comprises:
a power component (11), comprising a power panel (111) and at least two pump pipeline sections (112), wherein the power panel (111) is configured to be detachably mounted to the device housing (201), the at least two pump pipeline sections (112) are mounted to the power panel (111), at least one of the pump pipeline sections (112) is configured to cooperate with the pump component (203) to drive blood to flow along the at least one of the pump pipeline sections (112), and at least another one of the pump pipeline sections (112) is configured to cooperate with the pump component (203) to drive a medical liquid to flow along the at least another one of the pump pipeline sections (112); if the power panel (111) is mounted at a first target position of the device housing (201), the pump pipeline sections (112) are adapted to the pump component (203), and if the pump pipeline sections (112) are adapted to the pump component (203), the pump pipeline sections (112) can be driven by the pump component (203) to drive the corresponding blood or medical liquid to flow; and
a monitoring component (12), comprising a monitoring panel (121) and at least two monitoring pipeline sections (122), wherein the monitoring panel (121) is configured to be detachably mounted to the device housing (201), the monitoring panel (121) is arranged separately from the power panel (111) such that the monitoring panel (121) and the power panel (111) are independent of each other and can be assembled and disassembled separately, the at least two monitoring pipeline sections (122) are mounted to the monitoring panel (121) and are configured to monitor parameter information of the medical liquid and/or the blood, and the parameter information at least comprises a pressure parameter; at least one of the monitoring pipeline sections (122) is provided with a first interface (1221); if the second interface (204) is connected to the first interface (1221), the blood purification device (200) is capable of monitoring the parameter information through the monitoring pipeline sections (122); the first interface (1221) is movably arranged relative to the monitoring panel (121), and the movable arrangement allows a user to achieve connection of the first interface (1221) to the second interface (204) by operating the first interface (1221); or the first interface (1221) is fixedly arranged relative to the monitoring panel (121) in a process of mounting the monitoring panel (121) to the device housing (201).

5. A blood purification system, **characterized in that** the blood purification system comprises a panel kit (100) and a blood purification device (200), wherein the panel kit (100) comprises a power component (11) and a monitoring component (12), the power component (11) comprises a power panel (111) and at least two pump pipeline sections (112) mounted to the power panel (111); the monitoring component (12) comprises a monitoring panel (121), and at least two monitoring pipeline sections (122) mounted to the monitoring panel (121) and configured to monitor parameter information of a medical liquid and/or a blood, the parameter information at least comprises a pressure parameter, and at least one of the monitoring pipeline sections (122) is provided with a first interface (1221); wherein the blood purification device (200) comprises:
a device housing (201), provided with a power region (2011) and a parameter region (2012);
an assembly and disassembly component (202), comprising a first assembly and disassembly member (2021) and a second assembly and disassembly member (2022), wherein the power panel (111) can be detachably connected to the first assembly and disassembly member (2021) so as to achieve that the power panel (111) is mounted at a first target position in the power region (2011), the monitoring panel (121) can be detachably connected to the second assembly and disassembly member (2022) so as to achieve that the monitoring panel (121) is mounted at a second target position in the parameter region (2012), the power panel (111) and the monitoring panel (121) are independent of each other and can be assembled and disassembled separately;
a pump component (203), arranged in the power region (2011) and comprising a blood pump (2031) and at least one medical liquid pump (2032), wherein if the power panel (111) is mounted at the first target position, the blood pump (2031) is capable of being adapted to at least one of the pump pipeline sections (112) so as to drive the blood to flow along the at least one of the pump pipeline sections (112), and the at least one medical liquid pump (2032) is capable of being adapted to other pump pipeline sections (112) so as to drive the medical liquid to flow along the other pump pipeline sections (112); and
at least two second interfaces (204), arranged in the parameter region (2012), wherein if the monitoring panel (121) is mounted at the second target position, the first interfaces (1221) are capable of being manually operated to achieve connection of the first interfaces (1221) to the second interfaces (204), and if the second interfaces (204) are connected to the first interfaces (1221) respectively, the blood purification device (200) is capable of monitoring the parameter information through the monitoring pipeline sections (122).

6. The blood purification system of any one of claims 1 to 5, wherein the second interface (204) is fixedly arranged relative to the device housing (201); or
the second interface (204) is movably arranged relative to the device housing (201).

7. The blood purification system of any one of claims 1 to 5, wherein at least two of the monitoring pipeline sections (122) are provided with the first interfaces (1221) respectively, and when the blood purification device (200) comprises at least two second interfaces (204), the at least two second interfaces (204) comprise at least two pressure interfaces, and if the pressure interfaces are connected to the first interfaces (1221) respectively, the blood purification device (200) is capable of monitoring the pressure parameter through the monitoring pipeline sections (122); or
the at least two second interfaces (204) comprise at least one pressure interface, and if the pressure interface is connected to the first interface (1221), the blood purification device (200) is capable of monitoring the pressure parameter through the monitoring pipeline sections (122); the blood purification device (200) further comprises a blood leakage monitoring module which is capable of monitoring, through the monitoring pipeline sections (122), whether the blood at one side of a filtration membrane of a filter (300) permeates to the other side of the filtration membrane.

8. The blood purification system of any one of claims 1 to 3 and 5, wherein the device housing (201) is provided with a first surface (2013), each of the parameter region (2012) and the power region (2011) is arranged on the first surface (2013), and the parameter region (2012) is located at a lower side of the power region (2011) along a vertical direction,
preferably, the device housing (201) is further provided with a second surface (2014) that faces toward a direction different from the first surface (2013), is located at a side of the first surface (2013) along a horizontal direction, and is provided with a heating region (207) where a heating component (15) of the panel kit (100) is configured to be detachably mounted;
if the heating component (15) is mounted on the heating region (207), the medical liquid and/or the blood in the heating component (15) are capable of being heated by the blood purification device (200),
more preferably, the heating component (15) is provided with a plurality of liquid flow paths configured to deliver the medical liquid;
the blood purification device (200) further comprises a pipeline switching unit (208) arranged on the device housing (201), and if the heating component (15) is mounted on the heating region (207), the pipeline switching unit (208) is capable of controlling on/off of at least two of the liquid flow paths.

9. A blood purification device, **characterized in that** the blood purification device (200) is configured to be adapted to a panel kit (100) so as to perform extracorporeal blood purification, wherein the panel kit (100) comprises a power component (11) and a monitoring component (12), the power component (11) comprises a power panel (111) and at least two pump pipeline sections (112) mounted to the power panel (111); the monitoring component (12) comprises a monitoring panel (121), and at least two monitoring pipeline sections (122) mounted to the monitoring panel (121) and configured to monitor parameter information of a medical liquid and/or a blood, the parameter information at least comprises a pressure parameter, and at least one of the monitoring pipeline sections (122) is provided with a first interface (1221); wherein the blood purification device (200) comprises:
a device housing (201), provided with a power region (2011) and a parameter region (2012);
an assembly and disassembly component (202), comprising a first assembly and disassembly member (2021) and a second assembly and disassembly member (2022), wherein the power panel (111) can be detachably connected to the first assembly and disassembly member (2021) so as to achieve that the power panel (111) is mounted at a first target position in the power region (2011), the monitoring panel (121) can be detachably connected to the second assembly and disassembly member (2022) so as to achieve that the monitoring panel (121) is mounted at a second target position in the parameter region (2012), the power panel (111) and the monitoring panel (121) are independent of each other and can be assembled and disassembled separately;
a pump component (203), arranged in the power region (2011) and comprising a blood pump (2031) and at least one medical liquid pump (2032), wherein if the power panel (111) is mounted at the first target position, the blood pump (2031) is capable of being adapted to at least one of the pump pipeline sections (112) so as to drive the blood to flow along the at least one of the pump pipeline sections (112), and the at least one medical liquid pump (2032) is capable of being adapted to other pump pipeline sections (112) so as to drive the medical liquid to flow along the other pump pipeline sections (112); and
at least two second interfaces (204), arranged in the parameter region (2012), wherein the monitoring component (12) is capable of being manually mounted such that the monitoring panel (121) is mounted at the second target position and the second interfaces (204) are synchronously connected to the first interfaces (1221) respectively, and if the second interfaces (204) are connected to the first interfaces (1221) respectively, the blood purification device (200) is capable of monitoring the parameter information through the monitoring pipeline sections (122).

10. A blood purification device, **characterized in that** the blood purification device (200) is configured to be adapted to a panel kit (100) so as to perform extracorporeal blood purification, wherein the panel kit (100) comprises a power component (11) and a monitoring component (12), the power component (11) comprises a power panel (111) and at least two pump pipeline sections (112) mounted to the power panel (111); the monitoring component (12) comprises a monitoring panel (121), and at least two monitoring pipeline sections (122) mounted to the monitoring panel (121) and configured to monitor parameter information of a medical liquid and/or a blood, the parameter information at least comprises a pressure parameter, and at least one of the monitoring pipeline sections (122) is provided with a first interface (1221); wherein the blood purification device (200) comprises:
a device housing (201), provided with a power region (2011) and a parameter region (2012);
an assembly and disassembly component (202), comprising a first assembly and disassembly member (2021) and a second assembly and disassembly member (2022), wherein the power panel (111) can be detachably connected to the first assembly and disassembly member (2021) so as to achieve that the power panel (111) is mounted at a first target position in the power region (2011), the monitoring panel (121) can be detachably connected to the second assembly and disassembly member (2022) so as to achieve that the monitoring panel (121) is mounted at a second target position in the parameter region (2012), the power panel (111) and the monitoring panel (121) are independent of each other and can be assembled and disassembled separately;
a pump component (203), arranged in the power region (2011) and comprising a blood pump (2031) and at least one medical liquid pump (2032), wherein if the power panel (111) is mounted at the first target position, the blood pump (2031) is capable of being adapted to at least one of the pump pipeline sections (112) so as to drive the blood to flow along the at least one of the pump pipeline sections (112), and the at least one medical liquid pump (2032) is capable of being adapted to other pump pipeline sections (112) so as to drive the medical liquid to flow along the other pump pipeline sections (112); and
at least two second interfaces (204), arranged in the parameter region (2012) telescopically, wherein if the monitoring panel (121) is mounted at the second target position, the second interfaces (204) can be extended to be connected to the first interfaces (1221) respectively, and if the second interfaces (204) are connected to the first interfaces (1221) respectively, the blood purification device (200) is capable of monitoring the parameter information through the monitoring pipeline sections (122).

11. A blood purification device, **characterized in that** the blood purification device (200) is configured to be adapted to a panel kit (100) so as to perform extracorporeal blood purification, wherein the panel kit (100) comprises a power component (11) and a monitoring component (12), the power component (11) comprises a power panel (111) and at least two pump pipeline sections (112) mounted to the power panel (111); the monitoring component (12) comprises a monitoring panel (121), and at least two monitoring pipeline sections (122) mounted to the monitoring panel (121) and configured to monitor parameter information of a medical liquid and/or a blood, the parameter information at least comprises a pressure parameter, and at least one of the monitoring pipeline sections (122) is provided with a first interface (1221); wherein the blood purification device (200) comprises:
a device housing (201), provided with a power region (2011) and a parameter region (2012);
an assembly and disassembly component (202), comprising a first assembly and disassembly member (2021) and a second assembly and disassembly member (2022), wherein the power panel (111) can be detachably connected to the first assembly and disassembly member (2021) so as to achieve that the power panel (111) is mounted at a first target position in the power region (2011), the monitoring panel (121) can be detachably connected to the second assembly and disassembly member (2022) so as to achieve that the monitoring panel (121) is mounted at a second target position in the parameter region (2012), the power panel (111) and the monitoring panel (121) are independent of each other and can be assembled and disassembled separately;
a pump component (203), arranged in the power region (2011) and comprising a blood pump (2031) and at least one medical liquid pump (2032), wherein if the power panel (111) is mounted at the first target position, the blood pump (2031) is capable of being adapted to at least one of the pump pipeline sections (112) so as to drive the blood to flow along the at least one of the pump pipeline sections (112), and the at least one medical liquid pump (2032) is capable of being adapted to other pump pipeline sections (112) so as to drive the medical liquid to flow along the other pump pipeline sections (112); and
at least two second interfaces (204), arranged in the parameter region (2012), wherein if the monitoring panel (121) is mounted at a second preset position of the device housing (201), the second assembly and disassembly member (2022) is capable of driving the monitoring panel (121) to move to the second target position, such that the second interfaces (204) are connected to the first interfaces (1221) respectively, and if the second interfaces (204) are connected to the first interfaces (1221) respectively, the blood purification device (200) is capable of monitoring the parameter information through the monitoring pipeline sections (122).

12. A blood purification device, **characterized in that** the blood purification device (200) is configured to be adapted to a panel kit (100) so as to perform extracorporeal blood purification, wherein the panel kit (100) comprises a power component (11) and a monitoring component (12), the power component (11) comprises a power panel (111) and at least two pump pipeline sections (112) mounted to the power panel (111); the monitoring component (12) comprises a monitoring panel (121), and at least two monitoring pipeline sections (122) mounted to the monitoring panel (121) and configured to monitor parameter information of a medical liquid and/or a blood, the parameter information at least comprises a pressure parameter, and at least one of the monitoring pipeline sections (122) is provided with a first interface (1221); wherein the blood purification device (200) comprises:
a device housing (201), provided with a power region (2011) and a parameter region (2012);
an assembly and disassembly component (202), comprising a first assembly and disassembly member (2021) and a second assembly and disassembly member (2022), wherein the power panel (111) can be detachably connected to the first assembly and disassembly member (2021) so as to achieve that the power panel (111) is mounted at a first target position in the power region (2011), the monitoring panel (121) can be detachably connected to the second assembly and disassembly member (2022) so as to achieve that the monitoring panel (121) is mounted at a second target position in the parameter region (2012), the power panel (111) and the monitoring panel (121) are independent of each other and can be assembled and disassembled separately;
a pump component (203), arranged in the power region (2011) and comprising a blood pump (2031) and at least one medical liquid pump (2032), wherein if the power panel (111) is mounted at the first target position, the blood pump (2031) is capable of being adapted to at least one of the pump pipeline sections (112) so as to drive the blood to flow along the at least one of the pump pipeline sections (112), and the at least one medical liquid pump (2032) is capable of being adapted to other pump pipeline sections (112) so as to drive the medical liquid to flow along the other pump pipeline sections (112); and
at least two second interfaces (204), arranged in the parameter region (2012), wherein if the monitoring panel (121) is mounted at the second target position, the first interfaces (1221) are capable of being manually operated to achieve connection of the first interfaces (1221) to the second interfaces (204), and if the second interfaces (204) are connected to the first interfaces (1221) respectively, the blood purification device (200) is capable of monitoring the parameter information through the monitoring pipeline sections (122).

13. The blood purification device of any one of claims 9 to 12, wherein the second interface (204) is fixedly arranged relative to the device housing (201); or
the second interface (204) is movably arranged relative to the device housing (201).

14. The blood purification device of any one of claims 9 to 12, wherein at least two of the monitoring pipeline sections (122) are provided with the first interfaces (1221) respectively, and the at least two second interfaces (204) comprise at least two pressure interfaces, and if the pressure interfaces are connected to the first interfaces (1221) respectively, the blood purification device (200) is capable of monitoring the pressure parameter through the monitoring pipeline sections (122); or
the at least two second interfaces (204) comprise at least one pressure interface, and if the pressure interface is connected to the first interface (1221), the blood purification device (200) is capable of monitoring the pressure parameter through the monitoring pipeline sections (122); the blood purification device (200) further comprises a blood leakage monitoring module which is capable of monitoring, through the monitoring pipeline sections (122), whether the blood at one side of a filtration membrane of a filter (300) permeates to the other side of the filtration membrane.

15. The blood purification device of any one of claims 9 to 12, wherein the device housing (201) is provided with a first surface (2013), each of the parameter region (2012) and the power region (2011) is arranged on the first surface (2013), and the parameter region (2012) is located at a lower side of the power region (2011) along a vertical direction,
preferably, the device housing (201) is further provided with a second surface (2014) that faces toward a direction different from the first surface (2013), is located at a side of the first surface (2013) along a horizontal direction, and is provided with a heating region (207) where a heating component (15) of the panel kit (100) is configured to be detachably mounted;
if the heating component (15) is mounted on the heating region (207), the medical liquid and/or the blood in the heating component (15) are capable of being heated by the blood purification device (200),
more preferably, the heating component (15) is provided with a plurality of liquid flow paths configured to deliver the medical liquid;
the blood purification device (200) further comprises a pipeline switching unit (208) arranged on the device housing (201), and if the heating component (15) is mounted on the heating region (207), the pipeline switching unit (208) is capable of controlling on/off of at least two of the liquid flow paths.
